(19) **Europäisches Patentamt / European Patent Office / Office européen des brevets**

(11) **EP 3 858 465 B1**

(12) **FASCICULE DE BREVET EUROPEEN**

(45) Date de publication et mention de la délivrance du brevet:
**10.01.2024 Bulletin 2024/02**

(21) Numéro de dépôt: **21152437.6**

(22) Date de dépôt: **20.01.2021**

(51) Classification Internationale des Brevets (IPC):
**B01D 53/14** *(2006.01)*  **B01D 53/52** *(2006.01)*
**B01D 53/58** *(2006.01)*  **B01D 53/62** *(2006.01)*
**B01D 53/78** *(2006.01)*  **B01D 53/22** *(2006.01)*
**B01D 53/96** *(2006.01)*

(52) Classification Coopérative des Brevets (CPC):
**B01D 53/1425; B01D 53/229; B01D 53/52; B01D 53/58; B01D 53/62; B01D 53/78; B01D 53/96; B01D 53/965; C12M 47/18;** B01D 2251/302; B01D 2251/304; B01D 2251/306; B01D 2251/502; B01D 2251/604; B01D 2251/608;

(Cont.)

(54) **PROCÉDÉ DE PURIFICATION D'UN GAZ PAR ABSORPTION GAZ-LIQUIDE**

VERFAHREN ZUR REINIGUNG EINES GASES DURCH GAS-FLÜSSIGKEITSABSORPTION

METHOD FOR PURIFYING A GAS BY GAS-LIQUID ABSORPTION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.01.2020 FR 2000593**

(43) Date de publication de la demande:
**04.08.2021 Bulletin 2021/31**

(73) Titulaire: **CentraleSupélec**
**91190 - Gif-sur-Yvette (FR)**

(72) Inventeurs:
• **LEMAIRE, Julien**
**08300 Saint-Loup en Champagne (FR)**
• **DUVAL, Fanny**
**51100 Reims (FR)**
• **CHAVAN, Sayali Ramdas**
**51100 Reims (FR)**
• **POZZOBON, Victor**
**08300 Rethel (FR)**

• **PERRE, Patrick**
**91120 Palaiseau (FR)**

(74) Mandataire: **Cabinet Bleger-Rhein-Poupon**
**4a rue de l'Industrie**
**67450 Mundolsheim (FR)**

(56) Documents cités:
EP-A1- 2 543 427      EP-A1- 3 372 297
AT-A4- 521 381        DE-A1- 4 235 125
US-A1- 2007 004 023   US-A1- 2010 059 377
US-A1- 2011 174 156   US-A1- 2011 229 403
US-A1- 2012 039 787

**EP 3 858 465 B1**

(52) Classification Coopérative des Brevets (CPC):
(Cont.)
B01D 2256/16; B01D 2256/245; B01D 2257/504;
B01D 2258/05; Y02C 20/40; Y02P 20/59

(52) Classification Coopérative des Brevets (CPC):
(Cont.)
B01D 2256/16; B01D 2256/245; B01D 2257/504;
B01D 2258/05; Y02C 20/40; Y02P 20/59

**Description**

La présente invention concerne le domaine général de la séparation des gaz par absorption gaz-liquide.

Un procédé d'épuration de gaz par absorption gaz-liquide joue sur la différence de solubilité des gaz dans un solvant. Il permet de séparer efficacement des gaz considérés comme étant peu solubles, présentant une Constante de Henry inférieure à $10^{-2}$ mol.$L^{-1}$.$bar^{-1}$, de gaz acides ou basiques présentant une solubilité plus importante, avec une Constante de Henry supérieure à $10^{-2}$ mol.$L^{-1}$.$bar^{-1}$.

La présente invention concerne, plus particulièrement, un procédé permettant la purification d'un gaz d'intérêt, contenu dans un mélange de deux ou plusieurs gaz, ledit gaz d'intérêt à purifier présentant, dans un solvant, une solubilité inférieure à la solubilité des autres gaz à éliminer.

Le procédé de purification de gaz par absorption gaz-liquide selon l'invention est applicable, en particulier mais non limitativement, au syngaz qui consiste en un mélange de gaz comprenant essentiellement du dioxyde de carbone $CO_2$ à absorber dans un solvant et du dihydrogène $H_2$ à purifier, et au biogaz comprenant du méthane $CH_4$ à purifier ainsi que, en tant que gaz à éliminer par absorption, du $CO_2$ et, dans une moindre mesure, du sulfure d'hydrogène $H_2S$.

Cette invention cherche à permettre la production d'un gaz d'intérêt, par exemple $H_2$ ou $CH_4$, de très haute pureté, renouvelable, écologique et économique, principalement pour des applications dans le domaine du transport ou de l'énergie.

Le développement d'un tel procédé de purification de gaz s'inscrit dans un programme global et national dont le but est de développer des voies de valorisation des biomasses renouvelables, notamment.

Dans ce cadre, notamment, des travaux sur l'épuration du biogaz, mélange constitué essentiellement de $CH_4$ et de $CO_2$, ont démarré avec l'objectif d'optimiser des procédés de méthanisation des co-produits agricoles et agro-industriels.

La méthanisation représente à la fois une voie de valorisation des déchets et coproduits agricoles, industriels ou urbains, et un moyen de production d'une énergie renouvelable. D'impact limité sur la qualité de l'air, la méthanisation permettrait de limiter l'émission des gaz à effet de serre et de réduire la consommation de ressources fossiles.

Le processus de méthanisation de matières organiques par des populations de micro-organismes permet d'aboutir à la production de biogaz composé notamment de $CH_4$, dans une proportion comprise entre 50 et 70% en volume, de $CO_2$, dans une proportion allant de 25 à 50% en volume, par rapport au volume total du biogaz produit, et de $H_2S$, dans une concentration de 20 000 ppm maximum.

La filière de la méthanisation est en constant développement, notamment en France, et cela depuis de nombreuses années. Le gisement méthanisable a été évalué à 55,9 TWh (térawattheure) à l'horizon des années 2030.

Il est donc particulièrement intéressant de chercher à développer des process pour la valorisation du biogaz issu de la méthanisation de matières organiques et plus particulièrement l'utilisation de celui-ci dans le réseau de gaz naturel ou en tant que carburant.

Toutefois, l'injection de biogaz dans le réseau ne peut être effectuée qu'une fois que les impuretés et les gaz indésirables, notamment le $CO_2$ et l'$H_2S$, ont été éliminés du biogaz directement issu de la méthanisation.

Ainsi, pour augmenter sa valeur calorifique et répondre aux spécifications des opérateurs de réseau de gaz naturel pour une injection dans le réseau et/ou son utilisation comme carburant automobile, le biogaz issu directement du processus de méthanisation doit subir une épuration, en sorte de comporter, finalement, une proportion de l'ordre de 92% et de préférence supérieure à 96%, de $CH_4$ suivant la qualité du gaz distribué, on parle alors de « biométhane ».

Ainsi, on connait, dans l'état de la technique, la demande de brevet européen publiée sous le n° EP 3 372 297 qui divulgue un procédé d'épuration, notamment, d'un flux de biogaz entrant composé d'un mélange de gaz comprenant du méthane $CH_4$ à purifier et au moins un gaz indésirable à éliminer, comme du dioxyde de carbone $CO_2$ ou du sulfure d'hydrogène $H_2S$.

Ce procédé s'appuie sur l'absorption du $CO_2$ et des autres gaz solubles, acides ou basiques, dans une saumure concentrée au moyen de contacteurs membranaires afin d'intensifier et d'optimiser le processus. Il comprend, plus particulièrement, les étapes suivantes :

- lors d'une étape d'absorption, on met en contact, au travers d'une unité membranaire d'absorption comprenant au moins une membrane perméable aux gaz et imperméable aux liquides, le flux de biogaz entrant d'un côté de ladite membrane et un liquide absorbant circulant de l'autre côté de ladite membrane, et on obtient un liquide absorbant riche comportant ledit gaz indésirable dissous et un flux de biométhane sortant appauvri en gaz indésirable à éliminer ;
- lors d'une étape de désorption, on met en contact ledit liquide absorbant riche avec une unité membranaire de désorption comprenant au moins une membrane, perméable aux gaz et imperméable aux liquides, et on élimine, par application d'une dépression transmembranaire, dudit liquide absorbant riche circulant d'un côté de ladite membrane, au moins une partie dudit gaz dissous, par passage au travers de ladite membrane vers l'autre côté de celle-ci, pour obtenir un liquide dégazé ;
- on recycle ledit liquide dégazé à l'étape d'absorption.

Dans ce procédé, le liquide absorbant consiste en une saumure concentrée comportant de l'eau et au moins un sel choisi parmi le chlorure de sodium NaCl, le chlorure de potassium KCl, et le sulfate de sodium $Na_2SO_4$, la concentration molaire en sel dans le liquide absorbant étant comprise entre 0,2 et 6 mol.$L^{-1}$.

On a cherché ici, par la mise au point d'un tel procédé, à obtenir une élimination satisfaisante des gaz indésirables et des impuretés mélangés, à l'origine dans le biogaz, au méthane valorisable, tout en réduisant substantiellement les pertes en méthane.

Ainsi, ce procédé présente déjà une amélioration considérable par rapport à l'état de la technique, en étant à la fois sobre et économique, en termes de consommation énergétique et de réactifs, et en permettant de produire un biométhane présentant une pureté supérieure à 97% et un rendement de 98%.

De telles valeurs, en termes de pureté et de rendement, sont suffisantes pour envisager une injection réseau viable du $CH_4$ obtenu, à la fois techniquement et économiquement, pour des petites et des moyennes unités de méthanisation.

Toutefois, la pureté du $CH_4$ récupéré à l'issue de la mise en oeuvre de ce procédé pourrait encore être améliorée, une certaine proportion d'impuretés résiduelles, pouvant aller jusqu'à 3% en volume, étant encore présente dans le flux de biométhane sortant.

En plus de la production de biogaz, une des autres voies de valorisation des biomasses renouvelables consiste à produire du syngaz, composé essentiellement, à la base, de monoxyde de carbone CO et de $H_2$, par thermolyse et pyrogazéification de déchets.

Plus particulièrement, la pyrogazéification de déchets consiste à chauffer ces derniers à des températures élevées, pouvant aller jusqu'à 1500°C, en l'absence de dioxygène $O_2$ ou en présence d'une faible quantité de ce gaz, apportée, dans ce cas, soit par de l'air, soit par de l'air enrichi en $O_2$, du $O_2$ pur, du $CO_2$ ou de la vapeur d'eau.

Un tel chauffage, effectué à pression atmosphérique, entraine la transformation de la matière carbonée présente dans les déchets en gaz de synthèse, ou syngaz, dont la composition première a déjà été indiquée précédemment.

Cette voie permet de produire un vecteur énergétique gazeux intéressant, le dihydrogène $H_2$, tout comme le $CH_4$ produit lors de la méthanisation.

Le dihydrogène $H_2$ peut être, en partie, injecté dans le réseau de gaz ou peut alimenter des piles à combustible, en particulier pour des applications dans le domaine de l'automobile.

Généralement, des procédés de type « Water Gas Shift » ou réaction du gaz à l'eau, encore dénommée réaction de Dussan, sont mis en oeuvre dans l'optique d'enrichir le syngaz en $H_2$. Une telle réaction chimique permet de convertir du CO et de la vapeur d'eau en $CO_2$ et $H_2$.

Par conséquent, l'épuration du syngaz, enrichi en $H_2$, comporte également, suite à une réaction du gaz à l'eau, en tant que principal coproduit généré, du $CO_2$ qu'il conviendra d'éliminer.

Le procédé d'épuration de biogaz par absorption gaz-liquide tel que décrit dans la demande de brevet européen EP 3 372 297 est également applicable à la purification de $H_2$ contenu dans le syngaz. Dans une telle application, le procédé permettrait une élimination du $CO_2$ contenu en mélange dans le syngaz avec le $H_2$ d'intérêt. Préalablement, la teneur en $CH_4$ du syngaz devrait être réduite autant que possible par pyrolyse à plus de 1000°C, tandis que la teneur en CO est réduite en dessous de 0,1% après un procédé « Water Gas Shift ».

Cependant, ce procédé d'épuration de gaz par absorption gaz-liquide, aboutissant à une pureté du gaz à récupérer supérieure à 97%, correspondant à une teneur résiduelle en $CO_2$ supérieure à 1 %, ne permet pas d'atteindre les spécifications requises pour que le dihydrogène $H_2$ purifié puisse alimenter les piles à combustibles conventionnelles.

En effet, pour une telle application, notamment, le dihydrogène $H_2$ doit présenter une pureté supérieure à 99,97%, avec, en particulier, une teneur en $CO_2$ inférieure à 2 ppm, une teneur en CO inférieure à 0,2 ppm, une teneur en $H_2S$ inférieure à 4 ppb, et une teneur en chlorure d'hydrogène HCl inférieure à 50 ppb, selon les spécifications pile à hydrogène de la norme 2014.

Par conséquent, il est indispensable de mettre au point un procédé de purification d'un mélange de gaz, applicable notamment à la purification de biogaz ou de syngaz, contenant un gaz d'intérêt ($H_2$ pour le syngaz, $CH_4$ pour le biogaz) mélangé à des impuretés à éliminer, afin d'obtenir le gaz d'intérêt, respectivement $H_2$ ou $CH_4$, d'une pureté supérieure à 99,97%, et plus préférentiellement encore supérieure à 99,99%, suffisante, notamment, pour pouvoir utiliser le $H_2$ dans des applications « mobilité », en particulier dans les piles à combustibles.

Dans ce but, les inventeurs ont déterminé que les performances du procédé décrit dans la demande de brevet européen EP 3 372 297, présentant une étape d'absorption des gaz à éliminer d'un mélange dans un solvant, et une étape de dégazage afin de recycler le solvant utilisé, étaient limitées par une résistance au transfert des gaz à éliminer, notamment le $CO_2$, au travers de la membrane d'absorption.

Une telle résistance est, par ailleurs, amplifiée par un phénomène progressif d'humidification des pores de la membrane mise en oeuvre dans l'étape d'absorption.

Cette limitation du transfert de matière ($CO_2$ vers solvant liquide) permet d'expliquer la baisse de la performance observée lors de l'utilisation prolongée des contacteurs membranaires, ainsi que les écarts observés entre la théorie et les résultats effectivement obtenus lors de la mise en oeuvre pratique.

Cependant, dans le cas de l'absorption du $CO_2$ dans un solvant aqueux recyclé par dégazage sous un vide grossier, d'environ 100 mbar, cela ne permet pas d'expliquer la limitation observée de la pureté du gaz qui reste, dans tous les cas, inférieure à 99%.

En effet, quelles que soient les conditions opératoires mises en oeuvre, le gaz purifié présente systématiquement une teneur résiduelle en $CO_2$ d'au moins 1%.

Dans l'état de la technique, certaines solutions développées pour chercher à améliorer la pureté du gaz à récupérer consistent à utiliser des solvants chimiques présentant une plus forte capacité d'absorption du $CO_2$, notamment des solvants de type bases fortes ou faibles.

Toutefois, de tels solvants présentent l'inconvénient d'être beaucoup plus difficiles à régénérer, par dégazage sous vide, que la saumure concentrée mise en oeuvre dans le procédé de la demande EP 3 372 297. Cette étape de régénération nécessite alors une augmentation de la température pour que le recyclage du solvant soit efficace. En outre, le solvant est parfois non-réutilisable, entraînant alors une consommation importante de produit chimique, ainsi que la production d'un effluent à traiter.

D'autres solutions consistent à faciliter le transfert du $CO_2$ entre le gaz entrant et le solvant en utilisant différents types de membranes, ou bien encore en ajoutant des catalyseurs dans le solvant.

Ainsi, par exemple, le document de brevet US 2011/223650 divulgue un procédé mettant en oeuvre deux contacteurs membranaires en boucle, un pour l'étape d'absorption et un pour l'étape de désorption, ainsi que des enzymes de type anhydrase carbonique, pour améliorer les performances de séparation du $CO_2$, à partir d'un mélange de plusieurs gaz, les enzymes catalysant la conversion du $CO_2$ dissous en ions carbonates.

Le document US 2011/174156 divulgue un réacteur modulaire et un procédé pour extraire le $CO_2$ d'un mélange gazeux avec deux modules, l'un pour l'absorption et l'autre pour la désorption, l'extraction du $CO_2$ vers un liquide porteur étant facilitée également à l'aide d'enzymes de type anhydrase carbonique, qui favorise notamment le transfert du $CO_2$ gazeux vers le liquide porteur, en accélérant la réaction du $CO_2$ dissous et de l'eau pour former l'acide carbonique qui se dissocie ensuite en carbonate et en bicarbonate. Préférentiellement, les deux modules, absorption et désorption, contiennent de l'anhydrase carbonique.

L'enzyme anhydrase carbonique peut être soit en solution dans le liquide porteur qui circule dans le réacteur, soit immobilisée au niveau des membranes des modules d'absorption et de désorption.

Le liquide porteur peut être un solvant physique ayant une sélectivité pour le $CO_2$, notamment le glycérol, le polyéthylène glycol, les éthers de polyéthylène glycol, les éthers diméthyliques de polyéthylène glycol, l'eau, le méthanol réfrigéré ou le glycérol carbonate, et il contient préférentiellement au moins un agent tampon (bicarbonate, phosphate, ...) pour maintenir le pH dans des gammes qui sont préconisées.

En effet, dans le procédé et le réacteur qui sont décrits dans ce document, le pH du liquide porteur est régulé au travers de moyens de régulation appropriés, par ajout d'une substance alcaline avant l'étape d'absorption, ou bien par ajout d'une substance neutre ou acide avant l'étape de désorption.

Une telle régulation du pH est effectuée dans un réservoir du liquide porteur, en utilisant un équipement d'ajustement automatique du pH, comme un titrateur automatique.

De telles solutions, si elles permettent effectivement d'améliorer les performances du procédé en termes d'extraction du $CO_2$, exprimées seulement en pourcentage de $CO_2$ capté du gaz traité, elles n'aboutissent pas à l'obtention d'une meilleure pureté en ce qui concerne un gaz d'intérêt, que l'on cherche à récupérer. En effet, les deux critères de performance, taux d'extraction du $CO_2$ et pureté du gaz d'intérêt à récupérer, ne sont pas directement corrélés, et peuvent même évoluer en sens inverse.

En outre, ces solutions présentent par ailleurs plusieurs inconvénients, en particulier celui d'engendrer une consommation importante de composés acides et basiques pour la régulation du pH du liquide porteur.

En outre, un autre inconvénient de la mise en oeuvre d'une telle régulation du pH par ajout de composés acide et basique dans le liquide porteur réside dans le fait qu'il entraîne la formation du sel correspondant à l'acide et la base utilisés, et à son accumulation dans le liquide porteur.

Cette opération nécessite de déminéraliser le liquide porteur pour maintenir sa composition chimique constante. Cela peut se faire indirectement par l'ajout continuel d'eau déminéralisée dans la boucle liquide, ce qui conduit à la production continuelle d'un effluent salin à déminéraliser. Une autre solution consiste à déminéraliser directement et en continu le liquide porteur par distillation, osmose inverse ou échange d'ions.

Quoi qu'il en soit, ce mode de régulation de pH entraîne la production de sel, et donc la nécessité d'éliminer ce sel par des méthodes qui sont soit coûteuses énergétiquement (distillation ou osmose inverse), soit coûteuses en produits chimiques (sels régénérants pour l'échange d'ions).

En outre, l'élimination du sel produit et sa concentration dans un effluent salin conduisent, au final, à la production d'un déchet.

Dans une démarche inventive, les inventeurs ont mis au point une solution particulièrement intéressante permettant l'obtention d'un gaz d'intérêt récupéré, dont la pureté peut aller, en proportion, jusqu'à plus de 99,99%, tout en proposant un procédé d'épuration de gaz qui reste sobre énergétiquement, ne consommant pas de produits chimiques de manière excessive, et ne générant aucun effluent ou déchet autre que le $CO_2$ et les autres gaz séparés.

Dans certains procédés connus de l'état de la technique, le dégazage insuffisant du $CO_2$ (ou autres impuretés gazeuses à éliminer), au niveau de la membrane de l'unité de désorption, est responsable d'un recyclage non optimal du solvant absorbant les gaz, en raison d'une variation de pH défavorable au sein de ce solvant.

Par exemple, lors du dégazage du $CO_2$ au moment de la désorption, en utilisant une pompe à vide, le pH du solvant va naturellement augmenter et une partie du $CO_2$ va rester en solution dans ce solvant sous la forme d'anions hydrogénocarbonates $HCO_3^-$ et carbonates $CO_3^{2-}$. Comme le procédé d'épuration de gaz par absorption du $CO_2$ est à contre-courant, la pureté en sortie du gaz est limitée thermodynamiquement par une teneur résiduelle en $CO_2$ dans le solvant recyclé entrant dans l'unité membranaire d'absorption.

Certaines solutions actuelles, reposant sur l'augmentation de la capacité d'absorption du solvant, ou la recherche d'une amélioration des cinétiques de transfert au sein du liquide, de la membrane ou du gaz, ne permettent pas de lever ce verrou, ou seulement au prix d'une consommation prohibitive d'énergie ou de produits chimiques, dans le cas notamment de l'utilisation de solvants basiques, en tant qu'absorbants chimiques pour réagir avec le $CO_2$ absorbé, ou bien dans le cas de l'utilisation de composés acides et basiques pour réguler le pH en vue d'améliorer l'efficacité de l'absorption et de la désorption du gaz à éliminer.

Pour pallier, au moins en partie, les inconvénients de l'état de la technique, la présente invention propose dans la revendication 1 un procédé d'épuration d'un flux de gaz entrant, de purification, par absorption gaz-liquide, d'un flux de gaz entrant comprenant un gaz $G_0$ à purifier et au moins un gaz acide $Ga_1$ à éliminer, ledit gaz $G_0$ à purifier présentant une Constante de Henry $H_{G0}$ inférieure à $10^{-2}$ $mol.L^{-1}.bar^{-1}$ et ledit gaz acide $Ga_1$ à éliminer présentant une Constante de Henry $H_{Ga1}$ supérieure à $10^{-2}$ $mol.L^{-1}.bar^{-1}$ et une constante d'acidité $pKa_{Ga1}$ supérieure ou égale à 1 et inférieure ou égale à 13, ledit procédé comprenant, entre autres, les étapes suivantes :

- lors d'une étape d'absorption, on met au contact, au travers d'une unité membranaire d'absorption comprenant au moins une membrane perméable aux gaz et hydrophobe, ledit flux de gaz entrant d'un côté de ladite membrane et un solvant aqueux, apte à absorber les gaz solubles et circulant de l'autre côté de ladite membrane, et on obtient un solvant aqueux riche comportant ledit gaz acide $Ga_1$ dissous et un flux de gaz sortant enrichi en gaz $G_0$ à purifier ;
- lors d'une étape de désorption, on met en contact ledit solvant aqueux riche avec une unité membranaire de désorption comprenant au moins une membrane, perméable aux gaz et hydrophobe, et on élimine, par application d'une dépression transmembranaire, dudit solvant aqueux riche circulant d'un côté de ladite membrane, ledit gaz acide $Ga_1$ dissous, par passage au travers de ladite membrane vers l'autre côté de celle-ci, pour obtenir un solvant aqueux dégazé ;
- on recycle ledit solvant aqueux dégazé à l'étape d'absorption, l'ensemble des étapes dudit procédé étant opérées en continu en boucle fermée, ledit solvant aqueux étant mis en circulation dans un circuit en boucle fermée entre ladite unité membranaire d'absorption et ladite unité membranaire de désorption.

Dans le procédé de l'invention, on ajuste le pH du solvant aqueux à une valeur supérieure ou égale à $pKa_{Ga1}+2$ et inférieure ou égale à 13, par ajout d'une solution basique, avant l'étape d'absorption, et on ajuste le pH du solvant aqueux riche à une valeur supérieure ou égale à $pKa_{Ga1}-2$ et inférieure ou égale à $pKa_{Ga1}+1$, par ajout d'une solution acide, avant l'étape de désorption. Ainsi, ledit flux de gaz sortant comprend préférentiellement plus de 99% en volume de gaz $G_0$ à purifier.

Le procédé de l'invention est particulier en ce que l'on régénère, à partir du solvant aqueux dégazé, la base et l'acide, ajoutés audit solvant aqueux pour ajuster le pH, par une étape d'électrodialyse à membrane bipolaire.

Selon la revendication 6, l'invention est également relative à un procédé de purification, par absorption gaz-liquide, d'un flux de gaz entrant comprenant un gaz $G_0$ à purifier et au moins un gaz basique $Gb_1$ à éliminer, ledit gaz $G_0$ à purifier présentant une Constante de Henry $H_{G0}$ inférieure à $10^{-2}$ $mol.L^{-1}.bar^{-1}$ et ledit gaz basique $Gb_1$ à éliminer présentant une Constante de Henry $H_{Gb1}$ supérieure à $10^{-2}$ $mol.L^{-1}.bar^{-1}$ et une constante d'acidité $pKa_{Gb1}$ supérieure ou égale à 1 et inférieure ou égale à 13.

Les étapes d'absorption et de désorption opérées en continu en boucle fermée sont similaires à celles mises en oeuvre pour l'élimination d'au moins un gaz acide, de même que l'étape de régénération de la base et de l'acide par la mise en oeuvre d'une étape d'électrodialyse à membrane bipolaire. Toutefois, pour l'élimination dudit gaz basique $Gb_1$, on ajuste le pH du solvant aqueux à une valeur supérieure ou égale à 1 et inférieure ou égale à $pKa_{Gb1}-2$, par ajout d'une solution acide, avant l'étape d'absorption et on ajuste le pH du solvant aqueux riche à une valeur supérieure ou égale à $pKa_{Gb1}-1$ et inférieure ou égale à $pKa_{Gb1}+2$, par ajout d'une solution basique, avant l'étape de désorption. Ledit flux de gaz sortant comprend plus de 99% en volume de gaz $G_0$ à purifier.

Selon la revendication 8, l'invention a encore pour objet un procédé de purification, par absorption gaz-liquide, d'un flux de gaz entrant comprenant un gaz $G_0$ à purifier et un mélange de gaz à éliminer, ledit mélange comportant au moins un gaz acide $Ga_1$ et un gaz basique $Gb_1$, ledit au moins un gaz acide $Ga_1$ étant majoritaire, ledit gaz $G_0$ à purifier présentant une Constante de Henry $H_{G0}$ inférieure à $10^{-2}$ $mol.L^{-1}.bar^{-1}$, ledit gaz acide $Ga_1$ à éliminer présentant une Constante de Henry $H_{Ga1}$ supérieure à $10^{-2}$ mol.L$^{-1}$.bar$^{-1}$ et une constante d'acidité pKa$_{Ga1}$ supérieure ou égale à 1 et inférieure ou égale à 13 et ledit gaz basique Gb$_1$ à éliminer présentant une Constante de Henry $H_{Gb1}$ supérieure à $10^{-2}$ mol.L$^{-1}$.bar$^{-1}$ et une constante d'acidité pKa$_{Gb1}$ supérieure ou égale à 1 et inférieure ou égale à 13, ledit procédé comprenant, entre autres, les étapes suivantes :

- lors d'une étape d'absorption, on met au contact, au travers d'une unité membranaire d'absorption comprenant au moins une membrane perméable aux gaz et hydrophobe, ledit flux de gaz entrant d'un côté de ladite membrane et un solvant aqueux, apte à absorber les gaz solubles et circulant de l'autre côté de ladite membrane, et on obtient un solvant aqueux riche comportant ledit gaz acide Ga$_1$ dissous et ledit gaz basique Gb$_1$ dissous et un flux de gaz sortant enrichi en gaz G$_0$ à purifier ;
- lors d'une première étape de désorption, on met en contact ledit solvant aqueux riche avec une première unité membranaire de désorption comprenant au moins une membrane, perméable aux gaz et hydrophobe, et on élimine, par application d'une dépression transmembranaire, dudit solvant aqueux riche circulant d'un côté de ladite membrane, ledit gaz acide Ga$_1$ dissous, par passage au travers de ladite membrane vers l'autre côté de celle-ci, pour obtenir un solvant aqueux riche en gaz basique Gb$_1$ et débarrassé dudit gaz acide Ga$_1$ dissous ;
- lors d'une deuxième étape de désorption, on met en contact ledit solvant aqueux riche en gaz basique Gb$_1$ avec une deuxième unité membranaire de désorption comprenant au moins une membrane, perméable aux gaz et hydrophobe, et on élimine, par application d'une dépression transmembranaire, du solvant aqueux riche en gaz basique Gb$_1$ circulant d'un côté de ladite membrane, ledit gaz basique Gb$_1$ dissous, par passage au travers de ladite membrane vers l'autre côté de celle-ci, pour obtenir un solvant aqueux dégazé ;
- on recycle ledit solvant aqueux dégazé à l'étape d'absorption, l'ensemble des étapes dudit procédé étant opérées en continu en boucle fermée, ledit solvant aqueux étant mis en circulation dans un circuit en boucle fermée entre ladite unité membranaire d'absorption et lesdites unités membranaires de désorption.

Dans ce procédé, on ajuste le pH du solvant aqueux à une valeur supérieure ou égale à pKa$_{Ga1}$+2 et inférieure ou égale à 13, par ajout d'une solution basique, avant l'étape d'absorption et on ajuste le pH du solvant aqueux riche à une valeur supérieure ou égale à pKa$_{Ga1}$-2 et inférieure ou égale à pKa$_{Ga1}$+1, par ajout d'une solution acide, avant la première étape de désorption, et à une valeur supérieure ou égale à pKa$_{Gb1}$-1 et inférieure ou égale à pKa$_{Gb1}$+2, par ajout d'une solution basique, avant la deuxième étape de désorption. Ainsi, ledit flux de gaz sortant comprend préférentiellement plus de 99% en volume de gaz G$_0$ à purifier.

Là encore, on régénère, à partir du solvant aqueux dégazé, la base et l'acide, ajoutés audit solvant aqueux pour ajuster le pH, par une étape d'électrodialyse à membrane bipolaire. Dans cette configuration où deux étapes de désorption sont mises en oeuvre, l'étape d'électrodialyse à membrane bipolaire est conduite sur le solvant dégazé suivant la deuxième étape de désorption.

Un autre objet de la présente invention est un procédé de purification par absorption gaz-liquide selon la revendication 10 d'un flux de gaz entrant comprenant un gaz G$_0$ à purifier et un mélange de gaz à éliminer, ledit mélange comportant au moins un gaz basique Gb$_1$ et un gaz acide Ga$_1$, ledit au moins un gaz basique Gb$_1$ étant majoritaire.

Les étapes d'absorption et de désorption opérées en continu en boucle fermée, de même que l'étape de régénération de la base et de l'acide par électrodialyse à membrane bipolaire, sont similaires à celles mises en oeuvre pour l'élimination d'un mélange de gaz à éliminer avec Ga$_1$ et Gb$_1$, Ga$_1$ étant majoritaire. A présent, lorsque Gb$_1$ est majoritaire, on ajuste le pH du solvant aqueux à une valeur supérieure ou égale à 1 et inférieure ou égale à pKa$_{Gb1}$-2, par ajout d'acide, avant l'étape d'absorption et en ce que l'on ajuste le pH du solvant aqueux riche à une valeur supérieure ou égale à pKa$_{Gb1}$-1 et inférieure ou égale à pKa$_{GbH}$2, par ajout de base, avant la première étape de désorption et à une valeur supérieure ou égale à pKa$_{Ga1}$-2 et inférieure ou égale à pKa$_{Ga1}$+1, par ajout d'acide, avant la deuxième étape de désorption, et en ce que ledit flux de gaz sortant comprend plus de 99% en volume de gaz G$_0$ à purifier.

De manière tout à fait préférentielle, pour la mise en oeuvre de l'étape d'électrodialyse à membrane bipolaire :

- on achemine un flux de solvant aqueux, notamment une fois celui-ci dégazé, depuis le réservoir que comporte avantageusement le circuit en boucle fermée vers un module d'électrodialyse à membrane bipolaire à trois compartiments, qui comporte un stack qui consiste en un assemblage, en alternance, de membranes bipolaires et monopolaires, compris entre une cathode et une anode ;
- on divise ledit flux de solvant aqueux arrivant au niveau du module d'électrodialyse, une première partie du flux étant acheminée au niveau d'une cuve de solution acide que comporte ledit module, une deuxième partie du flux étant acheminée au niveau d'une cuve de solution basique que comporte également ledit module, une troisième partie du flux alimentant un premier compartiment, dénommé compartiment « sels » dudit stack que comporte ledit module ;
- on applique un champ électrique entre la cathode et l'anode dudit stack d'électrodialyse et on forme, par génération et migration d'ions, la solution acide dans un deuxième compartiment, dénommé compartiment « acide » dudit stack que comporte ledit module et la solution basique dans un troisième compartiment, dénommé compartiment « base » dudit stack que comporte ledit module ;

- on fait recirculer la solution d'acide entre le deuxième compartiment « acide » dudit stack et ladite cuve de solution acide et on fait recirculer la solution basique entre le troisième compartiment « base » dudit stack et ladite cuve de solution basique ;
- on achemine la solution d'acide depuis ladite cuve de solution acide et la solution basique depuis ladite cuve de solution basique vers les moyens d'ajustement du pH que comporte le circuit en boucle fermée.
- on renvoie le solvant aqueux du premier compartiment « sels » dudit stack vers le réservoir de solvant aqueux dégazé que comporte avantageusement le circuit en boucle fermée.

La présente invention a encore pour objet une installation selon la revendication 21 de purification d'un flux de gaz entrant comprenant un gaz $G_0$ à purifier et au moins un gaz acide $Ga_1$ et/ou un gaz basique $Gb_1$, à éliminer, pour la mise en oeuvre du procédé de l'invention, comportant, au moins, une unité membranaire d'absorption comprenant au moins une membrane perméable aux gaz et hydrophobe, ladite unité étant reliée, d'un côté de ladite membrane, à une arrivée du flux de gaz entrant, et à une sortie du flux de gaz sortant enrichi en gaz $G_0$ à purifier et, de l'autre côté de ladite membrane, à un circuit fermé dans lequel circule en boucle un solvant aqueux absorbant ledit au moins un gaz à éliminer, ladite installation comportant encore, en aval de ladite unité membranaire d'absorption, en tenant compte du sens de circulation dudit solvant aqueux dans le circuit fermé, au moins une unité membranaire de désorption, laquelle comporte au moins une membrane perméable aux gaz et hydrophobe, apte à permettre le passage d'au moins une partie du gaz à éliminer dissous dans ledit solvant aqueux et son élimination.

Ladite installation de l'invention comporte encore au moins, d'une part, des premiers moyens de régulation du pH du solvant aqueux, positionnés sur le circuit fermé entre ladite au moins une unité membranaire de désorption et ladite unité membranaire d'absorption, en tenant compte du sens de circulation du solvant aqueux au sein dudit circuit fermé et, d'autre part, des deuxièmes moyens de régulation du pH du solvant aqueux positionnés sur le circuit fermé entre l'unité membranaire d'absorption et ladite au moins une unité membranaire de désorption, en tenant compte du sens de circulation du solvant aqueux au sein dudit circuit fermé.

La présente installation est particulière en ce qu'elle comporte au moins un module d'électrodialyse à membrane bipolaire à trois compartiments pour régénérer l'acide et la base utilisés à partir du solvant aqueux dégazé. Ledit module, constitué d'un stack qui consiste en un assemblage, en alternance, de membranes bipolaires et monopolaires, compris entre une cathode et une anode, est relié avantageusement à un réservoir de solvant aqueux dégazé positionné sur ledit circuit fermé, entre ladite au moins une unité membranaire de désorption et l'unité membranaire d'absorption, en tenant compte du sens de circulation du solvant aqueux au sein du circuit fermé, des moyens de recirculation dudit solvant aqueux, entre ledit réservoir et ledit module d'électrodialyse à membrane bipolaire étant également prévus, ainsi que des moyens d'acheminement des solutions acide et basique régénérées depuis, respectivement, une cuve de solution acide et une cuve de solution basique, que comporte ledit module, vers les moyens de régulation du pH.

Dans le procédé de la présente invention, les inventeurs ont réussi à lever la limitation thermodynamique des procédés existants, pour une amélioration substantielle de la pureté du gaz d'intérêt, tout en conservant un procédé peu énergivore et non consommateur de produits chimiques, et qui ne génère pas d'effluents ou de déchets à traiter, autres que les gaz à éliminer.

Cela a été effectué en appliquant, d'une part, une première variation de pH du solvant enrichi en gaz dissous à éliminer, après l'étape d'absorption et avant l'étape de désorption et, d'autre part, une deuxième variation de pH appliquée cette fois sur le solvant dégazé, entre l'étape de désorption qui a permis le recyclage du solvant, et l'étape d'absorption dans laquelle ledit solvant est réutilisé.

Dans le cas où le gaz à éliminer consiste en un gaz acide, ou lorsqu'il y a plusieurs gaz à éliminer et que les gaz acides sont majoritaires, l'ajustement sensible du pH, avant l'étape d'absorption est effectué par ajout de base tandis que, avant l'étape de désorption pour régénérer le solvant aqueux, l'ajustement de pH se fait par ajout d'acide. On procède inversement lorsque le gaz à éliminer est un gaz basique seul ou en proportion majoritaire dans un mélange.

Dans le procédé de l'invention, on joue ainsi, de manière astucieuse, sur les équilibres acido-basiques des gaz dissous afin de favoriser leur absorption puis leur désorption, évitant ainsi la présence résiduelle de gaz dissous après désorption, limitant, par conséquent, la pureté du gaz à valoriser en sortie de l'unité membranaire d'absorption.

La présente invention comporte de nombreux avantages.

Principalement, par la mise en oeuvre du procédé de l'invention, la pureté du gaz d'intérêt à récupérer est substantiellement améliorée par rapport aux procédés conventionnels. La pureté obtenue peut notamment être supérieure à 99% et plus particulièrement encore supérieure à 99,97%, correspondant, par exemple, aux spécifications requises pour que le dihydrogène $H_2$ purifié au moyen du procédé de l'invention puisse être utilisé pour alimenter des piles à combustible.

Par ailleurs, on arrive, de manière particulièrement avantageuse, à de tels niveaux de pureté sans une consommation excessive de réactifs pour l'ajustement du pH, et sans la nécessité d'éliminer les sels produits dans le liquide porteur par une méthode de déminéralisation coûteuse en énergie ou en réactifs, et en évitant, au final, une production de déchets que représente l'effluent salin issu du moyen de déminéralisation. En effet, la mise en oeuvre d'une étape d'électrodialyse à membrane bipolaire permet de régénérer efficacement l'acide et la base qui sont ajoutés pour améliorer la pureté du gaz d'intérêt récupéré, à partir du sel correspondant formé ou déjà présent dans le solvant aqueux.

Ainsi, le procédé conforme à l'invention reste écologique, durable et viable économiquement, même pour des petites unités de méthanisation de biomasse, où l'on cherche à purifier du $CH_4$, ou de pyrogazéification de biomasse, où l'on cherche à purifier du $H_2$.

D'autres caractéristiques et avantages de l'invention ressortiront de la description détaillée qui va suivre des modes de réalisation non limitatifs de l'invention, en référence aux figures annexées dans lesquelles :

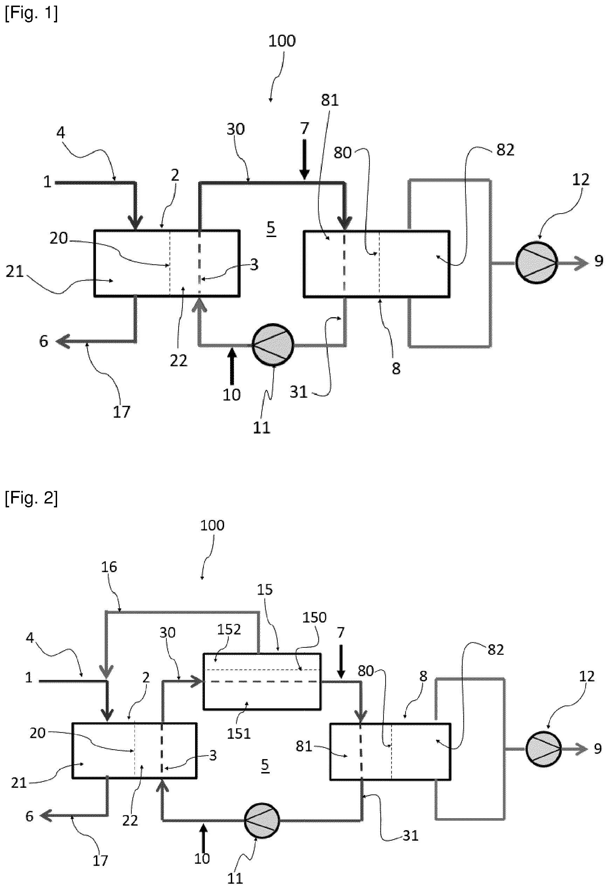

[Fig.1] représente, de manière schématisée, un premier mode de réalisation particulier d'une partie d'une installation selon l'invention pour la purification d'un flux de gaz entrant, pour la mise en oeuvre de certaines étapes du procédé de purification par absorption gaz-liquide selon l'invention, sans le module d'électrodialyse à membrane bipolaire, la partie de cette installation représentée comportant une unité membranaire d'absorption et une unité membranaire de désorption, ainsi que des moyens d'ajustement du pH du solvant aqueux circulant au sein de la boucle fermée reliant lesdites deux unités ;

[Fig.2] illustre, de manière schématisée, un deuxième mode de réalisation particulier d'une partie d'une installation de purification d'un flux de gaz entrant, similaire à celle de la figure 1 avec, en supplément, une unité membranaire de dégazage intermédiaire pour recycler une fraction du (ou des) gaz d'intérêt en partie dissous dans le solvant aqueux au niveau de l'unité membranaire d'absorption ;

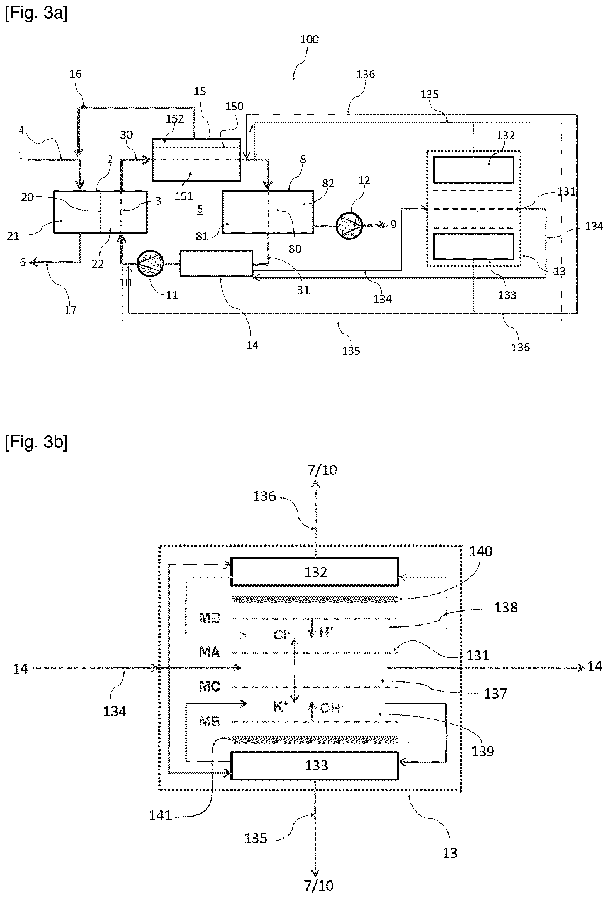

[Fig.3a] représente, de manière schématisée, une installation de purification d'un flux de gaz entrant, comprenant la partie d'installation selon le mode de réalisation de la figure 2, complétée par un module d'électrodialyse à membrane bipolaire pour régénérer l'acide et la base injectés au niveau des moyens d'ajustement du pH du solvant aqueux ;

[Fig.3b] illustre, schématiquement, en détails une cellule du module d'électrodialyse à membrane bipolaire à trois compartiments avec décomposition des flux d'ions et de liquides au sein de ladite cellule, MA signifiant « membrane anionique », MB signifiant « membrane bipolaire » et MC signifiant « membrane cationique », et dans le cas où l'acide consiste en de l'acide chlorhydrique HCl et la base consiste en de l'hydroxyde de potassium KOH ;

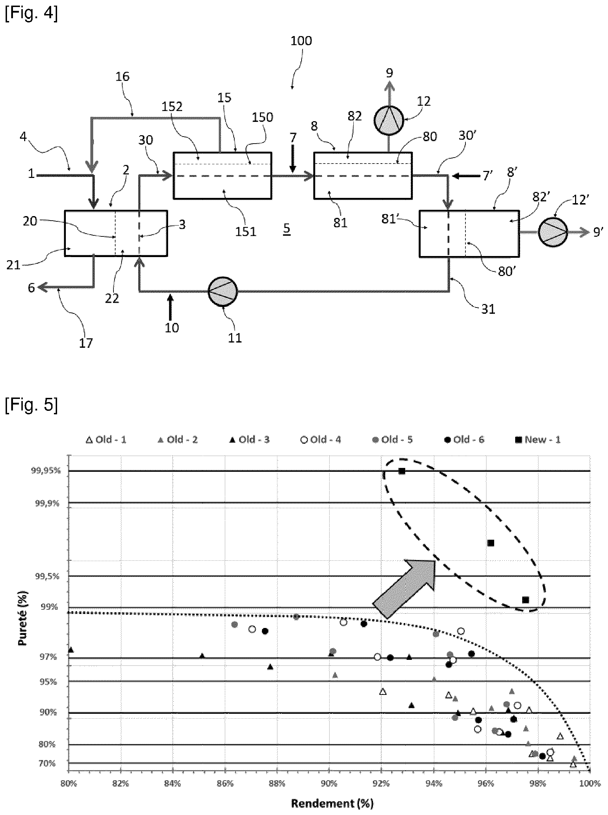

[Fig.4] représente, de manière schématisée, un troisième mode de réalisation d'une partie d'une installation particulièrement adaptée à la purification d'un flux de gaz entrant comprenant un gaz à purifier et un mélange de gaz acides et basiques à éliminer, ladite installation comprenant alors deux unités membranaires de désorption, en plus de l'unité membranaire d'absorption et de l'unité membranaire de dégazage intermédiaire, le pH du solvant aqueux pouvant alors être ajusté à trois endroits différents du circuit en boucle fermée reliant les différentes unités, le module d'électrodialyse à membrane bipolaire n'étant pas représenté sur cette figure ;

[Fig.5] correspond à une représentation graphique illustrant une comparaison des performances obtenues expérimentalement, en termes à la fois de rendement de gaz purifié, exprimé en pourcentage et représenté en abscisses, et de pureté, exprimée en pourcentage et représentée en ordonnées, du procédé de purification d'un flux de gaz entrant selon la présente invention (série « New-1 », résultats représentés par des carrés noirs), d'un procédé classique de purification de gaz par absorption-désorption (séries « Old-1 » à « Old-5 ») et du procédé objet de la demande EP 3 372 297 (série « Old-6 », le solvant absorbant consistant en une solution de KCl concentrée à 1 mol.L$^{-1}$), différentes conditions opératoires ayant été testées expérimentalement, dans le cas où le gaz à purifier consiste en du diazote $N_2$, comparable au dihydrogène $H_2$ (solubilité très faible) et le gaz à éliminer consiste en un gaz acide, le dioxyde de carbone $CO_2$ ;

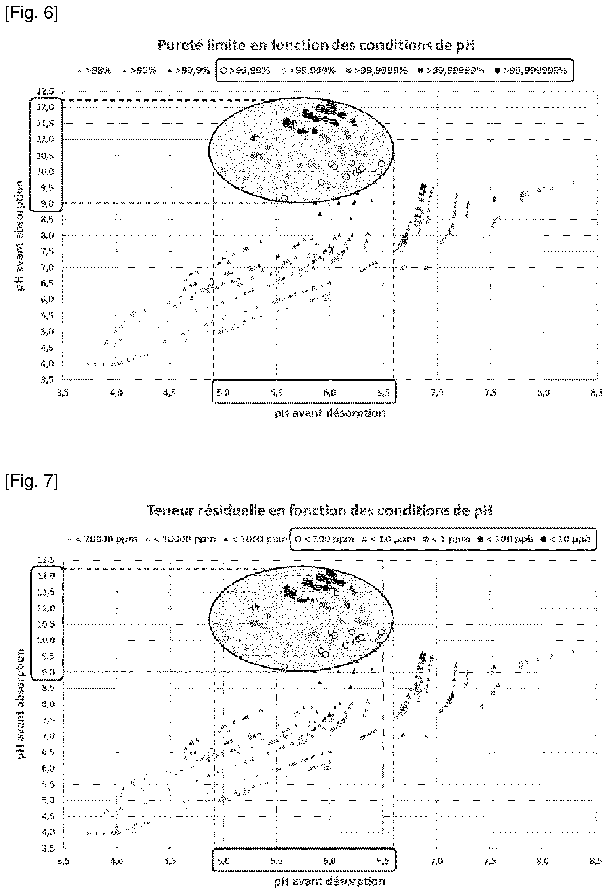

[Fig.6] correspond à une représentation graphique illustrant la pureté limite théorique du gaz purifié en fonction des conditions de pH (basiques) dans le solvant aqueux avant absorption (en ordonnées) et en fonction des conditions de pH (acides) dans le solvant aqueux avant désorption (en abscisses), dans le cas où le gaz à purifier consiste en du $H_2$ et le gaz à éliminer consiste en du $CO_2$, le mélange traité correspondant à un syngaz enrichi par « Water Gas Shift » et composé de 36,5% de $CO_2$ et de 63,5% de $H_2$, et dans l'hypothèse où le solvant aqueux absorbant est régénéré par une étape de désorption sous un vide qualifié de vide grossier et dénommé aussi vide industriel ;

[Fig.7] consiste en une représentation graphique illustrant, après mise en oeuvre du procédé de l'invention, la teneur résiduelle théorique en gaz à éliminer ($CO_2$) du gaz purifié en fonction des conditions de pH (basiques) dans le solvant aqueux avant absorption (en ordonnées) et en fonction des conditions de pH (acides) dans le solvant aqueux avant désorption (en abscisses), dans des conditions identiques à celles décrites pour la figure 6 ;

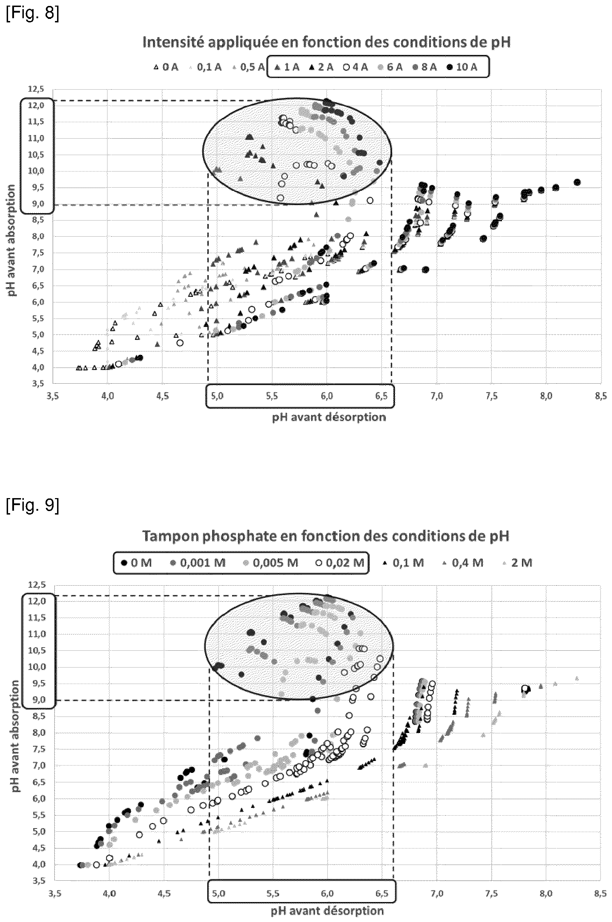

[Fig.8] consiste en une représentation graphique illustrant l'intensité appliquée (ajustement de pH), correspondant aux différents points de fonctionnement testés, dans des conditions identiques à celles décrites pour la figure 6 ;

[Fig.9] correspond à une représentation graphique illustrant les différentes concentrations en tampon phosphate dans le solvant aqueux, correspondant aux différents points de fonctionnement testés, en fonction des conditions de pH dans le solvant aqueux avant absorption (en ordonnées) et en fonction des conditions de pH (acides) dans le solvant aqueux avant désorption (en abscisses) dans des conditions identiques à celles décrites pour la figure 6 ;

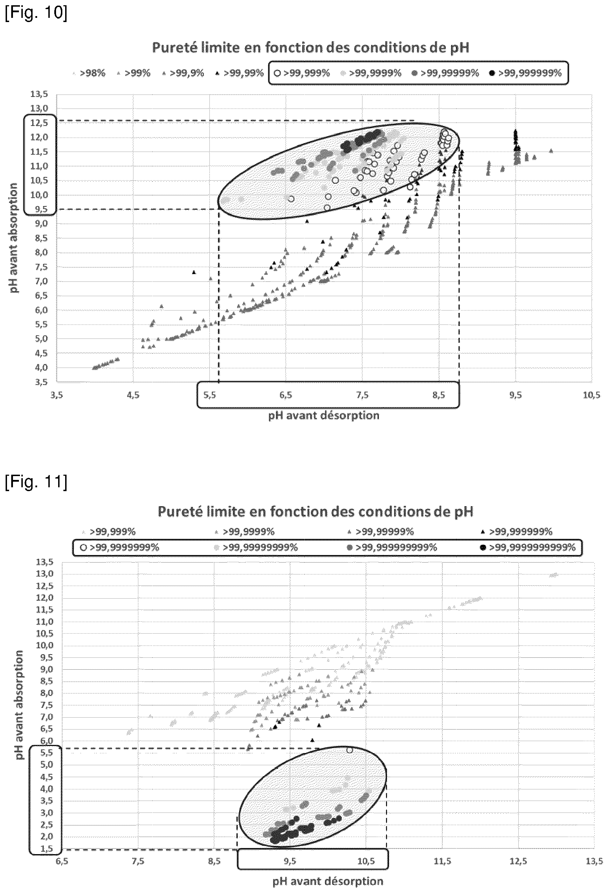

[Fig.10] correspond à une représentation graphique illustrant la pureté limite théorique du gaz purifié en fonction des conditions de pH (basiques) dans le solvant aqueux avant absorption (en ordonnées) et en fonction des conditions de pH (acides) dans le solvant aqueux avant désorption (en abscisses), dans le cas où le gaz à purifier consiste en du $H_2$ et le gaz à éliminer consiste en du $H_2S$, un gaz acide, le mélange traité étant composé de 1% de $H_2S$, et dans l'hypothèse où le solvant aqueux absorbant est régénéré par une étape de désorption sous un vide grossier, l'objectif étant d'atteindre une teneur en $H_2S$ inférieure à 10 ppm (partie par million), soit moins de 0,001 % ;

[Fig.11] correspond à une représentation graphique illustrant la pureté limite théorique du gaz purifié en fonction des conditions de pH (acides) dans le solvant aqueux avant absorption (en ordonnées) et en fonction des conditions de pH (basiques) dans le solvant aqueux avant désorption (en abscisses), dans le cas où le gaz à purifier consiste en du $H_2$ et le gaz à éliminer consiste en du NHs, un gaz basique, le mélange traité étant composé de 0,01% de NHs, et dans l'hypothèse où le solvant aqueux absorbant est régénéré par une étape de désorption sous un vide grossier, l'objectif étant d'atteindre une teneur en $NH_3$ inférieure à 1 ppb (partie par milliard), soit moins de 0,0000001 %.

En référence aux figures des dessins ci-joints, et plus particulièrement aux figures 1 à 4 dans un premier temps, la présente invention est relative à un procédé de purification, par absorption gaz-liquide, d'un flux de gaz entrant 1.

Ce flux de gaz entrant 1 comprend, avantageusement, un gaz $G_0$ à purifier et au moins un gaz à éliminer, noté $G_1$ de manière générale, qu'il s'agisse d'un seul gaz, acide ou basique, ou d'un mélange de plusieurs gaz à éliminer.

Les proportions initiales (v/v) du gaz $G_0$ à purifier et dudit au moins un gaz $G_1$ à éliminer au sein du flux de gaz entrant 1 peuvent être très variables et certains exemples seront donnés ci-après dans la description.

De préférence, mais non limitativement, le gaz $G_0$ à purifier est majoritaire au sein du flux de gaz entrant. Toutefois, selon la solubilité respective des gaz, la proportion de gaz $G_0$ au sein du flux entrant peut être inférieure à celle dudit au moins un gaz $G_1$ à éliminer. De manière générale, le ratio entre la teneur de $G_0$ et $G_1$ doit être supérieur au ratio entre leur solubilité respective.

Le gaz $G_0$ à purifier peut consister, par exemple mais non limitativement, en du méthane $CH_4$, dans l'optique notamment d'une injection dans un réseau de gaz, ou en du dihydrogène $H_2$, pour une utilisation dans des applications « mobilité », notamment pour alimenter des piles à combustibles.

En plus de ce gaz $G_0$ à purifier, et comme indiqué ci-dessus, le flux de gaz entrant 1 comporte une certaine proportion de gaz indésirable $G_1$ qu'il convient d'éliminer par la mise en oeuvre du présent procédé ; celle-ci peut consister soit en un gaz acide $Ga_1$, soit en un gaz basique $Gb_1$, soit en un mélange comportant au moins un gaz acide $Ga_1$ et au moins un gaz basique $Gb_1$, ledit au moins un gaz acide $Ga_1$ étant majoritaire, ou bien encore en un mélange comportant au moins un gaz acide $Ga_1$ et au moins un gaz basique $Gb_1$, ledit au moins un gaz basique $Gb_1$ étant majoritaire.

Il est entendu que, lorsque ledit flux de gaz entrant 1 comporte au moins un gaz acide $Ga_1$ et un gaz basique $Gb_1$, il peut s'agir de plusieurs gaz acides $Ga_1$, $Ga_2$, $Ga_n$ et/ou de plusieurs gaz basiques $Gb_1$, $Gb_2$, $Gb_n$.

Le flux de gaz entrant 1 peut également comporter, en plus gaz $G_0$ à purifier, en mélange, plusieurs gaz acides $Ga_1$, $Ga_2$, $Ga_n$, sans inclure de gaz basique ou, inversement, plusieurs gaz basiques $Gb_1$, $Gb_2$, $Gb_n$, sans contenir de gaz acide.

Le gaz acide $Ga_1$ à éliminer peut consister en du dioxyde de carbone $CO_2$ ou en du sulfure d'hydrogène $H_2S$. Comme indiqué précédemment, le flux de gaz entrant 1 peut également comporter un mélange de $CO_2$ ($Ga_1$) et de $H_2S$ ($Ga_2$) à éliminer, et éventuellement au moins encore un autre gaz acide et/ou au moins un gaz basique $Gb_1$.

Le gaz basique $Gb_1$ à éliminer peut consister en de l'ammoniac $NH_3$, seul ou en mélange avec au moins un autre gaz basique $Gb_2$ et/ou au moins un gaz acide $Ga_1$, tels que ceux susmentionnés.

Dans une application particulière du procédé de l'invention, le flux de gaz entrant 1 consiste en du biogaz, comprenant, au moins, en tant que gaz $G_0$ à purifier, du méthane $CH_4$, dans une proportion généralement comprise entre 45 et 70% en volume par rapport au volume total de biogaz, et du $CO_2$ en tant que gaz acide $Ga_1$ à éliminer, dans une proportion généralement comprise entre 25 et 50% (v/v).

Le biogaz peut également comporter d'autres gaz indésirables solubles à éliminer, comme le sulfure d'hydrogène $H_2S$, ou des gaz peu solubles tels que le dioxygène $O_2$ ou bien encore le diazote $N_2$, présents dans des proportions toutefois substantiellement plus faibles que les proportions en $CH_4$ et en $CO_2$.

Selon la teneur et la solubilité de ces gaz à éliminer, le procédé de l'invention peut éventuellement être couplé à une ou plusieurs technique(s) d'épuration spécifique(s) à ces impuretés, en particulier si leur solubilité est trop faible dans le solvant aqueux utilisé (notamment dans le cas de $O_2$ et $N_2$ à éliminer).

Il est également envisageable que le flux de gaz entrant 1 à traiter au moyen du procédé de l'invention consiste en du syngaz obtenu suite à une pyrogazéification de déchets, ou thermolyse, et craquage, suivis d'une réaction du gaz à l'eau ou réaction de « Water Gaz Shift », avec ou sans catalyseur. Suivant ces techniques d'épuration préliminaires, le syngaz comporte alors majoritairement du dihydrogène $H_2$ en tant que gaz $G_0$ à purifier, dans une proportion comprise entre 50 et 75%, de préférence de l'ordre de 63,5% (v/v), et du $CO_2$, en tant que gaz acide $Ga_1$ à éliminer, dans une proportion comprise entre 25 et 50%, de préférence de l'ordre de 36,5% (v/v).

A noter que toutes les teneurs en gaz exprimées dans la présente demande sont exprimées en « gaz sec », autrement dit sans tenir compte de l'eau, sous forme de vapeur, qui peut être présente dans le flux de gaz entrant et qui est facilement éliminée.

Le syngaz peut également comporter d'autres impuretés gazeuses peu solubles à éliminer, comme du $CH_4$ et du CO, et éventuellement des traces de $O_2$ et de $N_2$, par exemple. Celles-ci sont toutefois présentes dans de très faibles proportions, tout comme pour les impuretés du biogaz. Finalement, la proportion de l'ensemble des impuretés est généralement inférieure à 1% (v/v) dans le flux de gaz entrant 1 à traiter, après l'application des techniques d'épuration préliminaires susmentionnées. En effet, selon leur teneur résiduelle, le procédé de l'invention peut éventuellement être couplé à une ou plusieurs technique(s) d'épuration spécifique(s) à ces impuretés, en particulier si leur solubilité est trop faible dans le solvant aqueux utilisé.

De manière générale, le procédé de purification, par absorption gaz-liquide, d'un flux de gaz entrant 1 permet de séparer des gaz en jouant sur leur différence de solubilité dans un solvant aqueux absorbant le(s) gaz du mélange qui est (sont) le(s) plus soluble(s).

Ainsi, le procédé de l'invention a pour objectif, à partir d'un flux de gaz entrant 1 comprenant un gaz $G_0$ à purifier et au moins un gaz $G_1$ à éliminer, de séparer ledit gaz $G_0$ à purifier, celui-ci présentant une Constante de Henry $H_{G0}$ inférieure à $10^{-2}$ mol.$L^{-1}$.bar$^{-1}$, tandis que ledit gaz $G_1$ à éliminer présente une Constante de Henry $H_{G1}$ supérieure à $10^{-2}$ mol.$L^{-1}$.bar$^{-1}$.

En d'autres termes, le gaz $G_0$ à purifier est considéré comme peu soluble dans un solvant aqueux tandis que le gaz $G_1$ à éliminer est considéré comme étant soluble dans un tel solvant, qu'il s'agisse d'un gaz acide $Ga_1$ (avec une Constante de Henry $H_{Ga1}$ supérieure à $10^{-2}$ mol.$L^{-1}$.bar$^{-1}$), d'un gaz basique $Gb_1$ (également avec une Constante de Henry $H_{Gb1}$ supérieure à $10^{-2}$ mol.$L^{-1}$.bar$^{-1}$) ou d'un mélange de gaz avec chacun une Constante de Henry supérieure à $10^{-2}$ mol.$L^{-1}$.bar$^{-1}$.

Notons encore que, de manière générale, le gaz $G_1$ à éliminer, ou chacun des gaz $G_1$ à éliminer, présente une constante d'acidité $pKa_{G1}$ supérieure ou égale à 1 et inférieure ou égale à 13.

Quelle que soit la composition du flux de gaz entrant 1 à traiter, la première étape du procédé d'épuration de l'invention consiste en une étape d'absorption dudit au moins un gaz acide $Ga_1$ soluble et/ou dudit au moins un gaz basique soluble $Gb_1$, par un solvant aqueux 3, au niveau d'une unité membranaire d'absorption 2.

Cette unité membranaire d'absorption 2 comporte, au moins, une membrane 20, préférentiellement microporeuse et hydrophobe, et perméable aux gaz, tandis qu'elle est hydrophobe, pour éviter le passage des liquides.

Une telle membrane 20 pourra ainsi, par exemple, être fabriquée à partir de polypropylène (PP), de polyfluorure de vinylidène (PVDF), ou encore de polytétrafluoroéthylène (PTFE).

A noter que, plus particulièrement, le flux de gaz entrant 1 à traiter entre, au niveau de ladite unité membranaire d'absorption 2, au moyen d'une arrivée 4, et se retrouve d'un côté 21 de ladite membrane 20 perméable aux gaz et hydrophobe.

De l'autre côté 22 de cette membrane 20 circule le solvant aqueux 3 dans un circuit en boucle fermée 5, dans lequel ledit solvant 3 est avantageusement maintenu à une pression relative comprise entre 1 et 7 bars, plus avantageusement encore à une pression relative comprise entre 5 et 6 bars, grâce, par exemple à une pompe de recirculation 11, en maintenant une perte de charge minimum pour limiter la consommation énergétique du procédé.

A noter encore que, de préférence, le solvant aqueux 3 circule, au sein de la boucle fermée 5, à contre-courant par rapport au flux de gaz entrant 1, comme illustré sur les figures 1 à 4.

Ledit solvant aqueux 3 est apte à absorber le(s) gaz soluble(s) acide(s) $Ga_1$ et/ou basique(s) $Gb_1$ présent(s) dans ledit flux de gaz entrant 1, en particulier le(s) gaz dont la constante de Henry est supérieure à $10^{-2}$ mol.$L^{-1}$.bar$^{-1}$.

Ainsi, dans cette première étape d'absorption du procédé de l'invention, le gaz $G_1$ indésirable à éliminer du flux de gaz entrant 1, ainsi que les autres impuretés gazeuses éventuelles à éliminer et suffisamment solubles (c'est à dire avec une Constante de Henry supérieure à $10^{-2}$ mol.$L^{-1}$.bar$^{-1}$) traverse(nt) la membrane 20 pour venir se dissoudre dans le solvant aqueux 3.

On obtient ainsi un flux 6 de gaz purifié, enrichi en gaz $G_0$ à purifier, par exemple en $H_2$ (Constante de Henry égale à $7,6*10^{-4}$ mol.$L^{-1}$.bar$^{-1}$ à 25°C) dans le cas où le gaz de départ 1 consiste en du syngaz, ou en $CH_4$ (Constante de Henry égale à $1,3*10^{-3}$ mol.$L^{-1}$.bar$^{-1}$ à 25°C) lorsque le gaz entrant 1 est un flux de biogaz, et très substantiellement appauvri en gaz indésirable(s) soluble(s) à éliminer, par rapport au flux entrant 1.

Plus particulièrement, au moyen du procédé de l'invention, on obtient, d'un côté, un flux de gaz purifié 6 d'une pureté supérieure à 99% (v/v) en gaz $G_0$ à purifier et, encore plus préférentiellement, un flux 6 d'une pureté supérieure à 99,99%.

A la lecture de l'intégralité de la description, les raisons permettant d'aboutir à une telle pureté ainsi que les autres avantages et caractéristiques particulières du procédé objet de l'invention apparaîtront plus clairement.

D'un autre côté, lorsque le(s) gaz indésirable(s) se dissout/dissolvent dans le solvant aqueux 3, on obtient, en sortie de l'unité membranaire d'absorption 2, un solvant aqueux riche 30 comprenant lesdits gaz dissous.

Au niveau de cette unité membranaire d'absorption 2, le flux de gaz entrant est avantageusement maintenu à une pression relative comprise entre 10 mbars et 6 bars, cette pression étant plus avantageusement encore comprise entre 4,5 et 5,75 bars et, tout préférentiellement, égale à 5 bars.

Dans tous les cas, de manière préférentielle, la différence de pression entre la pression du solvant aqueux absorbant 3 circulant dans le circuit en boucle fermée 5, comprise entre 1 et 7 bars, et la pression du flux de gaz 1 à l'absorption, est comprise entre 0,2 et 1 bar.

Un tel différentiel de pression permet d'éviter le mélange des phases gaz et liquide par bullage du gaz dans le solvant 3 tout en limitant l'humidification des pores et la consommation énergétique.

En tant que solvant aqueux 3, on utilisera préférentiellement une solution de saumure concentrée, autrement dit une solution de chlorure de sodium NaCl concentrée entre 0,2 et 6 mol.L$^{-1}$, obtenue par dissolution de la quantité adéquate de NaCl dans de l'eau, cette dernière consistant avantageusement en de l'eau osmosée.

Une eau osmosée est une eau filtrée au travers d'une membrane d'osmose inverse, permettant de retenir les ions et les molécules de taille relativement importante. L'eau osmosée présente une conductivité inférieure à 25 pS.cm$^{-1}$, et, plus préférentiellement encore, une conductivité inférieure à 20 pS.cm$^{-1}$. Une telle eau est particulièrement avantageuse dans le procédé de l'invention car elle est notamment dépourvue d'ions $Ca^{2+}$ et $Mg^{2+}$, limitant ainsi le risque de précipitation des ions carbonates dans les unités membranaires d'absorption et de désorption, et le risque de précipitation des ions hydroxydes formés au niveau des membranes bipolaires du module d'électrodialyse.

Un solvant aqueux 3 à base de NaCl est particulièrement avantageux car il permet de réduire la solubilité du gaz d'intérêt $G_0$ à purifier, par exemple $CH_4$ ou $H_2$, et donc d'améliorer le rendement de récupération dudit gaz d'intérêt dans le flux de gaz sortant 6.

Le solvant aqueux 3 peut également consister en une solution de chlorure de potassium KCl, de sulfate de sodium $Na_2SO_4$, ou bien encore de sulfate de potassium $K_2SO_4$ dissous dans de l'eau, de préférence de l'eau osmosée, la concentration molaire finale en sel dans le solvant aqueux absorbant 3 étant comprise entre 0,2 et 6 mol.L$^{-1}$, selon la solubilité du sel.

Plus préférentiellement encore, la concentration molaire en sel dans le solvant aqueux absorbant 3 est comprise entre 1 et 2 mol.L$^{-1}$.

Cependant, il est tout à fait envisageable d'utiliser d'autres sels présentant une bonne solubilité, peu corrosifs et avec peu ou pas d'effet tampon (base très faible avec un pKa inférieur à 2 ou acide très faible avec un pKa supérieur à 12) tels que les nitrates, bromures et iodures de sodium ou potassium, ou les sels de lithium (chlorure, bromure, iodure, sulfate et nitrate).

Suivant cette première étape d'absorption du procédé de l'invention, le solvant aqueux 30, enrichi en gaz indésirable(s) dissous, circule dans le circuit en boucle fermée 5 pour être acheminé au niveau d'au moins une unité membranaire de désorption 8.

Le solvant aqueux riche 30 est alors mis au contact d'une membrane 80 que contient ladite unité de désorption 8, et qui est perméable aux gaz et hydrophobe, en sorte d'éviter le passage du solvant aqueux 30 au travers de celle-ci.

Au niveau de cette unité membranaire 8, lors d'au moins une étape de désorption, on va éliminer la quasi-totalité du ou des gaz indésirable(s) $G_1$ dissous, notamment le $CO_2$ dissous dans le cas de l'épuration du biogaz ou du syngaz, celui-ci traversant ladite membrane 80.

Pour procéder au dégazage du ou des gaz indésirable(s) $G_1$ dissous dans le solvant 30, on applique avantageusement une dépression transmembranaire au niveau de la membrane 80 de l'unité de désorption 8, préférentiellement au moyen d'une pompe à vide 12.

En ce qui concerne la membrane 80, microporeuse et hydrophobe, celle-ci pourra par exemple être fabriquée à partir de PP, de PVDF ou de PTFE, tout comme la membrane 20 que comporte l'unité membranaire d'absorption 2 et décrite en détail précédemment.

Plus précisément, au cours de l'étape de désorption, le solvant aqueux 30 riche en gaz indésirable(s) $G_1$ dissous est amené par le circuit 5 au niveau de l'unité de désorption 8, d'un côté 81 de la membrane 80 que comporte ladite unité 8, et ledit gaz $G_1$ va être éliminé du solvant, par l'application d'une dépression transmembranaire entrainant un passage dudit gaz $G_1$ de l'autre côté 82 de la membrane 80.

Plus particulièrement, on applique une pression absolue, du côté 82 de la membrane 80 de sortie des gaz dissous 9, comprise entre 25 mbar et 1 bar, de préférence entre 50 et 100 mbar, par exemple au moyen d'une pompe à vide 12 pour un compromis entre une élimination optimale des gaz, tout en limitant la consommation d'énergie. Une telle pression correspond à l'application, au niveau de l'unité de désorption 8, d'un vide qualifié de grossier, ou d'industriel, et dont la fourchette de pression est communément définie comme étant comprise entre 1 mbar et 1 bar.

Suivant cette étape de désorption, on obtient alors, d'une part un flux de gaz sortant 9 ou « off gaz », destiné à être éliminé (par exemple du $CO_2$ dans le cas de la purification de biogaz ou de syngaz), et, d'autre part, un solvant aqueux dégazé 31, pouvant également être dénommé solvant recyclé, qui va être acheminé, toujours au moyen du circuit en boucle fermée 5, vers l'unité membranaire d'absorption 2 pour une nouvelle étape d'absorption de gaz $G_1$ indésirable(s) présent(s) dans le flux de gaz entrant 1.

Ainsi, au cours du procédé d'épuration d'un flux de gaz entrant 1 selon l'invention, les étapes d'absorption et de désorption des gaz sont opérées en continu en boucle fermée pour permettre une purification de ce flux de gaz entrant 1, en mettant en circulation ledit solvant aqueux 3, 30, 31 dans la boucle de circulation fermée 5, dans laquelle ledit solvant 3, 30, 31 est entraîné, par exemple, au travers de la pompe de recirculation 11.

Il y a donc une étape d'absorption qui fonctionne en continu en boucle fermée avec le solvant recyclé après une étape de désorption fonctionnant elle-même en continu. En d'autres termes, et bien qu'une certaine séquentialité des étapes d'absorption et de désorption puisse être évoquée dans la suite de la description, il convient de considérer que, selon la présente invention, les différentes étapes d'absorption et de désorption, dans le procédé de l'invention, sont mises en oeuvre en série et fonctionnent simultanément en boucle fermée.

A noter que le flux de gaz sortant 9 est potentiellement valorisable. Par exemple dans le cas du $CO_2$, celui-ci peut être valorisé dans le domaine de la production de microalgues, ou être épuré pour une valorisation dans l'industrie chimique ou agro-alimentaire. Aussi, un dégazage sous vide grossier est privilégié, par rapport à un stripping à l'air, pour éviter de diluer le flux de gaz sortant dans de l'air, et permettre une valorisation potentielle.

Dans l'hypothèse où le procédé de purification d'un flux de gaz entrant 1 est mis en oeuvre dans le but de purifier un gaz $G_0$ en éliminant, dudit flux 1, un mélange de gaz comprenant à la fois au moins un gaz acide $Ga_1$ et au moins un gaz basique $Gb_1$, l'un des deux gaz à éliminer étant présent en majorité par rapport à l'autre, plusieurs étapes de désorption peuvent être appliquées entre deux étapes d'absorption,

Notons ici que, lorsque le flux de gaz entrant 1 consiste en un mélange comportant un seul gaz acide $Ga_1$ et un seul gaz basique $Gb_1$ à éliminer, on considère que le gaz à éliminer qui est présent en majorité dans le flux entrant 1 est celui pour lequel le rapport entre son volume (noté $V_{Ga1}$ pour le gaz acide et $V_{Gb1}$ pour le gaz basique) et le volume total de gaz à éliminer ($V_{Ga1} + V_{Gb1}$) est le plus grand.

En d'autres termes, si le rapport $V_{Ga1}/(V_{Ga1} + V_{Gb1})$ est supérieur à $V_{Gb1}/(V_{Ga1} + V_{Gb1})$, on considérera que le gaz acide $Ga_1$ est le gaz majoritaire à éliminer du flux de gaz entrant 1. Au contraire, si le rapport $V_{Gb1}/(V_{Ga1} + V_{Gb1})$ est supérieur au rapport $V_{Ga1}/(V_{Ga1} + V_{Gb1})$, le gaz basique $Gb_1$ est considéré comme étant le gaz majoritaire à éliminer dans le flux de gaz entrant 1.

Lorsque le flux de gaz entrant 1 consiste en un mélange comportant un seul gaz acide $Ga_1$ et au moins deux gaz basiques $Gb_1$, $Gb_2$ ... $Gb_n$ à éliminer, on considère que le gaz acide $Ga_1$ à éliminer est présent en majorité dans le flux entrant 1 si le rapport entre son volume $V_{Ga1}$ et le volume total de gaz à éliminer ($V_{Ga1} + V_{Gb1} + V_{Gb2} + V_{Gbn}$) est supérieur au rapport entre la somme totale du volume des gaz basiques à éliminer ($V_{Gb1} + V_{Gb2} + V_{Gbn}$) et le volume total de gaz à éliminer ($V_{Ga1} + V_{Gb1} + V_{Gb2} + V_{Gbn}$) du flux entrant 1, et inversement.

Autrement dit, si $V_{Ga1}/(V_{Ga1} + V_{Gb1} + V_{Gb2} + V_{Gbn})$ est supérieur à $(V_{Gb1} + V_{Gb2} + V_{Gbn})/(V_{Ga1} + V_{Gb1} + V_{Gb2} + V_{Gbn})$ alors le gaz acide $Ga_1$ est considéré comme étant le gaz majoritaire à éliminer du flux entrant 1. Inversement, dans le cas où $(V_{Gb1} + V_{Gb2} + V_{Gbn})/(V_{Ga1} + V_{Gb1} + V_{Gb2} + V_{Gbn})$ est supérieur à $V_{Ga1}/(V_{Ga1} + V_{Gb1} + V_{Gb2} + V_{Gbn})$, les gaz basiques sont les gaz majoritaires à éliminer dans le flux 1.

Le raisonnement est similaire dans le cas où le flux de gaz entrant 1 consiste en un mélange comportant un seul gaz basique $Gb_1$ et au moins deux gaz acides $Ga_1$, $Ga_2$ ... $Ga_n$ à éliminer.

Enfin, si le flux de gaz entrant 1 consiste en un mélange comportant, à la fois, au moins deux gaz acides $Ga_1$, $Ga_2$ ... $Ga_n$ et au moins deux gaz basiques $Gb_1$, $Gb_2$ ... $Gb_n$ à éliminer : si le rapport $(V_{Ga1} + V_{Ga2} + V_{Gan})/((V_{Ga1} + V_{Ga2} + V_{Gan} + V_{Gb1} + V_{Gb2} + V_{Gbn})$ est supérieur au rapport $(V_{Gb1} + V_{Gb2} + V_{Gbn})/((V_{Ga1} + V_{Ga2} + V_{Gan} + V_{Gb1} + V_{Gb2} + V_{Gbn})$, les gaz acides sont les gaz majoritaires à éliminer dans le flux entrant 1.

A l'inverse, si le rapport $(V_{Ga1} + V_{Ga2} + V_{Gan})/((V_{Ga1} + V_{Ga2} + V_{Gan} + V_{Gb1} + V_{Gb2} + V_{Gbn})$ est inférieur au rapport $(V_{Gb1} + V_{Gb2} + V_{Gbn})/((V_{Ga1} + V_{Ga2} + V_{Gan} + V_{Gb1} + V_{Gb2} + V_{Gbn})$, ce sont les gaz basiques qui sont les gaz majoritaires à éliminer dans le flux entrant 1.

Cela étant, ce mode de réalisation où deux étapes de désorption sont appliquées est, plus particulièrement, illustré sur la figure 4 des dessins ci-joints.

Dans cette hypothèse, lors d'une première étape de désorption, on met en contact le solvant aqueux riche 31 comportant ledit au moins un gaz acide $Ga_1$ dissous et ledit au moins gaz basique $Gb_1$ dissous avec une première unité membranaire de désorption 8 comprenant au moins une membrane 80, perméable aux gaz et hydrophobe, et on élimine, par application d'une dépression transmembranaire, dudit solvant aqueux riche 31 circulant d'un côté 81 de ladite membrane 80, ledit gaz ou mélange de gaz, acide ou basique, qui est présent en majorité dans le flux entrant 1.

Le(s) gaz sont éliminés par passage au travers de ladite membrane 80 vers l'autre côté 82 de celle-ci, pour obtenir, d'une part, un solvant aqueux 30' riche en gaz ou en mélange de gaz, acide ou basique, présent au départ en minorité dans le flux de gaz entrant 1. D'autre part, on obtient un premier flux de gaz sortant 9 ou « off gaz », destiné à être éliminé ou valorisé, et comprenant le(s) gaz présent(s) majoritairement dans le flux de gaz entrant 1.

Ensuite, lors d'une deuxième étape de désorption, on met en contact ledit solvant aqueux 30' riche en gaz, acide(s) ou basique(s), minoritaire(s) dans le flux de gaz entrant 1, avec une deuxième unité membranaire de désorption 8'.

Cette deuxième unité membranaire de désorption 8' est similaire à la première unité de désorption 8 décrite précédemment. Elle comprend notamment au moins une membrane 80', perméable aux gaz et hydrophobe.

Au cours de cette deuxième étape de désorption, on élimine, toujours par application d'une dépression transmembranaire, du solvant aqueux riche en gaz minoritaire(s) dissous 30', circulant d'un côté 81' de ladite membrane 80', le(s) gaz dissous. Ceux-ci passent au travers de la membrane 80' vers l'autre côté 82' de celle-ci, pour obtenir un solvant aqueux dégazé 31 d'une part, et un second flux de gaz sortant 9' ou « off gaz ».

Ce second flux de gaz sortant 9' comporte le(s) gaz présent(s) minoritairement dans le flux de gaz entrant 1.

A noter que, de manière avantageuse, pour procéder aux deux étapes de désorption et éliminer, dans un premier temps, le(s) gaz présent(s) en majorité puis le(s) gaz présent(s) en minorité dans le flux entrant 1, on applique une pression absolue comprise entre 25 mbar et 1 bar, de préférence entre 50 et 100 mbar, au niveau des membranes 80, 80', respectivement des unités de désorption 8, 8' au moyen respectivement des pompes à vide 12, 12'.

Suivant les deux étapes de désorption, on recycle le solvant aqueux dégazé 31 à l'étape d'absorption, et ces étapes sont opérées en continu au sein de la boucle fermée 5, pour permettre la purification de ce flux de gaz 1 comprenant à la fois au moins un gaz acide $Ga_1$ et au moins un gaz basique $Gb_1$, en plus du gaz $G_0$ à purifier.

Quelle que soit la composition du flux de gaz entrant 1, selon une particularité du présent procédé de purification d'un gaz, on procède, avant l'étape d'absorption et avant l'étape de désorption, à un ajustement du pH du solvant aqueux circulant dans la boucle fermée 5.

Ces étapes d'ajustement du pH sont mises en oeuvre dans l'optique d'optimiser l'extraction des gaz indésirables $G_1$ dans le solvant au moment de l'absorption, puis leur dégazage au moment de la désorption, et ainsi le recyclage du solvant aqueux riche 30 en solvant aqueux dégazé 31, ce qui a pour conséquence de permettre une amélioration substantielle de la pureté du gaz $G_0$ récupéré.

Plus précisément, suivant l'étape d'absorption de gaz $G_1$ dans ledit solvant aqueux 3 et l'obtention d'un solvant aqueux enrichi 30 en gaz $G_1$ à éliminer dissous, et préalablement l'étape de désorption, on va ajuster, de manière sensible, le pH dudit solvant aqueux riche 30. L'ajustement de pH dudit solvant 30 est réalisée en ajoutant, au travers de moyens 7 adaptés, un certain volume d'une solution acide ou basique.

De même, suivant cette désorption, et préalablement à l'étape d'absorption au cours de laquelle on recycle le solvant aqueux dégazé 31, on ajuste à nouveau sensiblement le pH, par ajout d'un volume adéquat de solution acide ou basique au travers de moyens 10 adaptés.

L'ajustement de pH du solvant aqueux auquel on procède dépend, principalement, d'une part, de la composition du flux de gaz entrant 1, et notamment de la nature du gaz soluble à éliminer, lorsqu'un seul gaz est à éliminer, à savoir acide $Ga_1$ ou basique $Gb_1$, et de sa constante d'acidité $pKa_{Ga1}$ ou $pKa_{Gb1}$, respectivement.

Lorsque plusieurs gaz sont à éliminer, différentes étapes de désorption peuvent être mises en oeuvre, comme évoqué précédemment, en référence à la figure 4. Dans ce cas, avant chacune des étapes de désorption, un ajustement du pH sera appliqué et cet ajustement de pH dépendra, entre autres, de la nature, acide ou basique, du gaz présent majoritairement au sein du mélange que l'on souhaite éliminer et, d'autre part, de la constante d'acidité de ce(s) gaz.

Les différents scénarios du procédé de l'invention, selon la composition de départ du flux de gaz entrant 1, sont détaillés ci-après.

En référence aux figures 1 à 3, dans le cas où le procédé de purification de l'invention est mis en oeuvre sur un flux de gaz entrant 1 comprenant un gaz $G_0$ à purifier et un gaz acide $Ga_1$ à éliminer, ledit gaz acide $Ga_1$ à éliminer présentant une constante d'acidité $pKa_{Ga1}$ supérieure ou égale à 1 et inférieure ou égale à 13, on ajustera le pH du solvant aqueux 3, 31, à une valeur supérieure ou égale à $pKa_{Ga1}+2$ et inférieure ou égale à 13, par ajout d'une solution de base, et cela avant l'étape d'absorption.

Cette étape d'ajustement du pH du solvant aqueux 3, 31, en l'occurrence par ajout de base, est illustrée, sur les figures 1 à 3 annexées, par une flèche symbolisant la présence de moyens d'ajustement 10 du pH, positionnés entre l'unité membranaire de désorption 8 et l'unité membranaire d'absorption 2, en tenant compte du trajet du solvant 3, 30, 31 au sein de la boucle fermée 5.

En outre, la valeur du pH du solvant aqueux 30 riche en gaz $Ga_1$ dissous après absorption de celui-ci, sera ajustée, quant à elle, à une valeur supérieure ou égale à $pKa_{Ga1}-2$ et inférieure ou égale à $pKa_{Ga1}+1$, et cela avant l'étape de désorption dudit gaz acide $Ga_1$. Le pH est ici ajusté par l'ajout, audit solvant aqueux riche 30, d'un volume adéquat d'une solution d'acide.

Cette étape d'ajustement du pH du solvant aqueux riche 30, par ajout d'acide dans ce mode de réalisation, est illustrée, sur les figures annexées, par une flèche symbolisant la présence de moyens d'ajustement 7 du pH, ces moyens 7 étant positionnés entre l'unité membranaire d'absorption 2 et l'unité membranaire de désorption 8, tenant compte du trajet du solvant aqueux 3, 30, 31 au sein du circuit en boucle fermée 5.

De manière préférentielle, le procédé de purification, par absorption gaz-liquide, est mis en oeuvre sur des mélanges de gaz avec un gaz $G_0$ à purifier et un gaz acide $Ga_1$ à éliminer dont le gaz acide $Ga_1$ présente une constante de Henry $H_{Ga1}$ au moins dix fois supérieure à la constante de Henry $H_{G0}$ du gaz $G_0$ à purifier.

Il est en effet préférable qu'il y ait un facteur de 10 de différence de solubilité pour atteindre des performances optimales, aussi bien en termes de pureté en gaz $G_0$ dans le flux de gaz purifié 6, que de rendement de récupération dudit gaz $G_0$ présent, à l'origine, dans le flux entrant 1.

Dans un exemple de réalisation tout particulier, ledit flux entrant 1 consiste en du syngaz, dans lequel gaz $G_0$ à purifier consiste en du dihydrogène $H_2$ présentant une Constante de Henry à 25°C égale à $7,6{\times}10^{-4}$ mol.L$^{-1}$.bar$^{-1}$ et ledit gaz acide $Ga_1$ à éliminer consiste en du dioxyde de carbone $CO_2$ présentant une Constante de Henry $H_{Ga1}$ à 25°C égale à $3,35{\times}10^{-2}$ mol.L$^{-1}$.bar$^{-1}$, une solubilité à 20°C et à pression atmosphérique égale à 1,7 g.L$^{-1}$ et une constante d'acidité p$Ka_{Ga1}$ égale à 6,35.

En d'autres termes, dans cet exemple de réalisation, la valeur du pH du solvant aqueux 3 est ajustée entre 8,35 (p$Ka_{Ga1}$+2) et 13 par ajout de base avant l'étape d'absorption, et entre 4,35 (p$Ka_{Ga1}$-2) et 7,35 (p$Ka_{Ga1}$+1) par ajout d'acide, avant l'étape de désorption.

Les résultats obtenus expérimentalement et illustrant les avantages d'un tel ajustement de pH pour un mélange $H_2/CO_2$ ($G_0/Ga_1$) seront exposés en détails, et en référence à la représentation graphique de la figure 5, dans l'exemple 1 ci-après. Notons d'ores et déjà ici que, de manière essentielle, de tels ajustements de pH permettent l'obtention d'un flux de gaz sortant 6 enrichi à plus de 99% en gaz d'intérêt $H_2$, voire enrichi à plus de 99,9% en gaz d'intérêt H2.

Plus préférentiellement encore, la valeur du pH du solvant aqueux 3, 31 est ajustée entre 9,0 et 12,25 par ajout de base, avant l'étape d'absorption et entre 4,9 et 6,6 par ajout d'acide au solvant riche 30 en gaz dissous, avant l'étape de désorption, toujours pour un mélange $H_2/CO_2$ ($G_0/Ga_1$) en tant que flux de gaz entrant 1.

Des ajustements du pH du solvant aqueux 3, 30, 31 dans de telles gammes de valeurs permettent l'obtention d'un flux de gaz sortant 6 enrichi à plus de 99,99% en gaz d'intérêt $H_2$, pour un mélange traité correspondant à un syngaz enrichi par « Water Gaz Shift » composé de 36,5% de $CO_2$ et de 63,5% de $H_2$, selon les résultats correspondant au maximum atteignable thermodynamiquement, c'est-à-dire avec une surface infinie de membrane, estimés par modélisation.

Ces résultats de modélisation, exprimés en termes de pureté limite en $H_2$ en fonction des conditions de pH avant absorption et avant désorption sont illustrés sur la figure 6, démontrent que de telles valeurs de pH permettent d'obtenir des puretés extrêmement intéressantes, allant jusqu'à plus de 99,999999% en $H_2$ dans le flux de gaz sortant 6, tout en maintenant un bon taux de récupération, sans consommation excessive de réactifs ou d'énergie et sans production de déchets (sels formés), objectif ne pouvant être atteint par les méthodes proposées dans l'état de la technique.

Par conséquent, toujours dans de telles conditions de pH, les teneurs résiduelles théoriques en $CO_2$ à éliminer, dans le flux de gaz sortant 6, illustrées sur le graphique de la figure 7, sont exceptionnellement faibles, allant jusqu'à être inférieures à 10 ppb.

Les représentations graphiques des figures 8 et 9 illustrent également des résultats de simulation correspondant à la limite thermodynamique, obtenus à partir d'un mélange $H_2/CO_2$ avec une teneur initiale en $CO_2$ de 36,5%.

Le graphique de la figure 8 représente l'intensité appliquée sur un module d'électrodialyse à membrane bipolaire pour régénérer l'acide et la base utilisés pour maintenir un certain ajustement de pH avant absorption et avant désorption, correspondant aux différents points de fonctionnement testés.

En effet, selon une caractéristique particulière au procédé de purification d'un flux de gaz entrant 1 conforme à la présente invention, qui sera décrite plus en détails ultérieurement, on effectue une électrodialyse à membrane bipolaire pour régénérer les solutions acide et basique qui sont ajoutées pour diminuer ou augmenter, respectivement, le pH du solvant.

Les résultats obtenus démontrent que, sans une intervention au niveau du pH et un ajustement significatif de celui-ci, et donc en dessous d'une intensité minimum, il n'est pas possible d'espérer atteindre un taux de pureté supérieur à 99% en gaz d'intérêt $H_2$, en modifiant les autres paramètres contrôlés, tels que la pression et le débit du flux de gaz entrant 1, débit de recirculation du solvant aqueux 3, 30, 31, pression du vide à la désorption, température, ou bien encore concentration en sel(s) au sein du solvant aqueux 3, 30, 31.

On peut directement lier l'intensité appliquée (en A) au niveau du module d'électrodialyse à membrane bipolaire, à la quantité d'ions $H^+$ et $OH^-$ qu'il est nécessaire de produire par unité de temps (en mol/s) pour régénérer l'acide (HCl par exemple) ou la base (KOH par exemple) utilisés pour ajuster le pH, en fonction des conditions opératoires (débits, pressions, composition du gaz entrant et souhaitée en sortie), selon l'équation suivante :

$$F_{H^+} = F_{HO^-} = \frac{\eta_F\, I\, N_{cell}}{\mathfrak{F}}$$

$F_H{}^+ = F_{HO}{}^- $ = Débit molaire d'ions H+ et OH- produits pour régénérer respectivement l'acide et la base utilisés pour les ajustements de pH (en mol/s) ;

$\eta_F$ = Rendement faradique (généralement compris entre 80 et 90%) ;

$I$ = Intensité appliquée aux bornes du module d'électrodialyse à membrane bipolaire (en A) ;

$N_{cell}$ = Nombre de cellules dans le stack d'électrodialyse à membrane bipolaire ;

$\mathfrak{F}$ = Constante de Faraday ($\approx$ 96450 C/mol).

Ainsi, le pH obtenu avant absorption, le pH obtenu avant désorption, la pureté et le rendement finalement obtenus en gaz d'intérêt, correspondant à la limite thermodynamique, peuvent être estimés par modélisation à partir des constantes thermodynamiques.

Par ailleurs, la représentation graphique de la figure 9 montre que, pour atteindre cette zone de fonctionnement où le taux de pureté en gaz d'intérêt $H_2$ est supérieur à 99,99%, il est essentiel que le solvant aqueux ne soit pas trop tamponné en vue d'augmenter sa capacité d'absorption. En l'occurrence, si la concentration en tampon phosphate, par exemple, est supérieure à 0,02 mol.$L^{-1}$, alors le pH ne varie pas assez entre l'étape d'absorption et l'étape de désorption, et la zone de fonctionnement qui est ciblée ne peut être atteinte avec un ajout de quantités modérées d'acide ou de base, c'est-à-dire en faibles proportions par rapport à la quantité de gaz à absorber (< 20%).

En conséquence, pour la mise en oeuvre du procédé de l'invention, on utilisera un solvant aqueux 3 absorbant les gaz à éliminer qui comporte un tampon phosphate, ou autre, dont la concentration est inférieure ou égale à 0,02 mol.$L^{-1}$ et, plus préférentiellement encore, un solvant aqueux 3 sans agent tampon qui aura une plus faible capacité d'absorption mais sera plus facilement et bien mieux régénéré.

La figure 10 illustre la représentation graphique des résultats de simulation, correspondant au maximum atteignable thermodynamiquement, c'est-à-dire avec une surface infinie de membrane, obtenus dans le cas d'un flux de gaz entrant 1 comportant du dioxyde de soufre $H_2S$ en tant que gaz acide $Ga_1$ à éliminer et du $H_2$ en tant que gaz d'intérêt $G_0$ à purifier, la teneur initiale en $H_2S$ étant de l'ordre de 1%, soit 10 000 ppm, l'objectif étant d'atteindre une teneur finale en $H_2S$, inférieure à 10 ppm (pureté en $H_2$ supérieure ou égale à 99,999%). Le gaz $H_2S$ est caractérisé par une constante de Henry à 25°C de $1,0 \times 10^{-1}$ mol.$L^{-1}$.$bar^{-1}$ et une solubilité à 20°C et à pression atmosphérique de 3,9 g.$L^{-1}$, et par une constante d'acidité égale à 7,04.

En appliquant les spécificités d'ajustement de pH indiquées ci-dessus, à savoir un ajustement à une valeur comprise entre $pKa_{Ga1}+2$ et 13, soit entre 9,04 et 13 avant absorption, et à une valeur comprise entre $pKa_{Ga1}-2$ et $pK_{Ga1}+1$, soit entre 5,04 et 8,04, l'objectif de pureté fixé peut être atteint.

Le procédé de purification de l'invention peut également être mis en oeuvre sur un flux de gaz entrant 1 comprenant un gaz $G_0$ à purifier et un mélange exclusif de plusieurs gaz acides $Ga_1$, $Ga_2$, $Ga_n$ à éliminer, présentant respectivement une constante d'acidité $pKa_{Ga1}$, $pKa_{Ga2}$, $pKa_{Gan}$ chacune de ces constantes d'acidité étant supérieure ou égale à 1 et inférieure ou égale à 13, tandis que chacun des gaz acides $Ga_1$, $Ga_2$, $Ga_n$ à éliminer présente une Constante de Henry $H_{Ga1}$, $H_{Ga2}$, $H_{Gan}$ supérieure à $10^{-2}$ mol.$L^{-1}$.$bar^{-1}$.

A noter que, préférentiellement, chacune des Constantes de Henry $H_{Ga1}$, $H_{Ga2}$, $H_{Gan}$ des gaz acides $Ga_1$, $Ga_2$, $Ga_n$ à éliminer est au moins dix fois supérieure à la Constante de Henry $H_{G0}$ du gaz $G_0$ à purifier.

Dans ce cas de figure, on ajustera le pH du solvant aqueux 3, 31, en tenant compte de la constante d'acidité pKa du gaz acide présentant la valeur la plus élevée. En d'autres termes, l'ajustement du pH du solvant aqueux 3, 31 avant absorption est effectué, par ajout de base, selon le pKa du gaz acide qui est le gaz le moins acide du mélange à éliminer.

Ainsi, par exemple, considérant un flux de gaz entrant 1 comprenant, outre le gaz $G_0$ à purifier, un premier gaz acide $Ga_1$ présentant une constante d'acidité $pKa_{Ga1}$ et un second gaz acide $Ga_2$ présentant une constante d'acidité $pKa_{Ga2}$ de telle sorte que $pKa_{Ga1}$ est supérieure à $pKa_{Ga2}$, on ajuste le pH à une valeur supérieure ou égale à $pKa_{Ga1}+2$ et inférieure ou égale à 13, par ajout d'une solution de base, avant l'étape d'absorption et on obtient un solvant aqueux riche 30 comportant lesdits gaz acides $Ga_1$, $Ga_2$ dissous.

Avant l'étape de désorption, on ajuste le pH dudit solvant aqueux riche 30 par ajout d'une solution acide, en fonction du pKa du gaz acide présentant la valeur la plus faible, autrement dit en fonction de la constante d'acidité du gaz le plus acide à éliminer.

En considérant l'exemple ci-dessus, où $pKa_{Ga1} > pKa_{Ga2}$, le gaz acide $Ga_2$ étant un acide plus fort que le gaz acide $Ga_1$, le pH du solvant aqueux riche 30 sera ajusté par ajout d'une solution acide à une valeur supérieure ou égale à $pKa_{Ga2}-2$ et inférieure ou égale à $pKa_{Ga2}+1$.

Toujours en référence aux figures 1 à 3, dans le cas où le procédé de purification de l'invention est mis en oeuvre sur un flux de gaz entrant 1 comprenant un gaz $G_0$ à purifier et un gaz basique $Gb_1$ à éliminer, ce dernier présentant une constante d'acidité $pKa_{Gb1}$ supérieure ou égale à 1 et inférieure ou égale à 13, on ajustera le pH du solvant aqueux 3, 31, à une valeur supérieure ou égale à 1 et inférieure ou égale à $pKa_{Gb1}-2$ par ajout d'une solution d'acide, avant l'étape d'absorption.

Cette étape d'ajustement du pH du solvant aqueux 3, 31, en l'occurrence par ajout d'acide, est illustrée, sur les figures 1 à 3 annexées, par une flèche symbolisant la présence de moyens d'ajustement 10 du pH, positionnés entre l'unité membranaire de désorption 8 et l'unité membranaire d'absorption 2, en tenant compte du trajet du solvant 3, 30, 31 au sein de la boucle fermée 5.

En outre, la valeur du pH du solvant aqueux 30 riche en gaz basique $Gb_1$ dissous après absorption de celui-ci, sera ajustée, quant à elle, à une valeur supérieure ou égale à $pKa_{Gb1}$-1 et inférieure ou égale à $pKa_{Gb1}$+2, et cela avant l'étape de désorption dudit gaz acide $Gb_1$. Le pH est ici ajusté par l'ajout audit solvant aqueux riche 30 d'un volume adéquat d'une solution basique.

Cette étape d'ajustement du pH du solvant aqueux riche 30, par ajout de base dans ce mode de réalisation, est illustrée, sur les figures 1 à 3, par une flèche symbolisant la présence de moyens d'ajustement 7 du pH, positionnés entre l'unité membranaire d'absorption 2 et l'unité membranaire de désorption 8, tenant compte du trajet du solvant aqueux 3, 30, 31 au sein du circuit en boucle fermée 5.

De manière préférentielle, le procédé de purification, par absorption gaz-liquide, est mis en oeuvre sur des mélanges de gaz avec un gaz $G_0$ à purifier et un gaz basique $Gb_1$ à éliminer dont le gaz basique $Gb_1$ présente une constante de Henry $H_{Gb1}$ au moins dix fois supérieure à la constante de Henry $H_{G0}$ du gaz $G_0$ à purifier.

Tout comme pour une purification d'un mélange avec un gaz acide, il est en effet préférable qu'il y ait un facteur de 10 de différence de solubilité pour atteindre des performances optimales, aussi bien en termes de pureté en gaz $G_0$ dans le flux de gaz purifié 6, que de rendement de récupération dudit gaz $G_0$ présent, à l'origine, dans le flux entrant 1.

Le gaz basique $Gb_1$ peut notamment consister en de l'ammoniac $NH_3$ présentant une Constante de Henry $H_{Gb1}$ à 25°C égale à 56 mol.L$^{-1}$.bar$^{-1}$ et une solubilité à 20°C et à pression atmosphérique égale à 540 g.L$^{-1}$ et une constante d'acidité $pKa_{Gb1}$ égale à 9,25.

En d'autres termes, dans ce cas de figure, la valeur du pH du solvant aqueux 3 est ajustée entre 1 et 7,25 ($pKa_{Gb1}$-2) par ajout d'acide avant l'étape d'absorption, et entre 8,25 ($pKa_{Gb1}$-1) et 11,25 ($pKa_{Gb1}$+2) par ajout de base, avant l'étape de désorption.

De telles valeurs de pH avant absorption et avant désorption permettent d'aboutir à un flux de gaz sortant 6 comprenant plus de 99% en volume de gaz $G_0$ à purifier.

La représentation graphique de la figure 11 illustre les résultats de modélisation théorique attendus avec un mélange de $NH_3/H_2$ dont la teneur initiale en $NH_3$ est de l'ordre de 0,01%, soit 100 ppm, correspondant à une teneur moyenne que l'on peut observer dans le syngaz après thermolyse et craquage. On cherche ici à atteindre une proportion de $NH_3$ finale dans le flux de gaz sortant inférieure à 1 ppb.

Cet objectif est atteint, selon les résultats de simulation correspondant au maximum atteignable thermodynamiquement, c'est-à-dire avec une surface infinie de membrane, avec un ajustement du pH avant absorption entre 1,5 et 5,7 et avec un ajustement de pH avant désorption entre 8,8 et 10,8.

Le procédé de purification de l'invention peut également être mis en oeuvre sur un flux de gaz entrant 1 comprenant un gaz $G_0$ à purifier et un mélange exclusif de plusieurs gaz basiques $Gb_1$, $Gb_2$, $Gb_n$ à éliminer, présentant respectivement une constante d'acidité $pKa_{Gb1}$, $pKa_{Gb2}$, $pKa_{Gbn}$, chacune de ces constantes d'acidité étant supérieure ou égale à 1 et inférieure ou égale à 13, tandis que chacun des gaz basiques $Gb_1$, $Gb_2$, $Gb_n$ à éliminer présente une Constante de Henry $H_{Gb1}$, $H_{Gb2}$, $H_{Gbn}$ supérieure à $10^{-2}$ mol.L$^{-1}$.bar$^{-1}$.

A noter que, préférentiellement, chacune des Constantes de Henry $H_{Gb1}$, $H_{Gb2}$, $H_{Gbn}$ des gaz basiques $Gb_1$, $Gb_2$, $Gb_n$ à éliminer est au moins dix fois supérieure à la Constante de Henry $H_{G0}$ du gaz $G_0$ à purifier.

Dans ce cas de figure, on ajustera le pH du solvant aqueux 3, 31, en tenant compte de la constante d'acidité pKa du gaz basique présentant la valeur la plus faible. En d'autres termes, l'ajustement du pH du solvant aqueux 3, 31 avant absorption est effectué, par ajout d'acide, selon le pKa du gaz basique qui est le gaz le moins basique du mélange à éliminer.

Ainsi, par exemple, considérant un flux de gaz entrant 1 comprenant, outre le gaz $G_0$ à purifier, un premier gaz basique $Gb_1$ présentant une constante d'acidité $pKa_{Gb1}$ et un second gaz basique $Gb_2$ présentant une constante d'acidité $pKa_{Gb2}$ de telle sorte que $pKa_{Gb1}$ est supérieure à $pKa_{Gb2}$, on ajuste le pH à une valeur supérieure ou égale à 1 et inférieure ou égale à $pKa_{Gb2}$-2, par ajout d'une solution d'acide, avant l'étape d'absorption, et on obtient un solvant aqueux riche 30 comportant lesdits gaz basiques $Gb_1$, $Gb_2$ dissous.

Avant l'étape de désorption, on ajuste le pH dudit solvant aqueux riche 30 par ajout d'une solution basique, en fonction du pKa du gaz basique présentant la valeur la plus élevée, autrement dit en fonction de la constante d'acidité du gaz le plus basique à éliminer.

En considérant l'exemple ci-dessus, où $pKa_{Gb1}$ > $pKa_{Gb2}$, le gaz $Gb_1$ étant une base plus forte que le gaz $Gb_2$, le pH du solvant aqueux riche 30 sera ajusté par ajout d'une solution basique à une valeur supérieure ou égale à $pKa_{Gb1}$-1 et inférieure ou égale à $pKa_{Gb1}$+2.

En référence, à présent, à la figure 4 des dessins ci-joints, le procédé de la présente invention peut également être appliqué dans l'optique de purifier un flux de gaz entrant 1 comprenant un gaz $G_0$ à purifier et un mélange de gaz à éliminer, ledit mélange comportant au moins un gaz acide $Ga_1$ et un gaz basique $Gb_1$.

Dans ce mélange de gaz à éliminer, ledit au moins un gaz acide $Ga_1$ peut être présent en majorité.

Ce que l'on entend par « gaz majoritaire », au sein d'un mélange, au sens de la présente invention, a été défini précédemment dans la description.

Dans un tel flux de gaz entrant 1, ledit gaz $G_0$ à purifier présente une Constante de Henry $H_{G0}$ inférieure à $10^{-2}$ mol.L$^{-1}$.bar$^{-1}$.

Ledit gaz acide $Ga_1$, majoritaire, de même que ledit gaz basique $Gb_1$, minoritaire, qu'il convient d'éliminer, présentent chacun, respectivement, une Constante de Henry $H_{Ga1}$, $H_{Gb1}$, supérieure à $10^{-2}$ mol.L$^{-1}$.bar$^{-1}$ et une constante d'acidité $pKa_{Ga1}$, $pKa_{Gb1}$ supérieure ou égale à 1 et inférieure ou égale à 13.

Préférentiellement, le procédé de purification, par absorption gaz-liquide, est mis en oeuvre sur des mélanges de gaz, dont chacun des gaz à éliminer $Ga_1$ et $Gb_1$ présente une constante de Henry $H_{Ga1}$, $H_{Gb1}$ au moins dix fois supérieure à la constante de Henry $H_{G0}$ du gaz $G_0$ à purifier, pour optimiser les performances, en termes de pureté en gaz $G_0$ dans le flux sortant 6 et de rendement de récupération de celui-ci.

Dans le cas de la purification d'un mélange de gaz contenant $G_0$, $Ga_1$ et $Gb_1$, et comme évoqué précédemment, plusieurs étapes de désorption sont mises en oeuvre et, selon l'invention, avant chacune de ces étapes de désorption, on procède à un ajustement du pH du solvant aqueux circulant dans le circuit en boucle fermée 5.

Plus particulièrement, lorsque le gaz acide $Ga_1$ est majoritaire dans le flux de gaz entrant, suivant l'étape d'absorption et l'obtention d'un solvant aqueux riche 30 en gaz $Ga_1$ et $Gb_1$, on ajuste le pH dudit solvant aqueux riche 30 en prenant en considération, dans un premier temps, la valeur de la constante d'acidité dudit gaz acide $Ga_1$.

Ainsi, avant la première étape de désorption mise en oeuvre au niveau de la première unité membranaire de désorption 8, le pH du solvant aqueux riche 30 en gaz dissous $Ga_1$ et $Gb_1$ sera ajusté par l'ajout d'une quantité adéquate d'une solution d'acide, à une valeur supérieure ou égale à $pKa_{Ga1}$-2 et inférieure ou égale à $pKa_{Ga1}$+1.

Cette première étape d'ajustement du pH du solvant aqueux riche 30 en gaz $Ga_1$ et $Gb_1$ est illustrée, figure 4, par une flèche symbolisant la présence de moyens d'ajustement 7 du pH du solvant 30 positionnés entre l'unité membranaire d'absorption 2 et la première unité membranaire de désorption 8, tenant compte du trajet du solvant aqueux 3, 30, 31 au sein du circuit en boucle fermée 5.

On optimise ici, dans un premier temps, le dégazage du gaz acide $Ga_1$ majoritaire en sorte d'obtenir, d'une part, un premier flux de gaz sortant 9 ou « off gaz », destiné à être éliminé ou valorisé, et comportant le gaz acide $Ga_1$ et, d'autre part, un solvant aqueux 30' riche en gaz basique $Gb_1$ minoritaire et débarrassé dudit gaz acide $Ga_1$ dissous.

Dans une étape suivante, on ajuste le pH de ce solvant aqueux 30', cette fois-ci en tenant compte de la valeur de la constante d'acidité $pKa_{Gb1}$ du gaz basique $Gb_1$ minoritaire. Plus précisément, le pH du solvant 30' est ajusté au moyen de l'ajout d'un volume adéquat d'une solution basique, à une valeur supérieure ou égale à $pKa_{Gb1}$-1 et inférieure ou égale à $pKa_{Gb1}$+2.

Cette étape d'ajustement du pH du solvant aqueux 30', en l'occurrence par ajout de base, est illustrée, sur la figure 4 annexée, par une flèche symbolisant la présence de moyens d'ajustement 7' du pH positionnés entre la première unité membranaire de désorption 8 et la deuxième unité membranaire de désorption 8', en tenant compte du trajet du solvant 3, 30, 30', 31 au sein de la boucle fermée 5.

La deuxième étape de désorption permet une élimination du gaz basique $Gb_1$ du solvant aqueux 30'. On obtient alors, d'une part, un deuxième flux de gaz sortant 9' ou « off gaz », destiné à être évacué ou valorisé, et comportant le gaz basique $Gb_1$ et, d'autre part, un solvant aqueux 31 dégazé dont la quasi-totalité des gaz dissous $Ga_1$ et $Gb_1$ ont été éliminés.

On recycle ensuite le solvant aqueux 31 dégazé à l'étape d'absorption avant laquelle on procède à un ajustement du pH dudit solvant 31, en tenant compte de la constante d'acidité du gaz acide majoritaire $Ga_1$, à une valeur supérieure ou égale à $pKa_{Ga1}$+2 et inférieure ou égale à 13.

Un tel ajustement du pH est réalisé, avant l'étape d'absorption, par l'ajout d'un certain volume d'une solution de base. Il est illustré, sur la figure 4, par une flèche symbolisant la présence de moyens d'ajustement 10 du pH, positionnés entre la deuxième unité membranaire de désorption 8' et l'unité membranaire d'absorption 2, en tenant compte du trajet du solvant 3, 30, 30', 31 au sein de la boucle fermée 5.

Les étapes d'absorption, de première désorption et de deuxième désorption sont opérées simultanément et en continu, en boucle fermée 5, pour aboutir à la purification de la totalité du volume du flux de gaz entrant 1 à traiter.

Dans le cas d'un flux de gaz entrant 1 dont il convient d'extraire un mélange de gaz, il est envisageable que celui-ci comporte, en majorité, plusieurs gaz acides $Ga_1$, $Ga_2$, $Ga_n$, présentant respectivement une constante d'acidité $pKa_{Ga1}$, $pKa_{Ga2}$, $pKa_{Gan}$ et, en minorité, plusieurs gaz basiques $Gb_1$, $Gb_2$, $Gb_n$, présentant respectivement une constante d'acidité $pKa_{Gb1}$, $pKa_{Gb2}$, $pKa_{Gbn}$.

Dans cette hypothèse, et selon l'invention, l'ajustement 10 du pH du solvant aqueux 3, 31 avant l'étape d'absorption est effectué, par l'ajout d'un volume d'une solution de base, en tenant compte de la constante d'acidité pKa du gaz acide présentant la valeur la plus élevée. En d'autres termes, l'ajustement du pH du solvant aqueux 3, 31 avant absorption est effectué selon le pKa du gaz acide qui est le gaz le moins acide du mélange à éliminer.

Ensuite, suivant l'absorption des gaz $Ga_1$, $Ga_2$, $Ga_n$ et $Gb_1$, $Gb_2$, $Gb_n$, l'ajustement 7 du pH du solvant aqueux riche 30 avant la première étape de désorption est effectuée, par ajout d'acide, en fonction du pKa du gaz acide présentant la valeur la plus faible, autrement dit en fonction du gaz le plus acide.

On extrait alors les gaz acides $Ga_1$, $Ga_2$, $Ga_n$ du solvant aqueux riche 30 et on obtient un premier flux de gaz sortant 9 ou « off gaz », destiné à être éliminé ou valorisé, et comportant $Ga_1$, $Ga_2$, $Ga_n$ et, d'autre part, un solvant aqueux 30' riche en gaz basiques $Gb_1$, $Gb_2$, $Gb_n$.

L'ajustement 7' du pH avant la deuxième étape de désorption est effectué, par ajout de base, en prenant en compte la constante d'acidité pKa du gaz basique présentant la valeur la plus élevée, c'est-à-dire le pKa du gaz le plus basique.

L'étape d'absorption et les deux étapes de désorption sont opérées en continu en boucle fermée dans les conditions susmentionnées.

Il ressort, de ce qui précède que, dans le cas où le mélange de gaz à éliminer comporte, en majorité, plusieurs gaz acides $Ga_1$, $Ga_2$, $Ga_n$, présentant respectivement une constante d'acidité $pKa_{Ga1}$, $pKa_{Ga2}$, $pKa_{Gan}$ et, en minorité, un seul gaz basique $Gb_1$ avec une constante d'acidité $pKa_{Gb1}$, il est tenu compte du pKa du gaz acide le moins acide (pKa le plus élevé) avant l'étape d'absorption, du pKa du gaz le plus acide (pKa le plus faible) avant la première étape de désorption et de $pKa_{Gb1}$ avant la deuxième étape de désorption.

En outre, dans le cas où le mélange de gaz à éliminer comporte, en majorité, un seul gaz acide $Ga_1$ présentant une constante d'acidité $pKa_{Ga1}$ et, en minorité, plusieurs gaz basiques $Gb_1$, $Gb_2$, $Gb_n$, présentant respectivement une constante d'acidité $pKa_{Gb1}$, $pKa_{Gb2}$, $pKa_{Gbn}$ : on considère $pKa_{Ga1}$ avant l'étape d'absorption et avant la première étape de désorption, et le pKa du gaz basique le plus basique (pKa le plus élevé) avant la deuxième étape de désorption.

Les prescriptions en matière d'ajustement du pH pour les deux derniers exemples cités restent identiques à celles indiquées précédemment.

En illustration de ce qui précède, considérons un flux de gaz entrant comprenant un mélange de gaz à éliminer contenant, en tant que gaz acides présents en majorité, du $CO_2$ ($Ga_1$, avec $pKa_{Ga1}$ égal à 6,35) et du $H_2S$ ($Ga_2$, $pKa_{Ga2}$ égal à 7,04) et, en tant que gaz basique minoritaire, du $NH_3$ ($Gb_1$, $pKa_{Gb1}$ égal à 9,25).

Dans ce cas, l'ajustement du pH du solvant aqueux sera effectué :

- à une valeur comprise entre 9,04 ($pKa_{Ga2}$+2) et 13, avant l'étape d'absorption, le $H_2S$ étant moins acide que le $CO_2$, et ;
- à une valeur comprise entre 4,35 ($pKa_{Ga1}$-2) et 7,35 ($pKa_{Ga1}$+1), avant la première étape de désorption, le $CO_2$ étant plus acide que le $H_2S$ ($pKa_{Ga1} < pKa_{Ga2}$), et ;
- à une valeur comprise entre 8,25 ($pKa_{Gb1}$-1) et 11,25 ($pKa_{Gb1}$+2) avant la deuxième étape de désorption.

Précédemment ont été décrites les étapes et les recommandations en termes d'ajustement de pH dans le cas d'un mélange de gaz à éliminer dans lequel au moins un gaz acide $Ga_1$ est majoritaire.

Il est également envisageable d'éliminer, d'un flux de gaz entrant 1, un mélange de gaz comportant au moins un gaz acide $Ga_1$ et un gaz basique $Gb_1$, ledit au moins un gaz basique $Gb_1$ étant, cette fois, majoritaire.

Dans un tel flux de gaz entrant 1, ledit gaz $G_0$ à purifier présente une Constante de Henry $H_{G0}$ inférieure à $10^{-2}$ mol.$L^{-1}$.$bar^{-1}$.

Ledit gaz basique $Gb_1$, majoritaire, et ledit gaz acide $Ga_1$, minoritaire, qu'il convient d'éliminer présentent chacun, respectivement, une Constante de Henry $H_{Gb1}$, $H_{Ga1}$, supérieure à $10^{-2}$ mol.$L^{-1}$.$bar^{-1}$ et une constante d'acidité $pKa_{Gb1}$, $pKa_{Ga1}$, supérieure ou égale à 1 et inférieure ou égale à 13.

Préférentiellement, le procédé de purification, par absorption gaz-liquide, est mis en oeuvre sur des mélanges de gaz, dont chacun des gaz à éliminer $Ga_1$ et $Gb_1$ présente une constante de Henry $H_{Ga1}$, $H_{Gb1}$ au moins dix fois supérieure à la constante de Henry $H_{G0}$ du gaz $G_0$ à purifier, pour optimiser les performances, en termes de pureté en gaz $G_0$ dans le flux sortant 6 et de rendement de récupération de celui-ci.

Dans le cas de la purification d'un mélange de gaz contenant $G_0$, $Ga_1$ et $Gb_1$ ($Gb_1$ majoritaire), et comme évoqué précédemment, plusieurs étapes de désorption sont mises en oeuvre et, selon l'invention, avant chacune de ces étapes de désorption, on procède à un ajustement du pH du solvant aqueux circulant dans le circuit en boucle fermée 5.

Plus particulièrement, lorsque le gaz basique $Gb_1$ est majoritaire dans le flux de gaz entrant 1, suivant l'étape d'absorption et l'obtention d'un solvant aqueux riche 30 en gaz $Ga_1$ et $Gb_1$, on ajuste le pH dudit solvant aqueux riche 30 en prenant en considération, dans un premier temps, la valeur de la constante d'acidité dudit gaz basique $Gb_1$.

Ainsi, avant la première étape de désorption mise en oeuvre au niveau de la première unité membranaire de désorption 8, le pH du solvant aqueux riche 30 sera ajusté par l'ajout d'une quantité adéquate d'une solution de base, à une valeur supérieure ou égale à $pKa_{Gb1}$-1 et inférieure ou égale à $pKa_{Gb1}$+2.

Cette première étape d'ajustement du pH du solvant aqueux riche 30 en gaz $Ga_1$ et $Gb_1$ ($Gb_1$ majoritaire) est illustrée, figure 4, par une flèche symbolisant la présence de moyens d'ajustement 7 du pH du solvant 30 positionnés entre l'unité membranaire d'absorption 2 et la première unité membranaire de désorption 8, tenant compte du trajet du solvant aqueux 3, 30, 31 au sein du circuit en boucle fermée 5.

On optimise ici, dans un premier temps, le dégazage du gaz majoritaire basique $Gb_1$ en sorte d'obtenir, d'une part, un premier flux de gaz sortant 9 ou « off gaz », destiné à être éliminé ou valorisé, et comportant le gaz basique

majoritaire Gb$_1$ et, d'autre part, un solvant aqueux 30' riche en gaz acide Ga$_1$ minoritaire et débarrassé dudit gaz basique Gb$_1$ dissous.

Dans une étape suivante, on ajuste le pH de ce solvant aqueux 30', cette fois-ci en tenant compte de la valeur de la constante d'acidité pKa$_{Ga1}$ du gaz acide minoritaire Ga$_1$. Plus précisément, le pH du solvant 30' est ajusté au moyen de l'ajout d'un volume adéquat d'une solution acide, supérieure ou égale à pKa$_{Ga1}$-2 et inférieure ou égale à pKa$_{Ga1}$+1.

Cette étape d'ajustement du pH du solvant aqueux 30', en l'occurrence par ajout d'acide, est illustrée, sur la figure 4 annexée, par une flèche symbolisant la présence de moyens d'ajustement 7' du pH positionnés entre la première unité membranaire de désorption 8 et la deuxième unité membranaire de désorption 8', en tenant compte du trajet du solvant 3, 30, 30', 31 au sein de la boucle fermée 5.

La deuxième étape de désorption permet une élimination du gaz acide minoritaire Ga$_1$ du solvant aqueux 30'. On obtient alors, d'une part, un deuxième flux de gaz sortant 9' ou « off gaz », destiné à être évacué ou valorisé, et comportant le gaz acide Ga$_1$ et, d'autre part, un solvant aqueux 31 dégazé dont la quasi-totalité des gaz dissous Ga$_1$ et Gb$_1$ ont été éliminés.

On recycle ensuite le solvant aqueux 31 dégazé à l'étape d'absorption avant laquelle on procède à un ajustement du pH dudit solvant 31, en tenant compte de la constante d'acidité du gaz majoritaire Gb$_1$, à une valeur supérieure ou égale à 1 et inférieure ou égale à pKa$_{Gb1}$-2.

Un tel ajustement du pH est réalisé, avant l'étape d'absorption, par l'ajout d'un certain volume d'une solution d'acide. Il est illustré, sur la figure 4, par une flèche symbolisant la présence de moyens d'ajustement 10 du pH, positionnés entre la deuxième unité membranaire de désorption 8' et l'unité membranaire d'absorption 2, en tenant compte du trajet du solvant 3, 30, 30', 31 au sein de la boucle fermée 5.

Les étapes d'absorption, de première désorption et de deuxième désorption sont opérées simultanément et en continu, en boucle fermée 5, pour aboutir à la purification de la totalité du volume du flux de gaz entrant 1 à traiter.

Dans le cas d'un flux de gaz entrant 1 dont il convient d'extraire un mélange de gaz, il est envisageable que celui-ci comporte, en majorité, plusieurs gaz basiques Gb$_1$, Gb$_2$, Gb$_n$, présentant respectivement une constante d'acidité pKa$_{Gb1}$, pKa$_{Gb2}$, pKa$_{Gbn}$ et, en minorité, plusieurs gaz acides Ga$_1$, Ga$_2$, Ga$_n$, présentant respectivement une constante d'acidité pKa$_{Ga1}$, pKa$_{Ga2}$, pKa$_{Gan}$.

Dans cette hypothèse, et selon l'invention, l'ajustement 10 du pH du solvant aqueux 3, 31 avant l'étape d'absorption est effectué, par l'ajout d'un volume d'une solution d'acide, en tenant compte de la constante d'acidité pKa du gaz basique présentant la valeur la plus faible. En d'autres termes, l'ajustement du pH du solvant aqueux 3, 31 avant absorption est effectué selon le pKa du gaz le moins basique du mélange à éliminer.

Ensuite, suivant l'absorption des gaz Ga$_1$, Ga$_2$, Ga$_n$ et Gb$_1$, Gb$_2$, Gb$_n$, l'ajustement 7 du pH du solvant aqueux riche 30 avant la première étape de désorption est effectué, par ajout de base, en fonction du pKa du gaz basique présentant la valeur la plus élevée, autrement dit en fonction du gaz le plus basique.

On extrait alors les gaz basiques Gb$_1$, Gb$_2$, Gb$_n$ du solvant aqueux riche 30 et on obtient un premier flux de gaz sortant 9 ou « off gaz », destiné à être éliminé, et comportant Gb$_1$, Gb$_2$, Gb$_n$ et, d'autre part, un solvant aqueux 30' riche en gaz acides Ga$_1$, Ga$_2$, Ga$_n$.

L'ajustement 7' du pH avant la deuxième étape de désorption est effectué, par ajout d'acide, en prenant en compte la constante d'acidité pKa du gaz acide présentant la valeur la plus faible, c'est-à-dire le pKa du gaz le plus acide.

L'étape d'absorption et les deux étapes de désorption sont opérées en continu en boucle fermée dans les conditions susmentionnées pour aboutir à la purification de la totalité du volume du flux de gaz entrant 1 à traiter.

Il ressort, de ce qui précède que, dans le cas où le mélange de gaz à éliminer comporte, en majorité, plusieurs gaz basiques Gb$_1$, Gb$_2$, Gb$_n$, présentant respectivement une constante d'acidité pKa$_{Gb1}$, pKa$_{Gb2}$, pKa$_{Gbn}$ et, en minorité, un seul gaz acide Ga$_1$ avec une constante d'acidité pKa$_{Ga1}$, il est tenu compte du pKa du gaz basique le moins basique (pKa le plus faible) avant l'étape d'absorption, du pKa du gaz le plus basique (pKa le plus élevé) avant la première étape de désorption et de pKa$_{Ga1}$ avant la deuxième étape de désorption.

En outre, dans le cas où le mélange de gaz à éliminer comporte, en majorité, un seul gaz basique Gb$_1$ présentant une constante d'acidité pKa$_{Gb1}$ et, en minorité, plusieurs gaz acides Ga$_1$, Ga$_2$, Ga$_n$, présentant respectivement une constante d'acidité pKa$_{Ga1}$, pKa$_{Ga2}$, pKa$_{Gan}$ : on considère pKa$_{Gb1}$ avant l'étape d'absorption et avant la première étape de désorption, et le pKa du gaz acide le plus acide (pKa le plus faible) avant la deuxième étape de désorption.

Les prescriptions en matière d'ajustement du pH en tenant compte des pKa, telles que définies précédemment dans la description, restent applicables à l'hypothèse d'un mélange de gaz à éliminer avec un gaz basique Gb$_1$ majoritaire.

Il a été indiqué, dans l'intégralité de la description qui précède, que le procédé de purification d'un flux de gaz entrant 1 selon l'invention est applicable pour éliminer au moins un gaz acide Ga$_1$ et/ou au moins un gaz basique Gb$_1$ dont la constante d'acidité est supérieure ou égale à 1 et inférieure ou égale à 13.

En effet, les inventeurs ont constaté que, dans le cas d'un gaz acide fort, tel que l'acide chlorhydrique HCl gazeux, présentant un pKa négatif égal à -6,1, ou d'un gaz basique fort, l'application de variations de pH sur le solvant aqueux aura très peu d'influence sur leur dégazage. En effet, ces gaz acides ou basiques forts restent quasi-totalement dissociés dans le solvant aqueux d'absorption des gaz, et ce quelles que soient les conditions de pH au sein de ce dernier.

Par conséquent, pour les gaz acides ou basiques forts, le procédé de l'invention peut être mis en oeuvre, mais il est imparfait. Il permet, en effet, d'améliorer la pureté sans permettre toutefois une régénération optimale du solvant, car l'accumulation de ces gaz acides ou basiques forts nécessiterait une dilution et une purge occasionnelle dudit solvant.

Cela étant, afin de faire varier ces conditions de pH pour l'élimination de gaz dont le pKa est compris entre 1 et 13, on ajoute, au solvant aqueux circulant dans la boucle fermée 5 une solution acide, au travers des moyens d'ajustement 7, 7',10 de pH.

Préférentiellement, on emploie un acide correspondant au sel utilisé dans ledit solvant aqueux, et la solution d'acide ajoutée présente une concentration molaire équivalente à la concentration molaire en sel dans ledit solvant, celle-ci étant comprise entre 0,2 et 6 mol.L$^{-1}$, selon la solubilité du sel, et ce afin que le solvant aqueux ait toujours la même concentration en sels sur l'ensemble de la boucle liquide.

Aussi, lorsque le solvant aqueux 3, 30, 30', 31 comporte de l'eau et, en tant que sel, du chlorure de sodium NaCl, du chlorure de potassium KCl ou du chlorure de lithium LiCl, l'acide ajouté consiste préférentiellement en de l'acide chlorhydrique HCl.

Lorsque le sel dans le solvant aqueux 3, 30, 30', 31 est choisi parmi le sulfate de sodium $Na_2SO_4$, le sulfate de potassium $K_2SO_4$ et le sulfate de lithium $Li_2SO_4$, l'acide ajouté pourra consister en de l'acide sulfurique $H_2SO_4$.

Lorsque le sel est choisi parmi le bromure de potassium KBr, le bromure de sodium NaBr et le bromure de lithium LiBr, l'acide ajouté sera avantageusement de l'acide bromhydrique HBr.

Lorsque le sel du solvant est de l'iodure de potassium KI, de sodium NaI ou de lithium LiI, l'acide ajouté peut être de l'acide iodhydrique HI.

Enfin lorsque le sel présent dans le solvant consiste en du nitrate de potassium KNOs, du nitrate de sodium NaNOs ou du nitrate de lithium LiNOs, l'acide ajouté consiste préférentiellement en de l'acide nitrique HNOs.

Quelle que soit la solution d'acide ajoutée, celle-ci présente avantageusement une concentration molaire équivalente à celle en sel dans le solvant aqueux 3, 30, 30', 31, autrement dit une concentration molaire équivalente en anion correspondant.

Cela signifie que, dans le cas de tous les sels de lithium, sodium ou potassium proposés, la concentration molaire en acide ajoutée doit être égale à la concentration molaire en sel correspondant. Ainsi, si le solvant aqueux comporte, en tant que sel, du $Na_2SO_4$ à une concentration de 1 mol.L$^{-1}$, la solution d'acide sulfurique $H_2SO_4$ injectée pour faire varier le pH présentera, de manière avantageuse, une concentration molaire égale à 1 mol.L$^{-1}$.

En ce qui concerne à présent la solution basique ajoutée au solvant aqueux 3, 30, 30', 31 pour augmenter le pH de celui-ci, elle correspond également, de manière avantageuse, au sel utilisé dans le solvant aqueux, et présente une concentration molaire équivalente à la concentration molaire en sel dans ledit solvant, celle-ci étant comprise entre 0,2 et 6 mol.L$^{-1}$, selon la solubilité du sel.

Cette solution basique consiste donc en une solution d'hydroxyde de potassium KOH (pour un solvant aqueux contenant du KCl, du $K_2SO_4$, du KBr, du KI, du KNOs) ou en une solution d'hydroxyde de sodium NaOH (pour un solvant aqueux contenant du NaCl, du $Na_2SO_4$, du NaBr, du NaI, du NaNOs) ou en une solution d'hydroxyde de lithium LiOH (pour un solvant aqueux contenant du LiCl, du $Li_2SO_4$, du LiBr, du LiI, du LiNOs).

La solution basique présente avantageusement une concentration molaire équivalente à la concentration molaire en sel dans le solvant aqueux 3, 30, 30', 31, autrement dit une concentration molaire équivalente en cation correspondant.

Cela signifie que dans le cas des sulfates ($Na_2SO_4$, $K_2SO_4$ ou $Li_2SO_4$) la concentration molaire en base ajoutée doit être égale à deux fois la concentration molaire en sel correspondant. Ainsi, si le solvant aqueux comporte, en tant que sel, du $Na_2SO_4$ à une concentration de 1 mol.L$^{-1}$, la solution basique d'hydroxyde de sodium NaOH injectée pour faire varier le pH présentera, de manière avantageuse, une concentration molaire égale à 2 mol.L$^{-1}$. En revanche, dans le cas des chlorures, bromures, iodures et nitrates (ex : NaCl, KBr, LiI, NaNO$_3$) la concentration molaire en base est équivalente à la concentration molaire en sel correspondant.

Chacune des étapes au cours desquelles on ajuste le pH du solvant aqueux 3, 30, 30', 31 circulant dans la boucle fermée 5, est préférentiellement mise en oeuvre, au moyen d'une pompe d'injection de la solution acide ou basique, régulée par un système de contrôle, relié à un pH-mètre placé après le point d'injection, afin d'ajuster en continu le pH à la valeur souhaitée.

Ces étapes d'ajustement du pH du solvant aqueux, par ajout d'acide ou de base avant chaque étape de dégazage ou avant l'étape d'absorption, permettent d'influer très efficacement sur les équilibres acido-basiques du ou des gaz indésirable(s) à éliminer du flux entrant 1, afin de favoriser, selon le cas, l'absorption ou la désorption dudit (desdits) gaz.

A noter également que, contrairement aux procédés qui utilisent des solvants acides ou basiques en tant qu'absorbant chimique, et qui entrainent une consommation importante de réactifs chimiques ou d'énergie pour les régénérer, les quantités d'acide et de base utilisés ici lors de la mise en oeuvre du procédé de l'invention, sont relativement faibles, en comparaison avec la quantité de gaz à absorber (estimées inférieures à 20%).

En outre, selon une caractéristique particulière du procédé, décrite à présent en référence aux figures 3a et 3b des dessins ci-joints, pour réduire encore la consommation de solutions acides et basiques, les inventeurs ont imaginé mettre en oeuvre une étape d'électrodialyse à membrane bipolaire pour régénérer, à la fois, la solution d'acide et la solution de base, ajoutées en cours de procédé pour diminuer ou augmenter, respectivement, le pH du solvant aqueux.

Au moyen de cette étape, en plus de la réduction de la consommation d'acides et de bases minérales, on évite, de manière avantageuse une augmentation progressive de la charge en sels dans le solvant aqueux 3, 30, 30', 31 circulant en boucle entre l'unité d'absorption 2, l'unité de désorption 8, et éventuellement la deuxième unité de désorption 8', qui nécessiterait d'être dessalé par distillation, osmose inverse ou échange d'ions, pour maintenir sa composition constante, aboutissant de nouveau à la consommation de réactifs ou d'énergie et à la production d'un effluent salin comme déchet.

De manière simplifiée, l'électrodialyse à membrane bipolaire, ou EDMB, consiste en une méthode de séparation ou de concentration d'ions en solution, sous l'effet d'un champ électrique, et à l'aide de membranes perméables à la fois aux anions ou aux cations. L'assemblage, en alternance, de membranes 131 bipolaires (MB pour « membrane bipolaire » sur la figure 3b) et monopolaires (MA pour « membrane anionique » et MC pour « membrane cationique » sur la figure 3b) entre une cathode 140 et une anode 141, appelé communément stack, au sein d'un module d'électrodialyse 13, permet de convertir des sels en acides ou en bases correspondants (ou conjugués).

A noter qu'un module d'électrodialyse 13 comporte, en plus du stack, qui constitue l'élément central du module, correspondant à l'assemblage membranes-électrodes, des réservoirs (ou cuves), des pompes de recirculation, d'un générateur électrique, d'instruments de mesure et de régulation et de toute la connectique (tuyauterie et câblage électrique).

Cette méthode, permettant donc une régénération d'acides ou de bases à partir de sel, reste toutefois très sensible à la présence, en solution de certaines espèces qui sont susceptibles d'encrasser les membranes bipolaires du stack d'électrodialyse, notamment $H_2S$ ou les ions calcium $Ca^{2+}$, magnésium $Mg^{2+}$, fer $Fe^{2+}$ et $Fe^{3+}$.

Or, de telle espèces indésirables sont susceptibles d'être produites lors de la mise en oeuvre de procédés de purification, par absorption gaz-liquide, d'un flux de gaz entrant, similaires au procédé de l'invention, c'est-à-dire qui utilisent une unité membranaire d'absorption des gaz ainsi qu'une unité membranaire de désorption, entre lesquelles circule, dans un circuit en boucle fermée, un liquide absorbant les gaz indésirables à éliminer ; notamment, les espèces indésirables susmentionnées se forment au niveau de connexions métalliques du circuit en boucle fermée 5, et vont ensuite circuler dans le solvant aqueux 3, 30, 30', 31 à partir duquel l'acide et la base doivent être régénérés.

Dans l'optique de réaliser une précipitation préventive de ces impuretés, susceptibles d'encrasser les membranes bipolaires du stack d'électrodialyse 13 et de dégrader rapidement les performances de séparation desdites membranes, tout en évitant une consommation énergétique importante pour régénérer l'acide et la base correspondants, les inventeurs ont imaginé maintenir une forte concentration en acide et en base, supérieure à 0,2 mol.$L^{-1}$, et de préférence entre 0,2 et 6 mol.$L^{-1}$, dans les compartiments correspondants du module d'électrodialyse 13.

De cette manière, on permet, au moyen du procédé de l'invention une régénération des solutions d'acide et de base qui sont employées pour améliorer l'absorption et la désorption du ou des gaz indésirables à éliminer du flux de gaz entrant tout en maintenant, de manière avantageuse, les performances de régénération de ces solutions par électrodialyse à membrane bipolaire, au cours du temps, sans risque d'entraîner une contamination des membranes bipolaires 131 du module d'électrodialyse 13.

A noter que la solution dans laquelle est mise en oeuvre une électrodialyse à membrane bipolaire peut être combinée à un moyen d'élimination desdites impuretés, comme des technologies membranaires ou par échange d'ions (adoucisseur), lorsque la charge en impuretés devient trop importante. De telles méthodes seraient mises en oeuvre pour traiter le solvant aqueux dégazé soit au niveau du réservoir (boucle de recirculation vers un système de nanofiltration pour éliminer les cations divalents), soit entre le réservoir et le module d'électrodialyse (cartouche d'échange d'ion piégeant les cations divalents avant que le solvant aqueux dégazé atteigne le module d'ED).

Cela étant, dans un tel exemple de réalisation du procédé de l'invention, où une étape d'EDMB est mise en oeuvre, on achemine le solvant aqueux absorbant les gaz, depuis la boucle fermée 5 vers le module d'électrodialyse 13, par des moyens de transfert adaptés.

Ce transfert de liquide vers le module 13 peut s'effectuer, avantageusement, depuis un réservoir 14 de solvant aqueux dégazé 31 positionné préférentiellement sur ladite boucle fermée 5, ou bien encore sur une boucle annexe d'alimentation de la boucle 5 (non représentée), après l'unité 8 ou les unités de désorption 8, 8'. Le transfert s'effectue au travers de moyens de recirculation 134 reliant, au travers d'une tuyauterie adaptée formant une boucle, ledit réservoir 14 de solvant aqueux 31 et ledit module d'électrodialyse 13 afin de maintenir constamment une recirculation entre ces deux éléments. De tels moyens de recirculation 134 comportent encore avantageusement une vanne permettant de contrôler le débit de solvant aqueux dégazé, sous pression, qui est envoyé depuis le réservoir 14 vers le module 13, tandis qu'une pompe complète préférentiellement ces moyens 134 pour renvoyer le solvant recyclé et dépressurisé depuis le module l'EDMB 13 vers la boucle fermée 5. La vanne et la pompe susmentionnées des moyens de recirculation 134 ne sont pas représentées sur les figures des dessins ci-joints.

Le transfert de solvant aqueux dégazé 31 vers le module 13 peut également provenir directement d'un piquage sur la boucle 5, dans la mesure où celui-ci est positionné entre l'unité de désorption 8 (ou la deuxième unité de désorption 8') et l'unité d'absorption 2, tenant compte du sens de circulation dudit solvant au sein de ladite boucle 5. En effet, il est nécessaire que ce soit le solvant aqueux dégazé 31 qui alimente le module 13. On évite ainsi la perte d'une partie des gaz dissous dans le stack d'électrodialyse, ce qui serait susceptible de poser des problèmes de bullage dans le stack, avec risque d'endommagement de celui-ci, voire un risque de libération de gaz explosif ou inflammable en trop grande quantité.

Dans ce cas, un réservoir de solvant aqueux permettant d'effectuer un appoint de liquide pour compenser des variations de volume au sein de la boucle 5 peut être prévu en un endroit quelconque de ladite boucle 5, voire à l'extérieur de celle-ci, sur une boucle annexe d'alimentation de la boucle 5.

Cela étant, lorsque le réservoir 14 de solvant aqueux est positionné sur ladite boucle fermée 5 après l'unité de désorption 8, constituant alors un réservoir 14 de solvant aqueux dégazé 31, comme visible sur la figure 3b, une première partie du flux de ce solvant aqueux dégazé 31 arrivant au niveau du module 13, via le réservoir 14, peut être acheminé au niveau d'une cuve de solution acide 132 tandis qu'une seconde partie de ce flux part vers une cuve de solution basique 133, afin de faire l'appoint en liquide dans ces cuves 132, 133. Une troisième partie du flux de solvant aqueux dégazé 31 alimente l'un des compartiments 137 du module d'électrodialyse 13, dénommé « compartiment sels » et où circule le solvant aqueux dégazé 31 provenant du réservoir 14 sur la boucle fermée 5.

Deux autres compartiments 138, 139, complètent avantageusement ledit module d'électrodialyse 13, respectivement dénommés « compartiment acide » 138, dans lequel circule la solution acide, et « compartiment base » 139, dans lequel circule la solution basique.

Ainsi, avantageusement, le module d'électrodialyse à membrane bipolaire 13 comporte trois compartiments 137, 138, 139, permettant le maintien d'une forte concentration en acide et en base forte dans les compartiments correspondants, dans l'optique d'éviter les problèmes de précipitation d'impuretés susmentionnés.

Le compartiment « sels » 137 est alimenté par le solvant aqueux dégazé 31 et sert à dissocier une partie des sels en solution : les anions allant vers le compartiment « acide » 138 et les cations allant vers le compartiment « base » 139.

Lesdits trois compartiments 137, 138 et 139 sont séparés les uns des autres par une alternance de membranes, comme illustré sur la figure 3B, la référence « MB » étant relative à des membranes bipolaires, composées d'une face échangeuse d'anions et d'une face échangeuse de cations, tandis que la référence « MA » désigne une membrane anionique autorisant le passage d'ions chargés négativement (comme les ions chlorure $Cl^-$) et la référence « MC » désigne une membrane cationique autorisant le passage d'ions chargés positivement (comme les ions potassium $K^+$), afin de produire des solutions acides et basiques concentrées, respectivement dans les compartiments 138 et 139, à partir du solvant utilisé.

En effet, la membrane bipolaire permet de bloquer le passage à la fois des anions et des cations provenant des compartiments voisins et de transférer simultanément des ions $H^+$ dans le compartiment acide 138 via sa face échangeuse de cations orientée vers la membrane anionique « MA », et des ions $OH^-$ dans le compartiment base 139 via sa face échangeuse d'anions orientée vers la membrane cationique « MC », grâce à l'effet du champ électrique généré entre la cathode 140 et l'anode 141, sachant que les ions $H^+$ et $OH^-$ sont générés au sein de la membrane bipolaire par la réaction spontanée de dissociation de l'eau.

Une fois l'acide et la base régénérés au moyen de l'étape d'électrodialyse à membrane bipolaire, la solution d'acide et la solution basique sont renvoyées, depuis lesdites cuves 132, 133, respectivement, du module d'électrodialyse 13, vers ladite boucle fermée 5, au niveau des moyens d'ajustement du pH, 7 ou 10 selon le cas, du solvant aqueux 30, 31 circulant au sein de la boucle fermée 5.

L'acheminement de la solution d'acide régénérée depuis la cuve d'acide 132 du module d'électrodialyse 13 vers les moyens d'ajustement 7 et 10 du pH est effectué par l'intermédiaire de conduites adaptées 135 et d'une pompe, non représentée sur les figures. Selon la nature du gaz à purifier, la solution d'acide est acheminée soit vers les moyens d'ajustement 7 du pH, soit vers les moyens d'ajustement 10 du pH.

Il en est de même pour la solution de base régénérée qui est renvoyée, depuis la cuve de base 133, vers les moyens d'ajustement 7 ou 10 selon le cas, par des conduites adaptées 136 et par une pompe, cette dernière n'étant pas représentée sur les figures.

La mise en oeuvre d'une étape d'électrodialyse à membrane bipolaire est représentée, sur la figure 3a, dans le cas où une seule étape de désorption est conduite, au niveau de l'unité membranaire 8.

Cependant, il est également envisageable qu'une telle étape d'électrodialyse à membrane bipolaire soit mise en oeuvre dans l'optique de régénérer l'acide et la base présents dans le solvant aqueux lorsque deux étapes de désorption sont mises en oeuvre, par exemple dans le cas de la configuration représentée schématiquement sur la figure 4, pour la purification d'un flux de gaz entrant comprenant, outre le gaz $G_0$ à purifier, un mélange de plusieurs gaz à éliminer.

Dans ce cas de figure, le réservoir de solvant aqueux au sein de la boucle fermée 5 est positionné entre la deuxième unité membranaire de désorption 8' et l'unité membranaire d'absorption 2, en tenant compte du sens de circulation dudit solvant dans ladite boucle fermée 5.

Notons ici que l'étape d'électrodialyse à membrane bipolaire est également opérée en continu, selon une boucle fermée, par une circulation en continu de solvant aqueux dégazé entre, préférentiellement, le réservoir 14 et le module 13. Le solvant aqueux dégazé part du réservoir 14 vers le module 13 puis y retourne en grande partie. Pour rappel, une petite fraction est dirigée vers les cuves « acide » et « base » du module d'électrodialyse 13 afin de maintenir leur niveau constant, cet apport étant destiné à compenser le volume de solution acide et basique consommé.

A noter encore que, quelle que soit la composition du flux de gaz entrant 1, il peut s'avérer avantageux, suivant l'étape d'absorption, et préalablement à l'ajustement du pH avant l'étape de désorption ou avant la première étape de désorption, de procéder à une étape de dégazage préliminaire, au niveau d'une unité membranaire de dégazage intermédiaire 15, illustrée sur les figures 2 à 4.

Cette unité membranaire de dégazage intermédiaire 15 comprend, au moins, une membrane 150 à la fois perméable aux gaz et hydrophobe.

Au travers de cette unité 15, on élimine, par application d'une dépression transmembranaire, dudit solvant aqueux riche 30 circulant d'un côté 151 de ladite membrane 150, l'essentiel de la faible fraction de gaz $G_0$ à purifier qui peut se dissoudre dans le solvant 30, ainsi qu'une partie des gaz $G_1$ dissous à éliminer, acides ou basiques, dans ce solvant 30.

Cette fraction des gaz dissous passe au travers de ladite membrane 150 vers l'autre côté 152 de celle-ci, pour obtenir un solvant aqueux partiellement dégazé, et on renvoie ladite fraction des gaz dissous vers le flux de gaz entrant 1, au travers, par exemple, d'une boucle de recirculation 16.

Au moyen de cette unité complémentaire de dégazage intermédiaire 15, et de cette boucle de recirculation 16, qui permet de renvoyer le mélange de gaz extrait par cette unité 15 vers le flux de gaz entrant, il est envisageable d'améliorer encore le rendement de récupération du gaz $G_0$ à purifier.

De préférence, au niveau de cette unité 15, aucun organe mécanique n'est utilisé pour faciliter le transfert des gaz dissous, le dégazage étant contrôlé par le maintien d'une différence de pression entre les deux côtés 151, 152 de la membrane 150 de ladite unité 15.

La présente invention est également relative à une installation 100 de purification d'un flux de gaz entrant 1 comprenant un gaz $G_0$ à purifier et au moins un gaz acide $G_{a1}$ et/ou un gaz basique $G_{b1}$, à éliminer. Des exemples de configuration de ladite installation 100, permettant avantageusement la mise en oeuvre du procédé de purification décrit précédemment, sont illustrés sur les figures 1 à 4.

Cette installation 100 est avantageusement composée, au moins, des éléments suivants :

- une arrivée 4 de flux de gaz entrant 1 et une sortie 17 du flux de gaz $G_0$ purifié 6, celui-ci pouvant être avantageusement du $CH_4$ ou du H2 ;
- entre ladite arrivée 4 et ladite sortie 17, une unité membranaire d'absorption 2 comprenant au moins une membrane 20 perméable aux gaz et hydrophobe ;
- un circuit en boucle fermée 5 reliant l'unité membranaire d'absorption 2 à au moins une unité membranaire de désorption 8, positionnée en aval de ladite unité membranaire d'absorption 2, en tenant compte du sens dans lequel circule un solvant aqueux 3, 30, 31 au sein du circuit fermé 5, ledit solvant étant apte à absorber ledit au moins un gaz à éliminer ;
- au moins une unité de désorption 8 comportant au moins une membrane 80 perméable aux gaz et hydrophobe, apte à permettre le passage d'au moins une partie du gaz à éliminer dissous dans ledit solvant aqueux 30 pour une élimination de ceux-ci.

Ladite installation 100 comporte encore, au moins, des premiers moyens 10 de régulation du pH du solvant aqueux, positionnés sur le circuit fermé 5, et plus particulièrement entre ladite au moins une unité membranaire de désorption 8 et ladite unité membranaire d'absorption 2, en tenant compte du sens de circulation du solvant aqueux au sein dudit circuit 5.

L'installation 100 comporte en outre des deuxièmes moyens 7 de régulation du pH du solvant aqueux, positionnés, toujours sur le circuit fermé 5, mais cette fois entre l'unité membranaire d'absorption 2 et ladite au moins une unité membranaire de désorption 8.

Ainsi, ces premiers et deuxièmes moyens de régulation 7, 10 du pH sont positionnés judicieusement sur le circuit en boucle fermée 5 de l'installation 100, entre ladite au moins une unité membranaire de désorption 8 et ladite unité membranaire d'absorption 2, au niveau des endroits du circuit 5 où il convient de procéder à une régulation du pH pour favoriser, selon le cas, l'absorption ou la désorption des gaz.

Dans un exemple de réalisation préférentiel, lesdits moyens 7, 10 de régulation du pH du solvant sont composés, chacun, d'au moins un moyen de mesure du pH, d'une cuve de stockage d'une solution acide et/ou d'une cuve de stockage d'une solution basique, externe(s) au circuit fermé 5, et de moyens d'injection de ladite solution acide ou basique au sein de ce circuit 5, selon le cas avant l'unité de désorption 8 ou avant l'unité d'absorption 2.

En référence, à présent, à la figure 4, ladite installation 100 selon l'invention peut également incorporer une unité membranaire de désorption supplémentaire 8', qui s'avère particulièrement avantageuse dans le cas, par exemple, où cette installation 100 doit être mise en oeuvre pour purifier un flux de gaz entrant 1 comprenant un gaz $G_0$ à purifier et un mélange de gaz à éliminer et comportant au moins un gaz acide $G_{a1}$ et un gaz basique $G_{b1}$.

Dans ce mode de réalisation, la configuration de l'installation 100 est telle qu'elle comporte, en aval de ladite unité membranaire d'absorption 2, en tenant compte du sens de circulation dudit solvant aqueux dans le circuit fermé 5, une première unité membranaire de désorption 8 et, encore en aval de celle-ci, une deuxième unité membranaire de désorption 8'.

Dans cette configuration, après le passage du solvant aqueux absorbant 30 riche en mélange de gaz dissous dans la première unité de désorption 8, on obtient un solvant aqueux riche 30' débarrassé d'une partie des gaz dissous, et l'installation 100 intègre des troisièmes moyens 7' de régulation du pH du solvant 30'.

On comprend donc que ces moyens 7' de régulation du pH sont positionnés sur le circuit fermé 5, entre la première unité membranaire de désorption 8 et la deuxième unité membranaire de désorption 8'.

Tout comme les premiers 10 et les deuxièmes 7 moyens de régulation du pH, ces troisièmes moyens de régulation 7' du pH du solvant aqueux 30' sont composés de plusieurs éléments, notamment au moins un moyen de mesure du pH, une cuve de stockage d'une solution acide et/ou d'une cuve de stockage d'une solution basique ainsi que des moyens d'injection de ladite solution acide ou basique au sein du circuit fermé 5, plus particulièrement entre ladite première unité membranaire de désorption 8 et ladite deuxième unité membranaire de désorption 8'.

Selon une particularité de la présente invention, ladite installation 100 comporte, en outre, au moins un module d'électrodialyse à membrane bipolaire 13. Ce dernier permet de régénérer l'acide et la base, injectés au niveau des premiers et deuxièmes moyens de régulation du pH, grâce à un stack constitué d'une série de membranes, 131 bipolaires et monopolaires. Ces membranes sont disposées en alternance et comprises entre une cathode et une anode, présentes au sein du module 13.

De préférence, ledit module 13 est constitué de trois compartiments, à savoir un premier compartiment également dénommé « compartiment sels » 137 au niveau duquel le solvant à régénérer est acheminé, le deuxième compartiment, ou « compartiment acide » 138, où circule la solution acide concentrée, et le troisième compartiment, également appelé « compartiment base » 139, où circule la solution basique concentrée. Les caractéristiques du module d'électrodialyse 13, décrites précédemment en relation avec le procédé, sont applicables à l'installation 100 conforme à l'invention.

Ainsi, avantageusement, ce module 13 est relié à un réservoir 14 de solvant aqueux dégazé 31 positionné sur ledit circuit fermé 5.

Ce réservoir 14 est, avantageusement, localisé entre ladite au moins une unité membranaire de désorption 8 et l'unité membranaire d'absorption 2, en tenant compte du sens de circulation du solvant aqueux 3, 30, 31 au sein du circuit fermé 5, afin que le solvant aqueux 31, après dégazage, soit envoyé vers le module d'électrodialyse à membrane bipolaire 13.

Aussi, dans cet exemple particulier, il convient de prévoir des moyens de transfert et de recirculation 134 dudit solvant aqueux dégazé 31 depuis ledit réservoir 14 vers ledit module d'électrodialyse à membrane bipolaire 13, par exemple sous la forme d'une tuyauterie adaptée 134 reliant ledit réservoir 14 audit module 13, ainsi que d'une vanne de contrôle du débit de liquide quittant ledit réservoir pour être acheminé vers le module 13, ladite tuyauterie formant par ailleurs une boucle fermée de recirculation munie d'une pompe pour renvoyer le solvant aqueux recyclé depuis ledit module 13 vers le réservoir ou directement la boucle fermée.

Des conduites adaptées 135, 136, munies également de pompes, permettent un acheminement de l'acide et la base concentrés, régénérés dans le module d'électrodialyse 13, depuis leur cuve respective 132, 133, vers les moyens d'ajustement 7 et 10 du pH, positionnés respectivement en amont de l'unité membranaire de désorption 8 et en amont de l'unité membranaire d'absorption 2, selon le sens de circulation du liquide dans la boucle 5.

Lorsque l'installation 100 comporte deux unités membranaires de désorption 8, 8', elle peut également intégrer un module 13 d'électrodialyse, relié à un réservoir 14 de solvant aqueux, celui étant alors positionné en aval de la deuxième unité membranaire de désorption 8'.

Enfin, le circuit fermé 5 de l'installation 100 peut également comporter, entre l'unité membranaire d'absorption 2 et ladite unité membranaire de désorption 8, en tenant compte du sens de circulation du solvant aqueux 3, 30, 31 au sein du circuit fermé 5, une unité membranaire de dégazage intermédiaire 15.

Lorsque l'installation 100 comporte deux unités membranaires de désorption 8, 8', l'unité membranaire de dégazage intermédiaire 15 est localisée avant la première unité membranaire de désorption 8.

Cette unité membranaire de dégazage intermédiaire 15 comprend au moins une membrane 150 perméable aux gaz et imperméable aux liquides, apte à permettre le passage d'une fraction des gaz dissous, ainsi qu'une boucle de recirculation 16 reliée à l'arrivée 4 de flux de gaz entrant 1, et ce pour recycler ladite fraction de gaz dissous vers cette arrivée 4 avant l'étape d'absorption.

A noter que de manière générale, et lorsque cela est applicable, les éléments et caractéristiques qui ont été décrits pour l'installation 100 d'épuration d'un flux de gaz entrant de l'invention peuvent être appliqués au procédé de l'invention, et inversement.

L'intérêt de la présente invention apparaitra encore plus clairement à la lecture de l'exemple détaillé ci-dessous, à titre illustratif et non limitatif, en référence au graphique de la figure 5 des dessins ci joints.

Exemple : Comparaison du rendement de récupération du gaz d'intérêt et de la pureté du gaz d'intérêt obtenus par mise en oeuvre du procédé de l'invention et d'un procédé connu de purification d'un flux de gaz entrant

Dans différentes conditions opératoires, le procédé de l'invention a été testé et les résultats, en termes de rendement de récupération du gaz d'intérêt, en l'occurrence du $N_2$, et de pureté de ce gaz, ont été mesurés et comparés aux résultats, obtenus dans des conditions similaires, lors de la mise en oeuvre d'un procédé classique d'épuration de gaz, sans ajustement des conditions de pH du solvant circulant entre les unités d'absorption et de désorption, notamment les résultats obtenus avec le procédé objet de la demande de brevet européen EP 3 372 297.

Les résultats obtenus avec le procédé de l'invention sont représentés par des carrés noirs (série « New-1 ») sur l'illustration graphique de la figure 5, tandis que plusieurs séries ont été testées, avec différentes conditions opératoires, pour un procédé classique d'épuration de gaz par absorptiondésorption avec une solution de $K_2CO_3$ à 0,5 mol.$L^{-1}$ (séries « Old-1 » et « Old-2 »), ou avec une solution de $KHPO_4$ 0,01 mol.$L^{-1}$ (séries « Old-3 » à « Old-5 ») en tant que solvant absorbant, ou bien encore avec le procédé objet de la demande EP 3 372 297 (série « Old-6 ») dans lequel le solvant absorbant consiste en une solution saline de KCl concentrée à 1 mol.$L^{-1}$.

Pour toutes les séries testées, le flux de gaz entrant comporte, en tant que gaz à purifier, du diazote $N_2$, très similaire au dihydrogène $H_2$, en matière de solubilité notamment, et, en tant que gaz à éliminer, un gaz acide, le dioxyde de carbone $CO_2$, celui-ci étant présent dans une proportion égale à 36,5% à l'absorption. Le $N_2$ présente une Constante de Henry égale à $6,2*10^{-4}$ mol.$L^{-1}$.$bar^{-1}$ à 25°C.

Lors de la mise en oeuvre du procédé de l'invention, les conditions opératoires testées et les résultats principaux obtenus sont récapitulés dans le tableau 1 ci-dessous :

[Table 1]

| Pression gaz (Bar) | Débit gaz (NL/h) | %$CO_2$ alim | Débit liquide (L/h) | Rapport | Pureté | Rendement |
|---|---|---|---|---|---|---|
| | | | | G/L | $N_2$(%) | $N_2$(%) |
| 3 | 100 | 36,5% | 100 | 1 | 99,16% | 97,52% |
| | | | 150 | 0,67 | 99,76% | 96,18% |
| | | | 200 | 0,5 | 99,95% | 92,77% |

Dans les différentes colonnes sont indiqués : la pression du gaz à l'absorption (en bar), le débit de flux de gaz entrant (débit gaz en NL/h-normolitre (NL) de gaz entrant par heure), le pourcentage de $CO_2$ dans le flux entrant, le débit du solvant circulant dans la boucle (en L/h - Litre de liquide par heure), le rapport entre le débit de flux de gaz entrant et le débit de solvant aqueux circulant dans la boucle (G/L en NL/L - normolitre (NL) de gaz entrant par Litre (L) de solvant absorbant), ainsi que les résultats en termes de pureté en $N_2$ (en pourcentage) et de rendement de récupération du $N_2$ (en pourcentage).

La température a été maintenue à 25,5°C, la solution de solvant absorbant circulant dans le circuit consiste en une solution de KCl à une concentration de 1 mol.$L^{-1}$, tamponnée avec une solution tampon de $KHPO_4$ à 0,01 mol.$L^{-1}$, et présentant un pH initial égal à 8.

Le pH avant désorption a été ajusté à une valeur de 6 ; 6,2 ; 6,5 et, avant absorption, à une valeur de 9,5 ; 8,9 ; 8,8 pour les débits de liquide suivants, respectivement : 100 ; 150 et 200 L/h. Le pH avant désorption a été ajusté par ajout d'une solution de HCl à 1 mol.$L^{-1}$ et le pH avant désorption a été ajusté avec une solution de KOH à 1 mol.$L^{-1}$. Avant l'ajustement du pH par l'ajout d'acide, le pH avait été mesuré respectivement pour les trois débits de liquide testés à des valeurs de 6,6 ; 7 ; 7,6. Les solutions d'acide et de base ont été systématiquement régénérées par la suite par électrodialyse à membrane bipolaire à partir de la saumure dégazé (KCl 1 mol.$L^{-1}$ usagé).

Pour chacune des différentes conditions opératoires du procédé de l'invention testées, la pureté du flux de gaz sortant est systématiquement supérieure à 99,1%, voire même, dans certains cas, supérieure à 99,7%, tandis que le rendement de récupération reste intéressant, limitant ainsi les pertes en gaz d'intérêt.

Au contraire, pour l'ensemble des nombreuses conditions opératoires testées avec les procédés de l'état de la technique, qui ne sont pas toutes reprises ici, mais qui sont similaires à celles testées pour le présent procédé, les résultats en termes de pureté du gaz d'intérêt récupéré n'atteignent jamais la limite symbolique des 99% de pureté.

Ainsi, par la mise en oeuvre du procédé de l'invention, et notamment la conduite d'étapes d'ajustement du pH du solvant aqueux dans lequel sont dissous les gaz à éliminer, ajustements systématiques et au cours desquels on cherche à induire les variations de pH les plus grandes possibles, avant désorption et avant absorption, on améliore substantiellement la pureté du flux de gaz sortant, sans consommer trop de réactifs (acide et base) et d'énergie, et sans produire de déchets. Pour cela, l'utilisation d'un solvant aqueux ayant une plus faible capacité d'absorption mais davantage réversible (tel qu'une saumure concentrée) est particulièrement intéressante, tandis que l'utilisation d'un agent tampon dans ce solvant aqueux limite drastiquement la pureté, et est donc absolument contre-indiquée, en particulier au-delà d'une certaine concentration, qui a été déterminée à 0,02 mol.L$^{-1}$, dans un objectif de purification de gaz.

**Revendications**

1. Procédé de purification, par absorption gaz-liquide, d'un flux de gaz entrant (1) comprenant un gaz $G_0$ à purifier et au moins un gaz acide $Ga_1$ à éliminer, ledit gaz $G_0$ à purifier présentant une Constante de Henry $H_{G0}$ inférieure à $10^{-2}$ mol.L$^{-1}$.bar$^{-1}$ et ledit gaz acide $Ga_1$ à éliminer présentant une Constante de Henry $H_{Ga1}$ supérieure à $10^{-2}$ mol.L$^{-1}$.bar$^{-1}$ et une constante d'acidité $pKa_{Ga1}$ supérieure ou égale à 1 et inférieure ou égale à 13, ledit procédé comprenant, au moins, les étapes suivantes :

   - lors d'une étape d'absorption, on met au contact, au travers d'une unité membranaire d'absorption (2) comprenant au moins une membrane (20) perméable aux gaz et hydrophobe, ledit flux de gaz entrant (1) d'un côté (21) de ladite membrane (20) et un solvant aqueux (3), apte à absorber les gaz solubles et circulant de l'autre côté (22) de ladite membrane (20), et on obtient un solvant aqueux riche (30) comportant ledit gaz acide $Ga_1$ dissous et un flux de gaz sortant (6) enrichi en gaz $G_0$ à purifier, ledit solvant comprenant au moins une solution aqueuse comportant de l'eau et au moins un sel choisi parmi NaCl, KCl, $Na_2SO_4$, $K_2SO_4$, NaNOs, KNOs NaBr, KBr, NaI, KI, LiCl, LiBr, LiI, $Li_2SO_4$ LiNOs, la concentration molaire en sel dans le liquide absorbant étant comprise entre 0,2 et 6 mol.L$^{-1}$ ;
   - lors d'une étape de désorption, on met en contact ledit solvant aqueux riche (30) avec une unité membranaire de désorption (8) comprenant au moins une membrane (80), perméable aux gaz et hydrophobe, et on élimine, par application d'une dépression transmembranaire, dudit solvant aqueux riche (30) circulant d'un côté (81) de ladite membrane (80), ledit gaz acide $Ga_1$ dissous, par passage au travers de ladite membrane (80) vers l'autre côté (82) de celle-ci, pour obtenir un solvant aqueux dégazé (31) ;
   - on recycle ledit solvant aqueux dégazé (31) à l'étape d'absorption, l'ensemble des étapes dudit procédé étant opérées en continu en boucle fermée, ledit solvant aqueux (3, 30, 31) étant mis en circulation dans un circuit en boucle fermée (5) entre ladite unité membranaire d'absorption (2) et ladite unité membranaire de désorption (8) ;

   ledit procédé étant **caractérisé en ce que** :

   - on ajuste le pH du solvant aqueux (3, 31) à une valeur supérieure ou égale à $pKa_{Ga1}+2$ et inférieure ou égale à 13, par ajout d'une solution basique, avant l'étape d'absorption, et on ajuste le pH du solvant aqueux riche (30) à une valeur supérieure ou égale à $pKa_{Ga1}-2$ et inférieure ou égale à $pKa_{Ga1}+1$, par ajout d'une solution acide, avant l'étape de désorption, ledit solvant aqueux présentant une concentration en agent tampon inférieure à 0,02 mol.L$^{-1}$ ;

   et **en ce que**
   l'on régénère, à partir du solvant aqueux dégazé (31), la base et l'acide, ajoutés audit solvant aqueux (3, 30, 30', 31) pour ajuster le pH, par une étape d'électrodialyse à membrane bipolaire.

2. Procédé de purification, par absorption gaz-liquide, d'un flux de gaz entrant (1) selon la revendication 1, **caractérisé en ce que** ledit gaz $G_0$ à purifier consiste en du dihydrogène $H_2$ présentant une Constante de Henry $H_{G0}$ égale à $7,6 \times 10^{-4}$ mol.L$^{-1}$.bar$^{-1}$ et ledit gaz acide $Ga_1$ à éliminer consiste en du dioxyde de carbone $CO_2$ présentant une Constante de Henry $H_{Ga1}$ égale à $3,35 \times 10^{-2}$ mol.L$^{-1}$.bar$^{-1}$ et une constante d'acidité $pKa_{Ga1}$ égale à 6,35.

3. Procédé de purification, par absorption gaz-liquide, d'un flux de gaz entrant (1) selon la revendication 1, **caractérisé en ce que** ledit gaz $G_0$ à purifier consiste en du méthane $CH_4$ présentant une Constante de Henry $H_{G0}$ égale à $1,3 \times 10^{-3}$ mol.L$^{-1}$.bar$^{-1}$ et ledit gaz acide $Ga_1$ à éliminer consiste en du dioxyde de carbone $CO_2$ présentant une Constante de Henry $H_{Ga1}$ égale à $3,35 \times 10^{-2}$ mol.L$^{-1}$.bar$^{-1}$ et une constante d'acidité $pKa_{Ga1}$ égale à 6,35.

4. Procédé de purification, par absorption gaz-liquide, d'un flux de gaz entrant (1) selon la revendication 2 ou la revendication 3, **caractérisé en ce que** l'on ajuste le pH du solvant aqueux (3, 31) entre 9,0 et 12,25, par ajout d'une solution basique au solvant aqueux (3, 31) avant l'étape d'absorption et on ajuste le pH du solvant aqueux riche (30) entre 4,9 et 6,6 par ajout d'une solution acide audit solvant (30) avant l'étape de désorption.

5. Procédé de purification, par absorption gaz-liquide, d'un flux de gaz entrant (1) selon la revendication 1, ledit flux de gaz entrant (1) comprenant un gaz $G_0$ à purifier et plusieurs gaz acides $Ga_1$, $Ga_2$, $Ga_n$ à éliminer, présentant respectivement une constante d'acidité $pKa_{Ga1}$, $pKa_{Ga2}$, $pKa_{Gan}$, **caractérisé en ce que** l'on ajuste le pH du solvant aqueux (3, 31), avant l'étape d'absorption, par ajout d'une solution basique, en fonction du pKa du gaz acide présentant la valeur la plus élevée, et on ajuste le pH du solvant aqueux riche (30) avant l'étape de désorption, par ajout d'une solution acide, en fonction du pKa du gaz acide présentant la valeur la plus faible.

6. Procédé de purification, par absorption gaz-liquide, d'un flux de gaz entrant (1) comprenant un gaz $G_0$ à purifier et au moins un gaz basique $Gb_1$ à éliminer, ledit gaz $G_0$ à purifier présentant une Constante de Henry $H_{G0}$ inférieure à $10^{-2}$ mol.L$^{-1}$.bar$^{-1}$ et ledit gaz basique $Gb_1$ à éliminer présentant une Constante de Henry $H_{Gb1}$ supérieure à $10^{-2}$ mol.L$^{-1}$.bar$^{-1}$ et une constante d'acidité $pKa_{Gb1}$ supérieure ou égale à 1 et inférieure ou égale à 13, ledit procédé comprenant, au moins, les étapes suivantes :

- lors d'une étape d'absorption, on met au contact, au travers d'une unité membranaire d'absorption (2) comprenant au moins une membrane (20) perméable aux gaz et hydrophobe, ledit flux de gaz entrant (1) d'un côté (21) de ladite membrane (20) et un solvant aqueux (3), apte à absorber les gaz solubles et circulant de l'autre côté (22) de ladite membrane (20), et on obtient un solvant aqueux riche (30) comportant ledit gaz basique $Gb_1$ dissous et un flux de gaz sortant (6) enrichi en gaz $G_0$ à purifier, ledit solvant comprenant au moins une solution aqueuse comportant de l'eau et au moins un sel choisi parmi NaCl, KCl, $Na_2SO_4$, $K_2SO_4$, NaNOs, KNOs NaBr, KBr, NaI, KI, LiCl, LiBr, LiI, $Li_2SO_4$ LiNOs, la concentration molaire en sel dans le liquide absorbant étant comprise entre 0,2 et 6 mol.L$^{-1}$ ;
- lors d'une étape de désorption, on met en contact ledit solvant aqueux riche (30) avec une unité membranaire de désorption (8) comprenant au moins une membrane (80), perméable aux gaz et hydrophobe, et on élimine, par application d'une dépression transmembranaire, dudit solvant aqueux riche (30) circulant d'un côté (81) de ladite membrane (80), ledit gaz basique $Gb_1$ dissous, par passage au travers de ladite membrane (80) vers l'autre côté (82) de celle-ci, pour obtenir un solvant aqueux dégazé (31) ;
- on recycle ledit solvant aqueux dégazé (31) à l'étape d'absorption, l'ensemble des étapes dudit procédé étant opérées en continu en boucle fermée, ledit solvant aqueux (3, 30, 31) étant mis en circulation dans un circuit en boucle fermée (5) entre ladite unité membranaire d'absorption (2) et ladite unité membranaire de désorption (8) ;

ledit procédé étant **caractérisé en ce que**

- on ajuste le pH du solvant aqueux (3, 31) à une valeur supérieure ou égale à 1 et inférieure ou égale à $pKa_{Gb1}$-2, par ajout d'une solution acide, avant l'étape d'absorption et on ajuste le pH du solvant aqueux riche (30) à une valeur supérieure ou égale à $pKa_{Gb1}$-1 et inférieure ou égale à $pKa_{Gb1}$+2, par ajout d'une solution basique, avant l'étape de désorption, ledit solvant aqueux présentant une concentration en agent tampon inférieure à 0,02 mol.L$^{-1}$ ;

et **en ce que** l'on régénère, à partir du solvant aqueux dégazé (31), la base et l'acide, ajoutés audit solvant aqueux (3, 30, 30', 31) pour ajuster le pH, par une étape d'électrodialyse à membrane bipolaire.

7. Procédé de purification, par absorption gaz-liquide, d'un flux de gaz entrant (1) selon la revendication 6, ledit flux de gaz entrant (1) comprenant un gaz $G_0$ à purifier et plusieurs gaz basiques $Gb_1$, $Gb_2$, $Gb_n$ à éliminer, présentant respectivement une constante d'acidité $pKa_{Gb1}$, $pKa_{Gb2}$, $pKa_{Gbn}$, **caractérisé en ce que** l'on ajuste le pH du solvant aqueux (3, 31), avant l'étape d'absorption, par ajout d'une solution acide, en fonction du pKa du gaz basique présentant la valeur la plus faible, et on ajuste le pH du solvant aqueux riche (30) avant l'étape de désorption, par ajout d'une solution basique , en fonction du pKa du gaz basique présentant la valeur la plus élevée.

8. Procédé de purification, par absorption gaz-liquide, d'un flux de gaz entrant (1) comprenant un gaz $G_0$ à purifier et un mélange de gaz à éliminer, ledit mélange comportant au moins un gaz acide $Ga_1$ et un gaz basique $Gb_1$, ledit au moins un gaz acide $Ga_1$ étant majoritaire, ledit gaz $G_0$ à purifier présentant une Constante de Henry $H_{G0}$ inférieure à $10^{-2}$ mol.L$^{-1}$.bar$^{-1}$, ledit gaz acide $Ga_1$ à éliminer présentant une Constante de Henry $H_{Ga1}$ supérieure à $10^{-2}$ mol.L$^{-1}$.bar$^{-1}$ et une constante d'acidité $pKa_{Ga1}$ supérieure ou égale à 1 et inférieure ou égale à 13 et ledit gaz

basique $Gb_1$ à éliminer présentant une Constante de Henry $H_{Gb1}$ supérieure à $10^{-2}$ mol.$L^{-1}$.bar$^{-1}$ et une constante d'acidité $pKa_{Gb1}$ supérieure ou égale à 1 et inférieure ou égale à 13, ledit procédé comprenant, au moins, les étapes suivantes :

- lors d'une étape d'absorption, on met au contact, au travers d'une unité membranaire d'absorption (2) comprenant au moins une membrane (20) perméable aux gaz et hydrophobe, ledit flux de gaz entrant (1) d'un côté (21) de ladite membrane (20) et un solvant aqueux (3), apte à absorber les gaz solubles et circulant de l'autre côté (22) de ladite membrane (20), et on obtient un solvant aqueux riche (30) comportant ledit gaz acide $Ga_1$ dissous et ledit gaz basique $Gb_1$ dissous et un flux de gaz sortant (6) enrichi en gaz $G_0$ à purifier, ledit solvant comprenant au moins une solution aqueuse comportant de l'eau et au moins un sel choisi parmi NaCl, KCl, $Na_2SO_4$, $K_2SO_4$, NaNOs, KNOs NaBr, KBr, Nal, KI, LiCl, LiBr, Lil, $Li_2SO_4$ LiNOs, la concentration molaire en sel dans le liquide absorbant étant comprise entre 0,2 et 6 mol.$L^{-1}$ ;
- lors d'une première étape de désorption, on met en contact ledit solvant aqueux riche (30) avec une première unité membranaire de désorption (8) comprenant au moins une membrane (80), perméable aux gaz et hydrophobe, et on élimine, par application d'une dépression transmembranaire, dudit solvant aqueux riche (30) circulant d'un côté (81) de ladite membrane (80), ledit gaz acide $Ga_1$ dissous, par passage au travers de ladite membrane (80) vers l'autre côté (82) de celle-ci, pour obtenir un solvant aqueux riche en gaz basique $Gb_1$ (30') et débarrassé dudit gaz acide $Ga_1$ dissous ;
- lors d'une deuxième étape de désorption, on met en contact ledit solvant aqueux riche en gaz basique $Gb_1$ (30') avec une deuxième unité membranaire de désorption (8') comprenant au moins une membrane (80'), perméable aux gaz et hydrophobe, et on élimine, par application d'une dépression transmembranaire, du solvant aqueux riche en gaz basique $Gb_1$ (30') circulant d'un côté (81') de ladite membrane (80'), ledit gaz basique $Gb_1$ dissous, par passage au travers de ladite membrane (80') vers l'autre côté (82') de celle-ci, pour obtenir un solvant aqueux (31) dégazé ;
- on recycle ledit solvant aqueux dégazé (31) à l'étape d'absorption, l'ensemble des étapes dudit procédé étant opérées en continu en boucle fermée, ledit solvant aqueux (3, 30, 30', 31) étant mis en circulation dans un circuit en boucle fermée (5) entre ladite unité membranaire d'absorption (2) et lesdites unités membranaires de désorption (8, 8') ;

ledit procédé étant **caractérisé en ce que**

- on ajuste le pH du solvant aqueux (3, 31) à une valeur supérieure ou égale à $pKa_{Ga1}+2$ et inférieure ou égale à 13, par ajout d'une solution basique, avant l'étape d'absorption et **en ce que** l'on ajuste le pH du solvant aqueux riche (30, 30') à une valeur supérieure ou égale à $pKa_{Ga1}-2$ et inférieure ou égale à $pKa_{Ga1}+1$, par ajout d'une solution acide, avant la première étape de désorption et à une valeur supérieure ou égale à $pKa_{Gb1}-1$ et inférieure ou égale à $pKa_{Gb1}+2$, par ajout de base, avant la deuxième étape de désorption, ledit solvant aqueux présentant une concentration en agent tampon inférieure à 0,02 mol.$L^{-1}$ ;

et **en ce que** l'on régénère, à partir du solvant aqueux dégazé (31), la base et l'acide, ajoutés audit solvant aqueux (3, 30, 30', 31) pour ajuster le pH, par une étape d'électrodialyse à membrane bipolaire.

9. Procédé de purification, par absorption gaz-liquide, d'un flux de gaz entrant (1) selon la revendication 8 , **caractérisé en ce que** ledit mélange de gaz à éliminer comporte, en majorité, plusieurs gaz acides $Ga_1$, $Ga_2$, $Ga_n$, présentant respectivement une constante d'acidité $pKa_{Ga1}$, $pKa_{Ga2}$, $pKa_{Gan}$ et, en minorité, plusieurs gaz basiques $Gb_1$, $Gb_2$, $Gb_n$, présentant respectivement une constante d'acidité $pKa_{Gb1}$, $pKa_{Gb2}$, $pKa_{Gbn}$, l'ajustement du pH du solvant aqueux (3, 31) avant l'étape d'absorption étant effectuée, par ajout d'une solution basique, en fonction du pKa du gaz acide présentant la valeur la plus élevée, l'ajustement du pH du solvant aqueux riche (30) avant la première étape de désorption étant effectuée, par ajout d'une solution acide, en fonction du pKa du gaz acide présentant la valeur la plus faible, et l'ajustement du pH du solvant aqueux (30') avant la deuxième étape de désorption étant effectuée, par ajout d'une solution basique, en fonction du pKa du gaz basique présentant la valeur la plus élevée.

10. Procédé de purification, par absorption gaz-liquide, d'un flux de gaz entrant (1) comprenant un gaz $G_0$ à purifier et un mélange de gaz à éliminer, ledit mélange comportant au moins un gaz acide $Ga_1$ et un gaz basique $Gb_1$, ledit au moins un gaz basique $Gb_1$ étant majoritaire, ledit gaz $G_0$ à purifier présentant une Constante de Henry $H_{G0}$ inférieure à $10^{-2}$ mol.$L^{-1}$.bar$^{-1}$, ledit gaz acide $Ga_1$ à éliminer présentant une Constante de Henry $H_{Ga1}$ supérieure à $10^{-2}$ mol.$L^{-1}$.bar$^{-1}$ et une constante d'acidité $pKa_{Ga1}$ supérieure ou égale à 1 et inférieure ou égale à 13 et ledit gaz basique $Gb_1$ à éliminer présentant une Constante de Henry $H_{Gb1}$ supérieure à $10^{-2}$ mol.$L^{-1}$.bar$^{-1}$ et une constante d'acidité $pKa_{Gb1}$ supérieure ou égale à 1 et inférieure ou égale à 13, ledit procédé comprenant, au moins, les étapes suivantes :

- lors d'une étape d'absorption, on met au contact, au travers d'une unité membranaire d'absorption (2) comprenant au moins une membrane (20) perméable aux gaz et hydrophobe, ledit flux de gaz entrant (1) d'un côté (21) de ladite membrane (20) et un solvant aqueux (3), apte à absorber les gaz solubles, circulant de l'autre côté (22) de ladite membrane (20), et on obtient un solvant aqueux riche (30) comportant ledit gaz acide $Ga_1$ dissous et ledit gaz basique $Gb_1$ dissous et un flux de gaz sortant (6) enrichi en gaz $G_0$ à purifier, ledit solvant comprenant au moins une solution aqueuse comportant de l'eau et au moins un sel choisi parmi NaCl, KCl, $Na_2SO_4$, $K_2SO_4$, NaNOs, KNOs NaBr, KBr, NaI, KI, LiCl, LiBr, LiI, $Li_2SO_4$ LiNOs, la concentration molaire en sel dans le liquide absorbant étant comprise entre 0,2 et 6 mol.L$^{-1}$ ;
- lors d'une première étape de désorption, on met en contact ledit solvant aqueux riche (30) avec une première unité membranaire de désorption (8) comprenant au moins une membrane (80), perméable aux gaz et hydrophobe, et on élimine, par application d'une dépression transmembranaire, dudit solvant aqueux riche (30) circulant d'un côté (81) de ladite membrane (80), ledit gaz basique $Gb_1$ dissous, par passage au travers de ladite membrane (80) vers l'autre côté (82) de celle-ci, pour obtenir un solvant aqueux (30') riche en gaz acide $Ga_1$ et débarrassé dudit gaz basique $Gb_1$ dissous ;
- lors d'une deuxième étape de désorption, on met en contact ledit solvant aqueux riche (30') en gaz acide $Ga_1$ avec une deuxième unité membranaire de désorption (8') comprenant au moins une membrane (80'), perméable aux gaz et hydrophobe, et on élimine, par application d'une dépression transmembranaire, du solvant aqueux riche (30') en gaz acide $Ga_1$ circulant d'un côté (81') de ladite membrane (80'), ledit gaz acide $Ga_1$ dissous, par passage au travers de ladite membrane (80') vers l'autre côté (82') de celle-ci, pour obtenir un solvant aqueux (31) dégazé ;
- on recycle ledit solvant aqueux dégazé (31) à l'étape d'absorption, l'ensemble des étapes dudit procédé étant opérées en continu en boucle fermée, ledit solvant aqueux (3, 30, 30', 31) étant mis en circulation dans un circuit en boucle fermée (5) entre ladite unité membranaire d'absorption (2) et lesdites unités membranaires de désorption (8, 8') ;

ledit procédé étant **caractérisé en ce que**

- on ajuste le pH du solvant aqueux (3, 31) à une valeur supérieure ou égale à 1 et inférieure ou égale à pKa$_{Gb1}$-2, par ajout d'une solution acide, avant l'étape d'absorption et **en ce que** l'on ajuste le pH du solvant aqueux riche (30, 30') à une valeur supérieure ou égale à pKa$_{Gb1}$-1 et inférieure ou égale à pKa$_{Gb1}$+2, par ajout d'une solution basique, avant la première étape de désorption et à une valeur supérieure ou égale à pKa$_{Ga1}$-2 et inférieure ou égale à pKa$_{Ga1}$+1, par ajout d'une solution acide, avant la deuxième étape de désorption, ledit solvant aqueux présentant une concentration en agent tampon inférieure à 0,02 mol.L$^{-1}$

et **en ce que** l'on régénère, à partir du solvant aqueux dégazé (31), la base et l'acide, ajoutés audit solvant aqueux (3, 30, 30', 31) pour ajuster le pH, par une étape d'électrodialyse à membrane bipolaire.

11. Procédé de purification, par absorption gaz-liquide, d'un flux de gaz entrant (1) selon la revendication 10, **caractérisé en ce que** ledit mélange de gaz à éliminer comporte, en minorité, plusieurs gaz acides $Ga_1$, $Ga_2$, $Ga_n$, présentant respectivement une constante d'acidité pKa$_{Ga1}$, pKa$_{Ga2}$, pKa$_{Gan}$, et, en majorité, plusieurs gaz basiques $Gb_1$, $Gb_2$, $Gb_n$, présentant respectivement une constante d'acidité pKa$_{Gb1}$, pKa$_{Gb2}$, pKa$_{Gbn}$, l'ajustement du pH du solvant aqueux (3, 31) avant l'étape d'absorption étant effectuée, par ajout d'une solution acide, en fonction du pKa du gaz basique présentant la valeur la plus faible, l'ajustement du pH du solvant aqueux riche (30) avant la première étape de désorption étant effectuée, par ajout d'une solution basique, en fonction du pKa du gaz basique présentant la valeur la plus élevée, et l'ajustement du pH du solvant aqueux (30') avant la deuxième étape de désorption étant effectuée, par ajout d'une solution acide, en fonction du pKa du gaz acide présentant la valeur la plus faible.

12. Procédé de purification, par absorption gaz-liquide, d'un flux de gaz entrant (1) selon l'une quelconque des revendications précédentes, **caractérisé en ce que** l'on utilise, en tant qu'acide pour l'ajustement du pH, une solution acide correspondant au sel utilisé dans le solvant aqueux (3, 30, 30', 31), ladite solution acide consistant soit en de l'acide chlorhydrique HCl lorsque le sel est choisi parmi le NaCl, le KCl et le LiCl, soit en de l'acide sulfurique $H_2SO_4$ lorsque ledit sel est choisi parmi le $Na_2SO_4$, le $K_2SO_4$ et le $Li_2SO_4$, soit en de l'acide bromhydrique HBr lorsque le sel est choisi parmi le KBr, le NaBr et le LiBr, soit en de l'acide iodhydrique HI lorsque ledit sel est choisi parmi le KI, de NaI et le lithium LiI, ou bien encore de l'acide nitrique HNOs lorsque ledit sel est choisi parmi le KNOs, le NaNOs et le LiNOs, ledit acide étant ajouté à une concentration molaire équivalente à celle en sel dans ledit solvant aqueux (3, 30, 30', 31).

**13.** Procédé de purification, par absorption gaz-liquide, d'un flux de gaz entrant (1) selon l'une quelconque des revendications précédentes, **caractérisé en ce que** l'on utilise, en tant que base pour l'ajustement du pH, correspondant au sel utilisé dans le solvant aqueux (3, 30, 30', 31), ladite solution basique consistant soit en de l'hydroxyde de potassium KOH lorsque le sel est choisi parmi le KCl, le $K_2SO_4$, le KBr, le KI et le KNOs, soit en de l'hydroxyde de sodium NaOH lorsque le sel est choisi parmi le NaCl, le $Na_2SO_4$, le NaBr, le NaI et le NaNOs, ou bien encore en de l'hydroxyde de lithium LiOH lorsque le sel est choisi parmi le LiCl, le $Li_2SO_4$, le LiBr, le LiI et le LiNOs, ladite base étant ajoutée à une concentration molaire équivalente à celle en sel dans ledit solvant aqueux (3, 30, 30', 31).

**14.** Procédé de purification, par absorption gaz-liquide, d'un flux de gaz entrant (1) selon l'une quelconque des revendication précédentes, **caractérisé en ce que**, pour la mise en oeuvre de l'étape d'électrodialyse à membrane bipolaire :

- on achemine un flux de solvant aqueux dégazé (31) depuis le circuit en boucle fermée (5) vers un module d'électrodialyse à membrane bipolaire (13) à trois compartiments (137, 138, 139), ledit module comportant un stack qui consiste en un assemblage, en alternance, de membranes (131) bipolaires (MB) et monopolaires (MA, MC), comprises entre une cathode (140) et une anode (141) ;
- on divise ledit flux de solvant aqueux arrivant au niveau du module d'électrodialyse (13), une première partie du flux étant acheminée au niveau d'une cuve de solution acide (132) que comporte ledit module (13), une deuxième partie du flux étant acheminée au niveau d'une cuve de solution basique (133) que comporte également ledit module (13), une troisième partie du flux alimentant un premier compartiment, dénommé compartiment « sels » (137), dudit stack que comporte ledit module (13) ;
- on applique un champ électrique entre la cathode (140) et l'anode (141) dudit stack d'électrodialyse et on forme, par génération et migration d'ions, la solution acide dans le deuxième compartiment, dénommé compartiment « acide » (138), dudit stack que comporte ledit module (13) et la solution basique dans le troisième compartiment, dénommé compartiment « base » (139) dudit stack que comporte ledit module (13) ;
- on fait recirculer la solution d'acide entre le compartiment « acide » (138) dudit stack et ladite cuve de solution acide (132) et on fait recirculer la solution basique entre le compartiment « base » (139) dudit stack et ladite cuve de solution basique (133) ;
- on achemine la solution d'acide depuis ladite cuve de solution acide (132) et la solution basique depuis ladite cuve de solution basique (133) vers les moyens d'ajustement du pH (7,10) que comporte le circuit en boucle fermée (5) ;
- on renvoie le solvant aqueux du premier compartiment « sels » (137) dudit stack vers le circuit en boucle fermée (5).

**15.** Procédé de purification, par absorption gaz-liquide, d'un flux de gaz entrant (1) selon la revendication précédente, **caractérisé en ce que** l'on maintient une concentration en solution acide supérieure à 0,2 mol.L$^{-1}$ dans le deuxième compartiment « acide » (138) dudit stack que comporte ledit module d'électrodialyse (13) et une concentration en solution basique supérieure à 0,2 mol.L$^{-1}$ dans le troisième compartiment « base » (139) dudit stack que comporte ledit module d'électrodialyse (13).

**16.** Procédé de purification, par absorption gaz-liquide, d'un flux de gaz entrant (1) selon l'une quelconque des revendications précédentes, **caractérisé en ce que**, suivant l'étape d'absorption, et préalablement à l'ajustement du pH avant l'étape de désorption ou avant la première étape de désorption, on procède à une étape de dégazage préliminaire au cours de laquelle on met en contact, au travers d'une unité membranaire de dégazage intermédiaire (15) comprenant au moins une membrane (150), perméable aux gaz et hydrophobe, et on élimine, par application d'une dépression transmembranaire, dudit solvant aqueux riche (30) circulant d'un côté (151) de ladite membrane (150), une fraction des gaz dissous, par passage au travers de ladite membrane (150) vers l'autre côté (152) de celle-ci, pour obtenir un solvant aqueux riche partiellement dégazé (30), et on renvoie ladite fraction des gaz dissous vers le flux de gaz entrant (1).

**17.** Procédé de purification, par absorption gaz-liquide, d'un flux de gaz entrant (1) selon l'une quelconque des revendications précédentes, **caractérisé en ce que** l'on met en circulation le solvant aqueux (3, 30, 30', 31) absorbant dans le circuit en boucle fermée (5) à une pression relative comprise entre 1 et 7 bars, de préférence entre 5 et 6 bars.

**18.** Procédé de purification, par absorption gaz-liquide, d'un flux de gaz entrant (1) selon l'une quelconque des revendications précédentes, **caractérisé en ce que** l'on maintient le flux de gaz entrant (1) qui arrive dans l'unité membranaire d'absorption (2) à une pression relative comprise entre 10 mbars et 6 bars, de préférence à une pression relative comprise entre 4,5 et 5,75 bars, plus préférentiellement encore égale à 5 bars.

**19.** Procédé de purification, par absorption gaz-liquide, d'un flux de gaz entrant (1) selon les revendications 17 et 18, **caractérisé en ce que** l'on maintient, entre la pression du solvant aqueux absorbant (3, 30, 30', 31) circulant dans le circuit en boucle fermée 5, et la pression du flux de gaz (1) à l'absorption, une différence de pression positive comprise entre 0,2 et 1 bar.

**20.** Procédé de purification, par absorption gaz-liquide, d'un flux de gaz entrant (1) selon l'une quelconque des revendications précédentes, **caractérisé en ce que** l'on applique une pression absolue, dans l'unité membranaire de désorption (8), du côté (82) de la membrane (80) de sortie des gaz dissous (9), comprise entre 25 mbars et 1 bar.

**21.** Installation (100) de purification d'un flux de gaz entrant (1) comprenant un gaz $G_0$ à purifier et au moins un gaz acide $Ga_1$ et/ou un gaz basique $Gb_1$, à éliminer, pour la mise en oeuvre du procédé selon l'une quelconque des revendications précédentes, comportant, au moins, une unité membranaire d'absorption (2) comprenant au moins une membrane (20) perméable aux gaz et hydrophobe, ladite unité (2) étant reliée, d'un côté (21) de ladite membrane (20), à une arrivée (4) du flux de gaz entrant (1), et à une sortie (17) du flux de gaz sortant (6) enrichi en gaz $G_0$ à purifier et, de l'autre côté (22) de ladite membrane (20), à un circuit fermé (5) dans lequel circule en boucle un solvant aqueux (3) absorbant ledit au moins un gaz à éliminer, ladite installation (100) comportant encore, en aval de ladite unité membranaire d'absorption (2), en tenant compte du sens de circulation dudit solvant aqueux dans le circuit fermé, au moins une unité membranaire de désorption (8), laquelle comporte au moins une membrane (80) perméable aux gaz et hydrophobe, apte à permettre le passage d'au moins une partie du gaz à éliminer dissous dans ledit solvant aqueux (3) et son élimination, ladite installation (100) comportant encore au moins, d'une part, des premiers moyens (10) de régulation du pH du solvant aqueux (3, 31), positionnés sur le circuit fermé (5) entre ladite au moins une unité membranaire de désorption (8) et ladite unité membranaire d'absorption (2), en tenant compte du sens de circulation du solvant aqueux (3, 30, 31) au sein dudit circuit fermé (5) et, d'autre part, des deuxièmes moyens (7) de régulation du pH du solvant aqueux (30) positionnés sur le circuit fermé (5) entre l'unité membranaire d'absorption (2) et ladite au moins une unité membranaire de désorption (8), en tenant compte du sens de circulation du solvant aqueux (3, 30, 31) au sein dudit circuit fermé (5), ledit solvant comprenant au moins une solution aqueuse comportant de l'eau et au moins un sel choisi parmi NaCl, KCl, $Na_2SO_4$, $K_2SO_4$, NaNOs, KNOs NaBr, KBr, Nal, KI, LiCl, LiBr, Lil, $Li_2SO_4$ LiNOs, la concentration molaire en sel dans le liquide absorbant étant comprise entre 0,2 et 6 mol.$L^{-1}$, ladite installation (100) étant **caractérisée en ce qu'**elle comporte au moins un module d'électrodialyse à membrane bipolaire (13) à trois compartiments (137, 138, 139) pour régénérer l'acide et la base utilisés à partir du solvant aqueux dégazé (31) au moyen d'un stack constitué d'une série de membranes (131) bipolaires et monopolaires, disposées en alternance, et comprises entre une cathode (140) et une anode (141), que comporte ledit module (13), celui-ci étant relié à un réservoir (14) de solvant aqueux dégazé (31) positionné sur ledit circuit fermé (5), entre ladite au moins une unité membranaire de désorption (8) et l'unité membranaire d'absorption (2), en tenant compte du sens de circulation du solvant aqueux (3, 30, 31) au sein dudit circuit fermé (5), des moyens de recirculation (134) dudit solvant aqueux (31) entre ledit réservoir (14) et ledit module d'électrodialyse à membrane bipolaire (13) étant également prévus, ainsi que des moyens d'acheminement (135, 136) des solutions acide et basique régénérées depuis, respectivement, une cuve de solution d'acide (132) et une cuve de solution basique (133) que comporte ledit module (13), vers les moyens (7, 10) de régulation du pH, ledit solvant aqueux présentant une concentration en agent tampon inférieure à 0,02 mol.$L^{-1}$ au sein de ladite installation (100).

**22.** Installation (100) de purification d'un flux de gaz entrant (1) selon la revendication 21, **caractérisée en ce que** lesdits premiers moyens (10) de régulation du pH du solvant aqueux (3, 31) sont composés d'au moins un moyen de mesure du pH, d'une cuve de stockage d'une solution acide et/ou d'une cuve de stockage d'une solution basique, et de moyens d'injection de ladite solution acide ou basique au sein du circuit fermé (5) entre ladite au moins une unité membranaire de désorption (8) et ladite unité membranaire d'absorption (2) et **en ce que** lesdits deuxièmes moyens (7) de régulation du pH du solvant aqueux (30) sont composés d'au moins un moyen de mesure du pH, d'une cuve de stockage d'une solution acide et/ou d'une cuve de stockage d'une solution basique et de moyens d'injection de ladite solution acide ou basique au sein du circuit fermé (5) entre l'unité membranaire d'absorption (2) et ladite au moins une unité membranaire de désorption (8).

**23.** Installation (100) d'épuration d'un flux de gaz entrant selon l'une quelconque des revendications 21 ou 22, **caractérisée en ce qu'**elle comporte, en aval de ladite unité membranaire d'absorption (2), en tenant compte du sens de circulation dudit solvant aqueux (3, 30, 30', 31) dans le circuit fermé (5), une première unité membranaire de désorption (8) et, en aval de celle-ci, une deuxième unité membranaire de désorption (8'), ainsi que des troisièmes moyens (7') de régulation du pH du solvant aqueux (30') positionnés sur le circuit fermé (5) entre ladite première unité membranaire de désorption (8) et ladite deuxième unité membranaire de désorption (8'), lesdits troisièmes moyens (7') de régulation du pH du solvant aqueux (30') étant composés d'au moins un moyen de mesure du pH, d'une cuve de stockage d'une solution acide et/ou d'une cuve de stockage d'une solution basique et de moyens d'injection de ladite solution acide ou basique au sein du circuit fermé (5) entre ladite première unité membranaire de désorption (8) et ladite deuxième unité membranaire de désorption (8').

24. Installation (100) d'épuration d'un flux de gaz entrant selon l'une quelconque des revendications 21 à 23, **caractérisée en ce qu'**elle comporte, sur le circuit fermé (5), entre l'unité membranaire d'absorption (2) et ladite au moins une unité membranaire de désorption (8), en tenant compte du sens de circulation du solvant aqueux (3, 30, 31) au sein du circuit fermé (5), une unité membranaire de dégazage intermédiaire (15) comprenant au moins une membrane (150) perméable aux gaz et hydrophobe, apte à permettre le passage d'une fraction du gaz dissous, ainsi qu'une boucle de recirculation (16) reliée à l'arrivée (4) de flux de gaz entrant (1) que comporte l'installation (100) pour renvoyer ladite fraction de gaz dissous vers cette arrivée (4) du flux de gaz entrant (1).

**Patentansprüche**

1. Verfahren zur Reinigung eines eintretenden Gasstroms (1) durch Gas-Flüssigkeitsabsorption, umfassend ein zu reinigendes Gas $G_0$ und mindestens ein zu beseitigendes saures Gas $Ga_1$, wobei das zu reinigende Gas $G_0$ eine Henry-Konstante $Hg_0$ von weniger als $10^{-2}$ mol.L$^{-1}$.bar$^{-1}$ und das zu beseitigende saure Gas $Ga_1$ eine Henry-Konstante $Hga_1$ größer als $10^{-2}$ mol.L$^{-1}$.bar$^{-1}$ und eine Säurekonstante $pKaga^1$ größer als oder gleich 1 und kleiner als oder gleich 13 aufweist, wobei das Verfahren mindestens die folgenden Schritte umfasst:

- während eines Absorptionsschritts, Inkontaktbringen, über eine Absorptionsmembraneinheit (2), die mindestens eine für Gase durchlässige und hydrophobe Membran (20) umfasst, des eintretenden Gasstroms (1) auf einer Seite (21) der Membran (20) mit einem wässrigen Lösungsmittel (3), das geeignet ist, lösliche Gase zu absorbieren, und das auf der anderen Seite (22) der Membran (20) zirkuliert, und Erhalten eines angereicherten wässrigen Lösungsmittels (30) mit dem gelösten sauren Gas $Ga_1$ und eines austretenden Gasstroms (6), der mit dem zu reinigenden Gas $G_0$ angereichert ist, wobei das Lösungsmittel mindestens eine wässrige Lösung bestehend aus Wasser und mindestens einem Salz ausgewählt aus NaCl, KCl, $Na_2SO_4$, $K_2SO_4$, NaNOs, KNOs NaBr, KBr, Nal, KI, LiCl, LiBr, Lil, $Li_2SO_4$ LiNOs umfasst, wobei die molare Konzentration des Salzes in der Absorptionsflüssigkeit zwischen 0,2 und 6 mol. L$^{-1}$ liegt;
- während eines Desorptionsschritts, Inkontaktbringen des angereicherten wässrigen Lösungsmittels (30) mit einer Desorptionsmembraneinheit (8), die mindestens eine Membran (80) umfasst, die für Gase durchlässig und hydrophob ist, und Beseitigen, durch Anwendung einer Transmembranvertiefung, des angereicherten wässrigen Lösungsmittels (30), das auf einer Seite (81) der Membran (80) zirkuliert, wobei das gelöste saure Gas $Ga_1$ durch die Membran (80) zu deren anderen Seite (82) zum Erhalten eines entgasten wässrigen Lösungsmittels (31) hindurchtritt;
- Rückführen des entgasten wässrigen Lösungsmittels (31) in den Absorptionsschritt, wobei alle Schritte des Verfahrens fortlaufend in einem geschlossenen Kreislauf durchgeführt werden, wobei das wässrige Lösungsmittel (3, 30, 31) in einem geschlossenen Kreislauf (5) zwischen der Absorptionsmembraneinheit (2) und der Desorptionsmembraneinheit (8) zirkuliert wird;

wobei das Verfahren **gekennzeichnet ist durch:**

- Einstellen des pH-Werts des wässrigen Lösungsmittels (3, 31) auf einen Wert größer oder gleich $pKaga_1$+2 und kleiner oder gleich 13 **durch** Zusetzen einer basischen Lösung vor dem Absorptionsschritt und Einstellen des pH-Werts des angereicherten wässrigen Lösungsmittels (30) auf einen Wert größer oder gleich $pKaga_1$-2 und kleiner oder gleich $pKaga_1$+1 **durch** Zusetzen einer sauren Lösung vor dem Desorptionsschritt, wobei das wässrige Lösungsmittel eine Pufferkonzentration von weniger als 0,02 mol.L$^{-1}$ aufweist;

**und durch** Regenerieren der Base und der Säure, die dem wässrigen Lösungsmittel (3, 30, 30', 31) zugesetzt wurden, aus dem entgasten wässrigen Lösungsmittel (31) zum Einstellen des pH-Werts **durch** einen Schritt der bipolaren Membranelektrodialyse.

2. Verfahren zur Reinigung eines eintretenden Gasstroms (1) durch Gas-Flüssigkeitsabsorption nach Anspruch 1, **dadurch gekennzeichnet, dass** das zu reinigende Gas $G_0$ aus Dihydrogen $H_2$ mit einer Henry-Konstante $Hg_0$ gleich $7,6 \times 10^{-4}$ mol.L$^{-1}$.bar$^{-1}$ besteht und das zu beseitigende saure Gas $Ga_1$ aus Kohlendioxid $CO_2$ mit einer Henry-Konstante $Hga_1$ gleich $3,35 \times 10^{-2}$ mol.L$^{-1}$.bar$^{-1}$ und einer Säurekonstante $pKaga^1$ gleich 6,35 besteht.

3. Verfahren zur Reinigung eines eintretenden Gasstroms (1) durch Gas-Flüssigkeitsabsorption nach Anspruch 1, **dadurch gekennzeichnet, dass** das zu reinigende Gas $G_0$ aus Methan $CH_4$ mit einer Henry-Konstante $Hg_0$ gleich $1,3 \times 10^{-3}$ mol.L$^{-1}$.bar$^{-1}$ besteht und das zu beseitigende saure Gas $Ga_1$ aus Kohlendioxid $CO_2$ mit einer Henry-Konstante $Hga_1$ gleich $3,35 \times 10^{-2}$ mol.L$^{-1}$.bar$^{-1}$ und einer Säurekonstante $pKaga_1$ gleich 6,35 besteht.

4. Verfahren zur Reinigung eines eintretenden Gasstroms (1) durch Gas-Flüssigkeitsabsorption nach Anspruch 2 oder 3, **dadurch gekennzeichnet, dass** der pH-Wert des wässrigen Lösungsmittels (3, 31) zwischen 9,0 und 12,25 durch Zusetzen einer basischen Lösung zu dem wässrigen Lösungsmittel (3, 31) vor dem Absorptionsschritt eingestellt wird und der pH-Wert des angereicherten wässrigen Lösungsmittels (30) zwischen 4,9 und 6,6 durch Zusetzen einer sauren Lösung zu dem Lösungsmittel (30) vor dem Desorptionsschritt eingestellt wird.

5. Verfahren zur Reinigung eines eintretenden Gasstroms (1) durch Gas-Flüssigkeitsabsorption nach Anspruch 1, wobei der eintretende Gasstrom (1) ein zu reinigendes Gas $G_0$ und mehrere zu beseitigende saure Gase $Ga_1$, $Ga_2$, $Ga_n$, jeweils aufweisend eine Säurekonstante $pKaga_1$, $pKaga_2$, $pKaga_n$, umfasst, **dadurch gekennzeichnet, dass der** pH-Wert des wässrigen Lösungsmittels (3, 31) vor dem Absorptionsschritt durch Zusetzen einer basischen Lösung, in Abhängigkeit des pKa des sauren Gases mit dem höchsten Wert, eingestellt wird, und der pH-Wert des angereicherten wässrigen Lösungsmittels (30) vor dem Desorptionsschritt durch Zusetzen einer sauren Lösung in Abhängigkeit des pKa-Werts des sauren Gases mit dem niedrigsten Wert eingestellt wird.

6. Verfahren zur Reinigung eines eintretenden Gasstroms (1) durch Gas-Flüssigkeitsabsorption, umfassend ein zu reinigendes Gas $G_0$ und mindestens ein zu beseitigendes basisches Gas $Gb^1$, wobei das zu reinigende Gas $G^0$ eine Henry-Konstante $Hg^0$ von weniger als $10^{-2}$ mol.L$^{-1}$.bar$^{-1}$ und das zu beseitigende basische Gas $Gb_1$ eine Henry-Konstante $Hgb^1$ größer als $10^{-2}$ mol.L$^{-1}$.bar$^{-1}$ und eine Säurekonstante $pKaGb1$ größer als oder gleich 1 und kleiner als oder gleich 13 aufweist, wobei das Verfahren mindestens die folgenden Schritte umfasst:

- während eines Absorptionsschritts, Inkontaktbringen, über eine Absorptionsmembraneinheit (2), die mindestens eine für Gase durchlässige und hydrophobe Membran (20) umfasst, des eintretenden Gasstroms (1) auf einer Seite (21) der Membran (20) mit einem wässrigen Lösungsmittel (3), das geeignet ist, lösliche Gase zu absorbieren, und das auf der anderen Seite (22) der Membran (20) zirkuliert, und Erhalten eines angereicherten wässrigen Lösungsmittels (30) mit dem gelösten basischen $Gb_1$ und eines austretenden Gasstroms (6), der mit dem zu reinigenden Gas $G_0$ angereichert ist, wobei das Lösungsmittel mindestens eine wässrige Lösung bestehend aus Wasser und mindestens einem Salz ausgewählt aus NaCl, KCl, $Na_2SO_4$, $K_2SO_4$, NaNOs, KNOs NaBr, KBr, Nal, KI, LiCl, LiBr, Lil, $Li_2SO_4$ LiNOs umfasst, wobei die molare Konzentration des Salzes in der Absorptionsflüssigkeit zwischen 0,2 und 6 mol. L$^{-1}$ liegt;
- während eines Desorptionsschritts, Inkontaktbringen des angereicherten wässrigen Lösungsmittels (30) mit einer Desorptionsmembraneinheit (8), die mindestens eine für Gase durchlässige und hydrophobe Membran (80) umfasst, und Beseitigen, durch Anwendung einer Transmembranvertiefung, des angereicherten wässrigen Lösungsmittels (30), das auf einer Seite (81) der Membran (80) zirkuliert, wobei das gelöste basische Gas $Gb_1$ durch die Membran (80) zu deren anderen Seite (82) zum Erhalten eines entgasten wässrigen Lösungsmittels (31) hindurchtritt;
- Rückführen des entgasten wässrigen Lösungsmittels (31) in den Absorptionsschritt, wobei alle Schritte des Verfahrens fortlaufend in einem geschlossenen Kreislauf durchgeführt werden, wobei das wässrige Lösungsmittel (3, 30, 31) in einem geschlossenen Kreislauf (5) zwischen der Absorptionsmembraneinheit (2) und der Desorptionsmembraneinheit (8) zirkuliert wird;

wobei das Verfahren **gekennzeichnet ist durch**

- Einstellen des pH-Werts des wässrigen Lösungsmittels (3, 31) auf einen Wert größer oder gleich 1 und kleiner oder gleich pKaGb1-2 **durch** Zusetzen einer sauren Lösung vor dem Absorptionsschritt und Einstellen des pH-Werts des angereicherten wässrigen Lösungsmittels (30) auf einen Wert größer oder gleich pKaGb1-1 und kleiner oder gleich pKaGb1+2 **durch** Zusetzen einer basischen Lösung vor dem Desorptionsschritt, wobei das wässrige Lösungsmittel eine Pufferkonzentration von weniger als 0,02 mol.L$^{-1}$ aufweist;

**und durch** Regenerieren der Base und der Säure, die dem wässrigen Lösungsmittel (3, 30, 30', 31) zugesetzt wurden, um den pH-Wert einzustellen, aus dem entgasten wässrigen Lösungsmittel (31) **durch** einen Schritt der bipolaren Membranelektrodialyse.

7. Verfahren zur Reinigung eines eintretenden Gasstroms (1) durch Gas-Flüssigkeitsabsorption nach Anspruch 6, wobei der eintretende Gasstrom (1) ein zu reinigendes Gas $G_0$ und mehrere zu beseitigende basische Gase $Gb_1$, $Gb_2$, $Gb_n$, jeweils aufweisend eine Säurekonstante pKaGb1, $pKa_{Gb2}$, pKaGbn, umfasst, **dadurch gekennzeichnet, dass** der pH-Wert des wässrigen Lösungsmittels (3,31) vor dem Absorptionsschritt durch Zusetzen einer sauren Lösung, in Abhängigkeit des pKa des basischen Gases mit dem kleinsten Wert, eingestellt wird und der pH-Wert des angereicherten wässrigen Lösungsmittels (30) vor dem Desorptionsschritt durch Zusetzen einer basischen Lösung, in Abhängigkeit des pKa des basischen Gases mit dem höchsten Wert, eingestellt wird.

8. Verfahren zur Reinigung eines eintretenden Gasstroms (1) durch Gas-Flüssigkeitsabsorption, umfassend ein zu reinigendes Gas $G_0$ und ein zu beseitigendes Gasgemisch umfasst, wobei das Gemisch mindestens ein saures Gas $Ga_1$ und ein basisches Gas $Gb_1$ aufweist, wobei das mindestens eine saure Gas $Ga_1$ überwiegt, wobei das zu reinigende Gas $G_0$ eine Henry-Konstante $Hg_0$ von weniger als $10^{-2}$ mol.L$^{-1}$.bar$^{-1}$ aufweist, das zu beseitigende saure Gas $Ga_1$ eine Henry-Konstante $Hga_1$ größer als $10^{-2}$ mol.L$^{-1}$.bar$^{-1}$ und eine Säurekonstante $pKaga_1$ größer oder gleich 1 und kleiner oder gleich 13 aufweist und das zu beseitigende basische Gas $Gb_1$ eine Henry-Konstante $Hgb_1$ größer als $10^{-2}$ mol.L$^{-1}$.bar$^{-1}$ und eine Säurekonstante $pKagb_1$ größer oder gleich 1 und kleiner oder gleich 13 aufweist, wobei das Verfahren mindestens die folgenden Schritte umfasst:

- während eines Absorptionsschritts, Inkontaktbringen, über eine Absorptionsmembraneinheit (2), die mindestens eine für Gase durchlässige und hydrophobe Membran (20) umfasst, des eintretenden Gasstroms (1) auf einer Seite (21) der Membran (20) mit einem wässrigen Lösungsmittel (3), das geeignet ist, lösliche Gase zu absorbieren, und das auf der anderen Seite (22) der Membran (20) zirkuliert, und Erhalten eines angereicherten wässrigen Lösungsmittels (30) mit dem gelösten sauren Gas $Ga_1$ und dem gelösten basischen Gas $Gb_1$ und eines austretenden Gasstroms (6), der mit dem zu reinigenden Gas $G_0$ angereichert ist, wobei das Lösungsmittel mindestens eine wässrige Lösung bestehend aus Wasser und mindestens einem Salz ausgewählt aus NaCl, KOI, $Na_2SO_4$, $K_2SO_4$, NaNOs, KNOs NaBr, KBr, Nal, KI, LiCl, LiBr, Lil, $Li_2SO_4$ LiNOs umfasst, wobei die molare Konzentration des Salzes in der Absorptionsflüssigkeit zwischen 0,2 und 6 mol. L$^{-1}$ liegt;
- während eines ersten Desorptionsschritts, Inkontaktbringen des angereicherten wässrigen Lösungsmittels (30) mit einer ersten Desorptionsmembraneinheit (8), die mindestens eine für Gase durchlässige und hydrophobe Membran (80) umfasst, und Beseitigen, durch Anwendung einer Transmembranvertiefung, des angereicherten wässrigen Lösungsmittels (30), das auf einer Seite (81) der Membran (80) zirkuliert, wobei das gelöste saure Gas $Ga_1$ durch die Membran (80) zu deren anderen Seite (82) hindurchtritt, um ein wässriges Lösungsmittel (30'), das mit basischem Gas $Gb_1$ angereichert ist und von dem gelösten sauren Gas $Ga_1$ befreit ist, zu erhalten;
- während eines zweiten Desorptionsschritts, Inkontaktbringen des mit basischem Gas $Gb_1$ angereicherten wässrigen Lösungsmittels (30') mit einer zweiten Desorptionsmembraneinheit (8'), die mindestens eine für Gase durchlässige und hydrophobe Membran (80') umfasst, und Beseitigen, durch Anwendung einer Transmembranvertiefung, des mit basischem Gas $Gb_1$ angereicherten wässrigen Lösungsmittels (30'), das auf einer Seite (81') der Membran (80') zirkuliert, wobei das gelöste basische Gas $Gb_1$ durch die Membran (80') zu deren anderen Seite (82') zum Erhalten eines entgasten wässrigen Lösungsmittels (31) hindurchtritt;
- Rückführen des entgasten wässrigen Lösungsmittels (31) in den Absorptionsschritt, wobei alle Schritte des Verfahrens fortlaufend in einem geschlossenen Kreislauf durchgeführt werden, wobei das wässrige Lösungsmittel (3, 30, 30', 31) in einem geschlossenen Kreislauf (5) zwischen der Absorptionsmembraneinheit (2) und den Desorptionsmembraneinheiten (8, 8') zirkuliert wird;

wobei das Verfahren **gekennzeichnet ist durch**

- Einstellen des pH-Werts des wässrigen Lösungsmittels (3, 31) auf einen Wert, der größer oder gleich $pKaga_1+2$ und kleiner oder gleich 13 ist, **durch** Zusetzen einer basischen Lösung vor dem Absorptionsschritt **und durch** Einstellen des pH-Werts des angereicherten wässrigen Lösungsmittels (30, 30') auf einen Wert, der größer oder gleich $pKaga_1-2$ und kleiner oder gleich $pKaga_1+1$ ist, **durch** Zusetzen einer sauren Lösung vor dem ersten Desorptionsschritt, und auf einen Wert größer oder gleich $pKagb_1-1$ und kleiner oder gleich $pKagb_1+2$, **durch** Zusetzen von Base vor dem zweiten Desorptionsschritt, wobei das wässrige Lösungsmittel eine Pufferkonzentration von weniger als 0,02 mol. L$^{-1}$ aufweist;

**und durch** Regenerieren der Base und der Säure, die dem wässrigen Lösungsmittel (3, 30, 30', 31) zugesetzt wurden, aus dem entgasten wässrigen Lösungsmittel (31) zum Einstellen des pH-Werts **durch** einen Schritt der bipolaren Membranelektrodialyse.

9. Verfahren zur Reinigung eines eintretenden Gasstroms (1) durch Gas-Flüssigkeitsabsorption nach Anspruch 8, **dadurch gekennzeichnet, dass** das zu beseitigende Gasgemisch mehrheitlich mehrere saure Gase $Ga_1$, $Ga_2$, $Ga_n$, aufweisend jeweils eine Säurekonstante $pKaga_1$, $pKaga_2$, $pKaga_n$, umfasst, und die Minderheit mehrere basische Gase $Gb_1$, $Gb_2$, $Gb_n$, aufweisend jeweils eine Säurekonstante $pKagb_1$, $pKagb_2$, $pKagb_n$, umfasst, wobei das Einstellen des pH-Werts des wässrigen Lösungsmittels (3, 31) vor dem Absorptionsschritt durch Zusetzen einer basischen Lösung in Abhängigkeit des pKa-Werts des sauren Gases mit dem höchsten Wert durchgeführt wird, das Einstellen des pH-Werts des angereicherten wässrigen Lösungsmittels (30) vor dem ersten Desorptionsschritt durch Zusetzen einer sauren Lösung in Abhängigkeit des pKa-Werts des sauren Gases mit dem niedrigsten Wert durchgeführt wird, und das Einstellen des pH-Werts des wässrigen Lösungsmittels (30') vor dem zweiten Desorptionsschritt durch Zusetzen einer basischen Lösung in Abhängigkeit des pKa-Werts des basischen Gases mit dem höchsten Wert durchgeführt wird.

10. Verfahren zur Reinigung eines eintretenden Gasstroms (1) durch Gas-Flüssigkeitsabsorption, der ein zu reinigendes Gas $G_0$ und ein zu beseitigendes Gasgemisch umfasst, wobei das Gemisch mindestens ein saures Gas $Ga_1$ und ein basisches Gas $Gb_1$ aufweist, wobei das mindestens eine basische Gas $Gb_1$ überwiegt, wobei das zu reinigende Gas $G_0$ eine Henry-Konstante $Hg_0$ von weniger als $10^{-2}$ mol.$L^{-1}$. $bar^{-1}$ aufweist, das zu beseitigende saure Gas $Ga_1$ eine Henry-Konstante $Hga_1$ größer als $10^{-2}$ mol.$L^{-1}$.$bar^{-1}$ und eine Säurekonstante $pKaga_1$ größer oder gleich 1 und kleiner oder gleich 13 aufweist, und das zu beseitigende basische Gas $Gb_1$ eine Henry-Konstante $Hgb_1$ größer als $10^{-2}$ mol.$L^{-1}$.$bar^{-1}$ und eine Säurekonstante $pKagb_1$ größer oder gleich 1 und kleiner oder gleich 13 aufweist, wobei das Verfahren mindestens die folgenden Schritte umfasst:

- während eines Absorptionsschritts Inkontaktbringen, über eine Absorptionsmembraneinheit (2), die mindestens eine für Gase durchlässige und hydrophobe Membran (20) umfasst, des eintretenden Gasstroms (1) auf einer Seite (21) der Membran (20) mit einem wässrigen Lösungsmittel (3), das geeignet ist, lösliche Gase zu absorbieren, und das auf der anderen Seite (22) der Membran (20) zirkuliert, und Erhalten eines angereicherten wässrigen Lösungsmittels (30) mit dem gelösten sauren Gas $Ga_1$ und dem gelösten basischen Gas Gb1 und eines austretenden Gasstroms (6), der mit dem zu reinigenden Gas $G_0$ angereichert ist, wobei das Lösungsmittel mindestens eine wässrige Lösung bestehend aus Wasser und mindestens einem Salz ausgewählt aus NaCl, KCl, $Na_2SO_4$, $K_2SO_4$, NaNos, KNos NaBr, KBr, NaI, KI, LiCl, LiBr, Lil, $Li_2SO_4$ LiNos umfasst, wobei die molare Konzentration des Salzes in der Absorptionsflüssigkeit zwischen 0,2 und 6 mol. $L^{-1}$ liegt;
- während eines ersten Desorptionsschritts, Inkontaktbringen des angereicherten wässrigen Lösungsmittels (30) mit einer ersten Desorptionsmembraneinheit (8), die mindestens eine für Gase durchlässige und hydrophobe Membran (80) umfasst, und Beseitigen, durch Anwendung einer Transmembranvertiefung, des angereicherten wässrigen Lösungsmittels (30), das auf einer Seite (81) der Membran (80) zirkuliert, wobei das gelöste basische Gas $Gb_1$ durch die Membran (80) zu deren anderen Seite (82) hindurchtritt, um ein wässriges Lösungsmittel (30') zu erhalten, das mit dem sauren Gas $Ga_1$ angereichert ist und von dem gelösten basischen Gas $Gb_1$ befreit ist;
- während eines zweiten Desorptionsschritts, Inkontaktbringen des mit saurem Gas $Ga_1$ angereicherten wässrigen Lösungsmittels (30') mit einer zweiten Desorptionsmembraneinheit (8'), die mindestens eine für Gase durchlässige und hydrophobe Membran (80') umfasst, und Beseitigen, durch Anwendung einer Transmembranvertiefung, des mit saurem Gas $Ga_1$ angereicherten wässrigen Lösungsmittels (30'), das auf einer Seite (81') der Membran (80') zirkuliert, wobei das gelöste basische Gas $Gb_1$ durch die Membran (80') zu deren anderen Seite (82') zum Erhalten eines entgasten wässrigen Lösungsmittels (31) hindurchtritt;
- Rückführen des entgasten wässrigen Lösungsmittels (31) in den Absorptionsschritt, wobei alle Schritte des Verfahrens fortlaufend in einem geschlossenen Kreislauf durchgeführt werden, wobei das wässrige Lösungsmittel (3, 30, 30', 31) in einem geschlossenen Kreislauf (5) zwischen der Absorptionsmembraneinheit (2) und den Desorptionsmembraneinheiten (8, 8') zirkuliert wird;

wobei das Verfahren **gekennzeichnet ist durch**

- Einstellen des pH-Werts des wässrigen Lösungsmittels (3, 31) auf einen Wert größer oder gleich 1 und kleiner oder gleich $pKagb_1$-2 **durch** Zusetzen einer sauren Lösung vor dem Absorptionsschritt, **und durch** Einstellen des pH-Werts des angereicherten wässrigen Lösungsmittels (30, 30') auf einen Wert größer oder gleich $pKagb_1$-1 und kleiner oder gleich $pKagb_1$+2, **durch** Zusetzen einer basischen Lösung vor dem ersten Desorptionsschritt und auf einen Wert größer oder gleich $pKaga_1$-2 und kleiner oder gleich pKagaii+1, **durch** Zusetzen einer sauren Lösung vor dem zweiten Desorptionsschritt, wobei das wässrige Lösungsmittel eine Pufferkonzentration von weniger als 0,02 mol. $L^{-1}$ aufweist, **und durch** Regenerieren der Base und der Säure, die dem wässrigen Lösungsmittel (3, 30, 30', 31) zugesetzt werden, um den pH-Wert einzustellen, aus dem entgasten wässrigen Lösungsmittel (31) **durch** einen Schritt der bipolaren Membranelektrodialyse.

**11.** Verfahren zur Reinigung eines eintretenden Gasstroms (1) durch Gas-Flüssigkeitsabsorption nach Anspruch 10, **dadurch gekennzeichnet, dass** das zu beseitigende Gasgemisch in der Minderheit mehrere saure Gase $Ga_1$, $Ga_2$, $Ga_n$, aufweisend jeweils eine Säurekonstante $pKaga_1$, $pKaga_2$, $pKaga_n$, umfasst, und mehrheitlich mehrere basische Gase $Gb_1$, $Gb_2$, $Gb_n$, aufweisend jeweils eine Säurekonstante $pKagb_1$, $pKagb_2$, $pKagb_n$, umfasst, wobei das Einstellen des pH-Werts des wässrigen Lösungsmittels (3, 31) vor dem Absorptionsschritt durch Zusetzen einer sauren Lösung in Abhängigkeit des pKa-Werts des basischen Gases mit dem niedrigsten Wert durchgeführt wird, das Einstellen des pH-Werts des angereicherten wässrigen Lösungsmittels (30) vor dem ersten Desorptionsschritt durch Zusetzen einer basischen Lösung in Abhängigkeit des pKa-Werts des basischen Gases mit dem höchsten Wert durchgeführt wird, und das Einstellen des pH-Werts des wässrigen Lösungsmittels (30') vor dem zweiten Desorptionsschritt durch Zusetzen einer sauren Lösung in Abhängigkeit des pKa-Werts des sauren Gases mit dem niedrigsten Wert durchgeführt wird.

**12.** Verfahren zur Reinigung eines eintretenden Gasstroms (1) durch Gas-Flüssigkeitsabsorption nach einem der vorstehenden Ansprüche,
**dadurch gekennzeichnet, dass** als Säure zum Einstellen des pH-Werts eine saure Lösung verwendet wird, die dem Salz entspricht, das in dem wässrigen Lösungsmittel (3, 30, 30', 31) verwendet wird, wobei die saure Lösung entweder aus Salzsäure HCl besteht, wenn das Salz aus NaCl, KCl und LiCl ausgewählt ist, oder aus Schwefelsäure $H_2SO_4$, besteht, wenn das Salz aus $Na_2SO_4$, $K_2SO_4$ und $Li_2SO_4$ ausgewählt ist, oder aus Bromwasserstoffsäure Hbr besteht, wenn das Salz aus KBr, NaBr und LiBr ausgewählt ist, oder aus Jodwasserstoffsäure HI besteht, wenn das Salz aus KI, NaI und Lithium LiI ausgewählt ist, oder aus Salpetersäure HNOs besteht, wenn das Salz aus KNOs, NaNOs und LiNOs ausgewählt ist, wobei die Säure in einer molaren Konzentration zugesetzt wird, die jener des Salzes in dem wässrigen Lösungsmittel (3, 30, 30', 31) äquivalent ist.

**13.** Verfahren zur Reinigung eines einströmenden Gasstroms (1) durch Gas-Flüssigkeitsabsorption nach einem der vorstehenden Ansprüche,
**dadurch gekennzeichnet, dass** als Basis für das Einstellen des pH-Werts, der dem in dem wässrigen Lösungsmittel (3, 30, 30', 31) verwendeten Salz entspricht, die basische Lösung verwendet wird, die entweder aus Kaliumhydroxid KOH besteht, wenn das Salz aus KCl, KSO und KNS ausgewählt ist, oder aus Kaliumhydroxid KOH besteht, wenn das Salz aus KCl, KSO, $K_2SO_4$, KBr, KI und KNOs ausgewählt ist, oder aus Natriumhydroxid NaOH besteht, wenn das Salz aus NaCl, $Na_2SO_4$, NaBr, NaI und NaNOs ausgewählt ist, oder aus Lithiumhydroxid LiOH besteht, wenn das Salz aus LiCl, $Li_2SO_4$, LiBr, LiI und LiNOs ausgewählt, wobei die Base in einer molaren Konzentration zugesetzt wird, die jener des Salzes in dem wässrigen Lösungsmittel (3, 30, 30', 31) entspricht.

**14.** Verfahren zur Reinigung eines eintretenden Gasstroms (1) durch Gas-Flüssigkeitsabsorption nach einem der vorstehenden Ansprüche,
**dadurch gekennzeichnet, dass** zur Durchführung des Schritts der Elektrodialyse mit bipolarer Membran:

- ein entgaster wässriger Lösungsmittelstrom (31) von dem geschlossenen Kreislauf (5) zu einem Elektrodialysemodul (13) mit bipolarer Membran mit drei Kammern (137, 138, 139) geleitet wird, wobei das Modul ein Stack umfasst, das aus einer abwechselnden Anordnung von bipolaren (MB) und monopolaren (MA, MC) Membranen (131) besteht, die zwischen einer Kathode (140) und einer Anode (141) liegen;
- der Strom des wässrigen Lösungsmittels, der am Elektrodialysemodul (13) eintrifft, geteilt wird, wobei ein erster Teil des Stroms in einen Tank für saure Lösung (132) geleitet wird, den das Modul (13) aufweist, ein zweiter Teil des Stroms in einen Tank für basische Lösung (133) geleitet wird, den das Modul (13) ebenfalls aufweist, und ein dritter Teil des Stroms in eine erste Kammer, die als "Salze"-Kammer (137) bezeichnet wird, des Stacks, den das Modul (13) umfasst, geleitet wird;
- ein elektrisches Feld zwischen der Kathode (140) und der Anode (141) des Elektrodialyse-Stacks angelegt wird und durch Erzeugen und Migration von Ionen die saure Lösung in der zweiten, als "Säure"-Kammer (138) bezeichneten Kammer des Stacks, den das Modul (13) umfasst, und die basische Lösung in der dritten, als "Base"-Kammer (139) bezeichneten Kammer des Stacks, den das Modul (13) umfasst, ausgebildet wird;
- die saure Lösung zwischen der "Säure"-Kammer (138) des Stacks und dem Tank für die saure Lösung (132) rezirkuliert wird und die basische Lösung zwischen der "Base"-Kammer (139) des Stacks und dem Tank für die basische Lösung (133) rezirkuliert wird;
- die saure Lösung aus dem Tank (132) für die saure Lösung und die basische Lösung aus dem Tank (133) für die basische Lösung zu den Mitteln zum Einstellen des pH-Werts (7, 10) in dem geschlossenen Kreislauf (5) geleitet werden;
- das wässrige Lösungsmittel aus der ersten "Salze"-Kammer (137) des Stacks in den geschlossenen Kreislauf (5) rückgeführt. wird.

EP 3 858 465 B1

15. Verfahren zur Reinigung eines eintretenden Gasstroms (1) durch Gas-Flüssigkeitsabsorption nach dem vorstehenden Anspruch, **dadurch gekennzeichnet, dass die** Konzentration einer sauren Lösung von mehr als 0,2 mol.L$^{-1}$ in der zweiten "Säure"-Kammer (138) des Stacks, den das Elektrodialysemodul (13) umfasst, aufrechterhalten wird und eine Konzentration einer basischen Lösung von mehr als 0,2 mol.L$^{-1}$ in der dritten "Base"-Kammer (139) des Stacks, den das Elektrodialysemodul (13) umfasst, aufrechterhalten wird.

16. Verfahren zur Reinigung eines einströmenden Gasstroms (1) durch Gas-Flüssigkeitsabsorption nach einem der vorstehenden Ansprüche,
**dadurch gekennzeichnet, dass** nach dem Absorptionsschritt und vor dem Einstellen des pH-Werts vor dem Desorptionsschritt oder vor dem ersten Desorptionsschritt ein vorläufiger Entgasungsschritt durchgeführt wird, in dessen Verlauf, durch eine Zwischenentgasungs-Membraneinheit (15), die mindestens eine für Gase durchlässige und hydrophobe Membran (150) umfasst, ein Teil der gelösten Gase durch den Durchgang durch die Membran (150) zu deren anderen Seite (152) in Kontakt gebracht wird und aus dem angereicherten wässrigen Lösungsmittel (30), das auf einer Seite (151) der Membran (150) zirkuliert, durch Anlegen eines transmembranen Unterdrucks beseitigt wird, um ein teilweise entgastes angereichertes wässriges Lösungsmittel (30) zu erhalten, und dieser Teil der gelösten Gase in den eintretenden Gasstrom (1) rückgeführt wird.

17. Verfahren zur Reinigung eines eintretenden Gasstroms (1) durch Gas-Flüssigkeitsabsorption nach einem der vorstehenden Ansprüche,
**dadurch gekennzeichnet, dass** das wässrige Lösungsmittel (3, 30, 30', 31) in dem geschlossenen Kreislauf (5) bei einem relativen Druck zwischen 1 und 7 bar, bevorzugt zwischen 5 und 6 bar, zirkuliert wird.

18. Verfahren zur Reinigung eines eintretenden Gasstroms (1) durch Gas-Flüssigkeitsabsorption nach einem der vorstehenden Ansprüche,
**dadurch gekennzeichnet, dass** der eintretende Gasstrom (1), der in die Absorptionsmembraneinheit (2) eintritt, bei einem relativen Druck zwischen 10 mbar und 6 bar, bevorzugt bei einem relativen Druck zwischen 4,5 und 5,75 bar, noch stärker bevorzugt von gleich 5 bar, aufrechterhalten wird.

19. Verfahren zur Reinigung eines eintretenden Gasstroms (1) durch Gas-Flüssigkeitsabsorption nach den Ansprüchen 17 und 18,
**dadurch gekennzeichnet, dass** zwischen dem Druck des absorbierenden wässrigen Lösungsmittels (3, 30, 30', 31), das in dem geschlossenen Kreislauf 5 zirkuliert, und dem Druck des Gasstroms (1) bei der Absorption eine positive Druckdifferenz zwischen 0,2 und 1 bar aufrechterhalten wird.

20. Verfahren zur Reinigung eines eintretenden Gasstroms (1) durch Gas-Flüssigkeitsabsorption nach einem der vorstehenden Ansprüche,
**dadurch gekennzeichnet, dass** in der Desorptionsmembraneinheit (8) auf der Seite (82) der Austrittsmembran (80) der gelösten Gase (9) ein absoluter Druck angelegt wird, der zwischen 25 mbar und 1 bar beträgt.

21. Anlage (100) zur Reinigung eines eintretenden Gasstroms (1), umfassend ein zu reinigendes Gas $G_0$ und mindestens ein zu beseitigendes saures Gas Ga1 und/oder basisches Gas $Gb_1$, zur Durchführung des Verfahrens nach einem der vorstehenden Ansprüche, umfassend mindestens eine Absorptionsmembraneinheit (2) mit mindestens einer für Gase durchlässige und hydrophobe Membran (20), wobei die Einheit (2) auf einer Seite (21) der Membran (20) mit einem Einlass (4) des eintretenden Gasstroms (1) und mit einem Auslass (17) des austretenden Gasstroms (6), der mit dem zu reinigenden Gas $G_0$ angereichert ist, verbunden ist, und auf der anderen Seite (22) der Membran (20) mit einem geschlossenen Kreislauf (5) verbunden ist, in dem ein wässriges Lösungsmittel (3), welches das mindestens eine zu beseitigende Gas absorbiert, in einer Schleife zirkuliert, wobei die Anlage (100) ferner stromabwärts der Absorptionsmembraneinheit (2), unter Berücksichtigung der Zirkulationsrichtung des wässrigen Lösungsmittels in dem geschlossenen Kreislauf, mindestens eine Desorptionsmembraneinheit (8) umfasst, die mindestens eine für Gase durchlässige und hydrophobe Membran (80) umfasst, die geeignet ist, den Durchgang von mindestens einem Teil des in dem wässrigen Lösungsmittel (3) gelösten zu beseitigenden Gases und seine Beseitigung zu ermöglichen, wobei die Anlage (100) noch mindestens einerseits erste Mittel (10) zum Regulieren des pH-Werts des wässrigen Lösungsmittels (3, 31) umfasst, die in dem geschlossenen Kreislauf (5) zwischen der mindestens einen Desorptionsmembraneinheit (8) und der Absorptionsmembraneinheit (2) positioniert sind, unter Berücksichtigung der Zirkulationsrichtung des wässrigen Lösungsmittels (3, 30, 31) innerhalb des geschlossenen Kreislaufs (5), und andererseits zweite Mittel (7) zum Regulieren des pH-Werts des wässrigen Lösungsmittels (30) umfasst, die in dem geschlossenen Kreislauf (5) zwischen der Absorptionsmembraneinheit (2) und der mindestens einen Desorptionsmembraneinheit (8) positioniert sind, unter Berücksichtigung der Zirkulationsrichtung des wässrigen Lösungsmittels (3, 30, 31) innerhalb des geschlossenen Kreislaufs (5), wobei das Lösungsmittel mindestens eine wässrige Lösung bestehend aus Wasser und mindestens einem Salz ausgewählt aus NaCl, KCl, Na₂SO₄, K₂SO₄, NaNOs, KNOs, NaBr, KBr, NaI, KI, LiCl, LiBr, LiI, Li₂SO₄, LiNOs umfasst, wobei die molare Konzentration des Salzes in der absorbierenden Flüssigkeit zwischen 0,2 und 6 mol. L$^{-1}$ beträgt, wobei die Anlage (100) **dadurch gekennzeichnet ist, dass** sie mindestens ein Elektrodialysemodul (13) mit bipolarer Membran mit drei Kammern (137, 138, 139) umfasst, um die verwendete Säure und Base aus dem entgasten wässrigen Lösungsmittel (31) mittels eines Stacks zu regenerieren, der aus einer Reihe von bipolaren und monopolaren Membranen (131) besteht, die abwechselnd angeordnet sind und zwischen einer Kathode (140) und einer Anode (141) liegen, die das Modul (13) umfasst, das mit einem Tank (14) für entgastes wässriges Lösungsmittel (31) verbunden ist, der in dem geschlossenen Kreislauf (5) zwischen der mindestens einen Desorptionsmembraneinheit (8) und der Absorptionsmembraneinheit (2) positioniert ist, unter Berücksichtigung der Zirkulationsrichtung des wässrigen Lösungsmittels (3, 30, 31) innerhalb des geschlossenen Kreislaufs (5), wobei Mittel zum Rezirkulieren (134) des wässrigen Lösungsmittels (31) zwischen dem Tank (14) und dem Elektrodialysemodul mit bipolarer Membran (13) ebenfalls vorgesehen sind, sowie Mittel zum Weiterleiten (135, 136) der regenerierten sauren und basischen Lösungen jeweils aus einem Tank für saure Lösung (132) und einem Tank für basische Lösung (133), die das Modul (13) umfasst, zu den Mitteln (7, 10) zum Regulieren des pH-Werts, wobei das wässrige Lösungsmittel eine Pufferkonzentration von weniger als 0,02 mol. L$^{-1}$ in der Anlage (100) aufweist.

22. Anlage (100) zur Reinigung eines eintretenden Gasstroms (1) nach Anspruch 21, **dadurch gekennzeichnet, dass** die ersten Mittel (10) zum Regulieren des pH-Werts des wässrigen Lösungsmittels (3, 31) aus mindestens einem Mittel zum Messen des pH-Werts, einem Tank zum Lagern einer sauren Lösung und/oder einem Tank zum Lagern einer basischen Lösung, und aus Mitteln zum Einspritzen der sauren oder basischen Lösung innerhalb des geschlossenen Kreislaufs (5) zwischen der mindestens einen Desorptionsmembraneinheit (8) und der Absorptionsmembraneinheit bestehen (2), **und dadurch, dass** die zweiten Mittel (7) zum Regulieren des pH-Werts des wässrigen Lösungsmittels (30) aus mindestens einem Mittel zum Messen des pH-Werts, einem Tank zum Lagern einer sauren Lösung und/oder einem Tank zum Lagern einer basischen Lösung und Mitteln zum Einspritzen der sauren oder basischen Lösung innerhalb des geschlossenen Kreislaufs (5) zwischen der Absorptionsmembraneinheit (2) und der mindestens einen Desorptionsmembraneinheit (8) bestehen.

23. Anlage (100) zur Reinigung eines eintretenden Gasstroms nach einem der Ansprüche 21 oder 22, **dadurch gekennzeichnet, dass** sie stromabwärts der Absorptionsmembraneinheit (2), unter Berücksichtigung der Zirkulationsrichtung des wässrigen Lösungsmittels (3, 30, 30', 31) im geschlossenen Kreislauf (5), eine erste Desorptionsmembraneinheit (8) und, stromabwärts davon, eine zweite Desorptionsmembraneinheit (8') sowie dritte Mittel (7') zum Regulieren des pH-Werts des wässrigen Lösungsmittels (30'), die in dem geschlossenen Kreislauf (5) zwischen der ersten Desorptionsmembraneinheit (8) und der zweiten Desorptionsmembraneinheit (8) positioniert sind, umfasst, wobei die dritten Mittel (7') zum Regulieren des pH-Werts des wässrigen Lösungsmittels (30') aus mindestens einem Mittel zum Messen des pH-Werts, einem Tank zum Lagern einer sauren Lösung und/oder einem Tank zum Lagern einer basischen Lösung und Mitteln zum Einspritzen der sauren oder basischen Lösung in den geschlossenen Kreislauf (5) zwischen der ersten Desorptionsmembraneinheit (8) und der zweiten Desorptionsmembraneinheit (8') bestehen.

24. Anlage (100) zur Reinigung eines eintretenden Gasstroms nach einem der Ansprüche 21 bis 23, **dadurch gekennzeichnet, dass** sie in dem geschlossenen Kreislauf (5) zwischen der Absorptionsmembraneinheit (2) und der mindestens einen Desorptionsmembraneinheit (8), unter Berücksichtigung der Zirkulationsrichtung des wässrigen Lösungsmittels (3, 30, 31) innerhalb des geschlossenen Kreislaufs (5), eine Zwischenentgasungs-Membraneinheit (15) mit mindestens einer für Gase durchlässigen und hydrophoben Membran (150) umfasst, die geeignet ist, den Durchgang eines Teils der gelösten Gase zu ermöglichen, sowie eine Rezirkulationsschleife (16), die mit dem Einlass (4) des eintretenden Gasstroms (1) verbunden ist, den die Anlage (100) umfasst, um den Teil der gelösten Gase zu diesem Einlass (4) des eintretenden Gasstroms (1) rückzuführen.

## Claims

1. A method for purifying, by gas-liquid absorption, an incoming gas flow (1) comprising a gas $G_0$ to be purified and at least one acid gas $Ga_1$ to be removed, said gas $G_0$ to be purified having a Henry's law $H_{G0}$ less than $10^{-2}$ mol.L$^{-1}$.bar$^{-1}$ and said acid gas $Ga_1$ to be removed having a Henry's law $H_{Ga1}$ greater than $10^{-2}$ mol.L$^{-1}$.bar$^{-1}$ and an acid dissociation constant $pKa_{Ga1}$ greater than or equal to 1 and less than or equal to 13, said method comprising at least the following steps:

- during an absorption step, through an absorption membrane unit (2) comprising at least one gas-permeable and hydrophobic membrane (20), said incoming gas flow (1) on one side (21) of said membrane (20) is brought into contact with an aqueous solvent (3), capable of absorbing the soluble gases and circulating on the other side (22) of said membrane (20), and a rich aqueous solvent (30) comprising said acid gas $Ga_1$ dissolved and an exiting gas flow (6) enriched with gas $G_0$ to be purified are obtained, said solvent comprising at least one aqueous solution comprising water and at least one salt selected from NaCl, KCl, $Na_2SO_4$, $K_2SO_4$, NaNOs, KNOs NaBr, KBr, NaI, KI, LiCl, LiBr, LiI, $Li_2SO_4$LiNO$_3$, the molar concentration of salt in the absorbent liquid is between 0.2 and 6 mol.$L^{-1}$ ;

- during a desorption step, said rich aqueous solvent (30) is brought into contact with a desorption membrane unit (8) comprising at least one membrane (80) that is permeable to gases and hydrophobic, and, by applying a transmembrane negative pressure, said dissolved acid gas $Ga_1$ is removed from said rich aqueous solvent (30) which circulates on one side (81) of said membrane (80), by passing through said membrane (80) toward the other side (82) thereof, in order to obtain a degassed aqueous solvent (31);

- said degassed aqueous solvent (31) is recycled in the absorption step, all of the steps of said method being continuously operated in a closed loop, said aqueous solvent (3, 30, 31) being circulated in a closed-loop circuit (5) between said absorption membrane unit (2) and said desorption membrane unit (8);

said method being **characterized in that:**

- the pH of the aqueous solvent (3, 31) is adjusted to a value greater than or equal to $pKa_{Ga1}+2$ and less than or equal to 13, by adding a basic solution, before the absorption step, and the pH of the rich aqueous solvent (30) is adjusted to a value greater than or equal to $pKa_{Ga1}-2$ and less than or equal to $pKa_{Ga1}+1$, by adding an acid solution, before the desorption step, said aqueous solvent having a buffer agent concentration of less than 0.02 mol.$L^{-1}$ ;

and **in that** the base and the acid are regenerated from the degassed aqueous solvent (31), added to said aqueous solvent (3, 30, 30', 31) to adjust the pH, by a step of bipolar membrane electrodialysis.

2. The method for purifying, by gas-liquid absorption, an incoming gas flow (1) according to claim 1, **characterized in that** said gas $G_0$ to be purified consists of dihydrogen $H_2$ having a Henry's law $H_{G0}$ equal to 7.6x$10^{-4}$mol.$L^{-1}$.bar$^{-1}$ and said acid gas $Ga_1$ to be removed consists of carbon dioxide $CO_2$ having a Henry's law $H_{Ga1}$ equal to $3.35 \times 10^{-2}$ mol.$L^{-1}$.bar$^{-1}$ and an acid dissociation constant $pKa_{Ga1}$ equal to 6.35.

3. The method for purifying, by gas-liquid absorption, an incoming gas flow (1) according to claim 1, **characterized in that** said gas $G_0$ to be purified consists of methane $CH_4$ having a Henry's law $H_{G0}$ equal to 1.3x$10^{-3}$mol.$L^{-1}$.bar$^{-1}$ and said acid gas $Ga_1$ to be removed consists of carbon dioxide $CO_2$ having a Henry's law $H_{Ga1}$ equal to $3.35 \times 10^{-2}$ mol.$L^{-1}$.bar$^{-1}$ and an acid dissociation constant $pKa_{Ga1}$ equal to 6.35.

4. The method for purifying, by gas-liquid absorption, an incoming gas flow (1) according to claim 2 or claim 3, **characterized in that** the pH of the aqueous solvent (3, 31) between 9.0 and 12.25 is adjusted by adding a basic solution to the aqueous solvent (3, 31) before the absorption step and the pH of the rich aqueous solvent (30) between 4.9 and 6.6 is adjusted by adding an acid solution to said solvent (30) before the desorption step.

5. The method for purifying, by gas-liquid absorption, an incoming gas flow (1) according to claim 1, said incoming gas flow (1) comprising a gas $G_0$ to be purified and a plurality of acid gases $Ga_1$, $Ga_2$, $Ga_n$ to be removed, having respectively an acid dissociation constant $pKa_{Ga1}$, $pKa_{Ga2}$, $pKa_{Gan}$,
**characterized in that** the pH of the aqueous solvent (3, 31) is adjusted before the absorption step, by adding a basic solution, depending on the pKa of the acid gas having the highest value, and the pH of the rich aqueous solvent (30) is adjusted before the desorption step, by adding an acid solution, depending on the pKa of the acid gas having the lowest value.

6. The method for purifying, by gas-liquid absorption, an incoming gas flow (1) comprising a gas $G_0$ to be purified and at least one basic gas $Ga_1$ to be removed, said gas $G_0$ to be purified having a Henry's law $H_{G0}$ less than $10^{-2}$mol.$L^{-1}$.bar$^{-1}$ and said basic gas $Ga_1$ to be removed having a Henry's law $H_{gb1}$ greater than $10^{-2}$mol.$L^{-1}$.bar$^{-1}$ and an acid dissociation constant $pKa_{Gb1}$ greater than or equal to 1 and less than or equal to 13, said method comprising at least the following steps:

- during an absorption step, through an absorption membrane unit (2) comprising at least one gas-permeable and hydrophobic membrane (20), said incoming gas flow (1) on one side (21) of said membrane (20) is brought into contact with an aqueous solvent (3), capable of absorbing the soluble gases and circulating on the other side (22) of said membrane (20), and a rich aqueous solvent (30) comprising said basic gas $Gb_1$ dissolved and an exiting gas flow (6) enriched with gas $G_0$ to be purified are obtained, said solvent comprising at least one aqueous solution comprising water and at least one salt selected from NaCl, KCl, $Na_2SO_4$, $K_2SO_4$, NaNOs, KNOs NaBr, KBr, Nal, KI, LiCI, LiBr, Lil, $Li_2SO_4LiNO_3$, the molar concentration of salt in the absorbent liquid is between 0.2 and 6 mol.L$^{-1}$ ;

- during a desorption step, said rich aqueous solvent (30) is brought into contact with a desorption membrane unit (8) comprising at least one membrane (80) that is permeable to gases and hydrophobic, and, by applying a transmembrane negative pressure, said dissolved basic gas $Gb_1$ is removed from said rich aqueous solvent (30) which circulates on one side (81) of said membrane (80), by passing through said membrane (80) toward the other side (82) thereof, in order to obtain a degassed aqueous solvent (31);

- said degassed aqueous solvent (31) is recycled in the absorption step, all of the steps of said method being continuously operated in a closed loop, said aqueous solvent (3, 30, 31) being circulated in a closed-loop circuit (5) between said absorption membrane unit (2) and said desorption membrane unit (8);

said method being **characterized in that:**

- the pH of the aqueous solvent (3, 31) is adjusted to a value greater than or equal to 1 and less than or equal to $pKa_{Ga1}-2$, by adding an acid solution, before the absorption step, and the pH of the rich aqueous solvent (30) is adjusted to a value greater than or equal to $pKa_{Gb1}-1$ and less than or equal to $pKa_{Gb1}+2$, by adding a basic solution, before the desorption step, said aqueous solvent having a buffer agent concentration of less than 0.02 mol.L$^{-1}$ ;

and **in that** the base and the acid are regenerated from the degassed aqueous solvent (31), added to said aqueous solvent (3, 30, 30', 31) to adjust the pH, by a step of bipolar membrane electrodialysis.

7. The method for purifying, by gas-liquid absorption, an incoming gas flow (1) according to claim 6, said incoming gas flow (1) comprising a gas $G_0$ to be purified and a plurality of basic gases $Gb_1$, $Gb_2$, $Gb_n$ to be removed, having respectively an acid dissociation constant $pKa_{Gb1}$, $pKa_{Gb2}$, $pKa_{Gbn}$,
**characterized in that** the pH of the aqueous solvent (3, 31) is adjusted before the absorption step, by adding an acid solution, depending on the pKa of the basic gas having the lowest value, and the pH of the rich aqueous solvent (30) is adjusted before the desorption step, by adding a basic solution, depending on the pKa of the basic gas having the highest value.

8. A method for purifying, by gas-liquid absorption, an incoming gas flow (1) comprising a gas $G_0$ to be purified and a mixture of gas to be removed, said mixture comprising at least one acid gas $Ga_1$ and a basic gas $Gb_1$, said at least one acid gas $Ga_1$ being predominant, said gas $G_0$ to be purified having a Henry's law $H_{G0}$ less than $10^{-2}$mol.L$^{-1}$.bar$^{-1}$, said acid gas $Ga_1$ to be removed having a Henry's law $H_{Ga1}$ greater than $10^{-2}$ mol.L$^{-1}$.bar$^{-1}$ and an acid dissociation constant $pKa_{Ga1}$ greater than or equal to 1 and less than or equal to 13 and said basic gas $Gb_1$ to be removed having a Henry's law $H_{Gb1}$ greater than $10^{-2}$mol.L$^{-1}$.bar$^{-1}$ and an acid dissociation constant $pKa_{Gb1}$ greater than or equal to 1 and less than or equal to 13, said method comprising, at least, the following steps:

- during an absorption step, through an absorption membrane unit (2) comprising at least one gas-permeable and hydrophobic membrane (20), said incoming gas flow (1) on one side (21) of said membrane (20) is brought into contact with an aqueous solvent (3), capable of absorbing the soluble gases and circulating on the other side (22) of said membrane (20), and a rich aqueous solvent (30) comprising said acid gas $Ga_1$ dissolved and said basic gas $Gb_1$ dissolved and an exiting gas flow (6) enriched with gas $G_0$ to be purified are obtained, said solvent comprising at least one aqueous solution comprising water and at least one salt selected from NaCl, KCl, $Na_2SO_4$, $K_2SO_4$, NaNOs, KNOs NaBr, KBr, Nal, KI, LiCI, LiBr, Lil, $Li_2SO_4LiNO_3$, the molar concentration of salt in the absorbent liquid is between 0.2 and 6 mol.L$^{-1}$ ;

- during a first desorption step, said rich aqueous solvent (30) is brought into contact with a first desorption membrane unit (8) comprising at least one membrane (80) that is permeable to gases and hydrophobic, and, by applying a transmembrane vacuum, said dissolved acid gas $Ga_1$ is removed from said rich aqueous solvent (30) which circulates on one side (81) of said membrane (80), by passing through said membrane (80) toward the other side (82) thereof, in order to obtain an aqueous solvent rich in basic gas $Gb_1$ (30') and stripped of said dissolved acid gas $Ga_1$ ;

- during a second desorption step, said aqueous solvent rich in basic gas $Gb_1$ (30') is brought into contact with a second desorption membrane unit (8') comprising at least one membrane (80') that is permeable to gases and hydrophobic, and, by applying a transmembrane negative pressure, said dissolved basic gas $Gb_1$ is removed from the aqueous solvent rich in basic gas $Gb_1$ (30') which circulates on one side (81') of said membrane (80'), by passing through said membrane (80') toward the other side (82) thereof, in order to obtain a degassed aqueous solvent (31);

- said degassed aqueous solvent (31) is recycled in the absorption step, all of the steps of said method being continuously operated in a closed loop, said aqueous solvent (3, 30, 30', 31) being circulated in a closed-loop circuit (5) between said absorption membrane unit (2) and said desorption membrane units (8, 8');

said method being **characterized in that:**

- the pH of the aqueous solvent (3, 31) is adjusted to a value greater than or equal to $pKa_{Ga1}+2$ and less than or equal to 13, by adding a basic solution, before the absorption step, and **in that** the pH of the rich aqueous solvent (30, 30') is adjusted to a value greater than or equal to $pKa_{Ga1}-2$ and less than or equal to $pKa_{Ga1}+1$, by adding an acid solution, before the first desorption step and to a value greater than or equal to $pKa_{Gb1}-1$ and less than or equal to $pKa_{Gb1}+2$ by adding a base, before the second desorption step, said aqueous solvent having a buffer agent concentration of less than 0.02 mol.L-1 ;

and **in that** the base and the acid are regenerated from the degassed aqueous solvent (31), added to said aqueous solvent (3, 30, 30', 31) to adjust the pH, by a step of bipolar membrane electrodialysis.

9. The method for purifying, by gas-liquid absorption, an incoming gas flow (1) according to claim 8, **characterized in that** said gas mixture to be removed predominantly comprises a plurality of acid gases $Ga_1$, $Ga_2$, $Ga_n$, respectively having an acid dissociation constant $pKa_{Ga1}$, $pKa_{Ga2}$, $pKa_{Gan}$ and, in the minority, a plurality of basic gases $Gb_1$, $Gb_2$, $Gb_n$, having respectively an acid dissociation constant $pKa_{Gb1}$, $pKa_{Gb2}$, $pKa_{Gbn}$, the adjustment of the pH of the aqueous solvent (3, 31) before the absorption step being carried out, by adding a basic solution, based on the pKa of the acid gas having the highest value, the adjustment of the pH of the rich aqueous solvent (30) before the first desorption step being carried out, by adding an acid solution, depending on the pKa of the acid gas having the lowest value, and the adjustment of the pH of the aqueous solvent (30') before the second desorption step being carried out, by adding a basic solution, as a function of the pKa of the basic gas having the highest value.

10. The method for purifying, by gas-liquid absorption, an incoming gas flow (1) comprising a gas $G_0$ to be purified and a mixture of gas to be removed, said mixture comprising at least one acid gas $Ga_1$ and a basic gas $Gb_1$, said at least one basic gas $Gb_1$ being predominant, said gas $G_0$ to be purified having a Henry's law $H_{G0}$ less than $10^{-2}$mol.L$^{-1}$.bar$^{-1}$, said acid gas $Ga_1$ to be removed having a Henry's law $H_{Ga1}$ greater than $10^{-2}$ mol.L$^{-1}$.bar$^{-1}$ and an acid dissociation constant $pKa_{Ga1}$ greater than or equal to 1 and less than or equal to 13 and said basic gas $Gb_1$ to be removed having a Henry's law $H_{Gb1}$ greater than $10^{-2}$mol.L$^{-1}$.bar$^{-1}$ and an acid dissociation constant $pKa_{Gb1}$ greater than or equal to 1 and less than or equal to 13, said method comprising, at least, the following steps:

- during an absorption step, through an absorption membrane unit (2) comprising at least one gas-permeable and hydrophobic membrane (20), said incoming gas flow (1) on one side (21) of said membrane (20) is brought into contact with an aqueous solvent (3), capable of absorbing the soluble gases, circulating on the other side (22) of said membrane (20), and a rich aqueous solvent (30) comprising said acid gas $Ga_1$ dissolved and said basic gas $Gb_1$ dissolved and an exiting gas flow (6) enriched with gas $G_0$ to be purified are obtained, said solvent comprising at least one aqueous solution comprising water and at least one salt selected from NaCl, KCl, $Na_2SO_4$, $K_2SO_4$, NaNOs, KNOs NaBr, KBr, NaI, KI, LiCl, LiBr, LiI, $Li_2SO_4$LiNO$_3$, the molar concentration of salt in the absorbent liquid is between 0.2 and 6 mol.L$^{-1}$ ;

- during a first desorption step, said rich aqueous solvent (30) is brought into contact with a first desorption membrane unit (8) comprising at least one membrane (80) that is permeable to gases and hydrophobic, and, by applying a transmembrane vacuum, said dissolved basic gas $Gb_1$ is removed from said rich aqueous solvent (30) which circulates on one side (81) of said membrane (80), by passing through said membrane (80) toward the other side (82) thereof, in order to obtain an aqueous solvent (30') rich in acid gas $Ga_1$ and stripped of said dissolved basic gas $Gb_1$ ;

- during a second desorption step, said aqueous solvent rich (30') in basic gas $Ga_1$ is brought into contact with a second desorption membrane unit (8') comprising at least one membrane (80') that is permeable to gases and hydrophobic, and, by applying a transmembrane negative pressure, said dissolved acid gas $Ga_1$ is removed from the aqueous solvent rich (30') in basic gas $Ga_1$ which circulates on one side (81') of said membrane (80'), by passing through said membrane (80') toward the other side (82') thereof, in order to obtain a degassed aqueous solvent (31);
- said degassed aqueous solvent (31) is recycled in the absorption step, all of the steps of said method being continuously operated in a closed loop, said aqueous solvent (3, 30, 30', 31) being circulated in a closed-loop circuit (5) between said absorption membrane unit (2) and said desorption membrane units (8, 8');

said method being **characterized in that:**

- the pH of the aqueous solvent (3, 31) is adjusted to a value greater than or equal to 1 and less than or equal to $pKa_{Gb1}$-2 by adding a basic solution, before the absorption step, and **in that** the pH of the rich aqueous solvent (30, 30') is adjusted to a value greater than or equal to $pKa_{Gb1}$-1 and less than or equal to $pKa_{Gb1}$+2, by adding a basic solution, before the first desorption step and to a value greater than or equal to $pKa_{Ga1}$-2 and less than or equal to $pKa_{Ga1}$+1 by adding an acid solution, before the second desorption step, said aqueous solvent having a buffer agent concentration of less than 0.02 mol.L$^{-1}$, and **in that** the base and acid added to said aqueous solvent (3, 30, 30', 31) are regenerated from the degassed aqueous solvent (31) to adjust the pH, through a step of bipolar membrane electrodialysis.

11. The method for purifying, by gas-liquid absorption, an incoming gas flow (1) according to claim 10, **characterized in that** said gas mixture to be removed comprises, in the minority, a plurality of acid gases $Ga_1$, $Ga_2$, $Ga_n$, respectively having an acid dissociation constant $pKa_{Ga1}$, $pKa_{Ga2}$, $pKa_{Gan}$, and predominantly, a plurality of basic gases $Gb_1$, $Gb_2$, $Gb_n$, having respectively an acid dissociation constant $pKa_{Gb1}$, $pKa_{Gb2}$, $pKa_{Gbn}$, the adjustment of the pH of the aqueous solvent (3, 31) before the absorption step being carried out, by adding an acid solution, based on the pKa of the basic gas having the lowest value, the adjustment of the pH of the rich aqueous solvent (30) before the first desorption step being carried out, by adding a basic solution, depending on the pKa of the basic gas having the highest value, and the adjustment of the pH of the aqueous solvent (30') before the second desorption step being carried out, by adding an acid solution, as a function of the pKa of the acid gas having the lowest value.

12. The method for purifying, by gas-liquid absorption, an incoming gas flow (1) according to any one of the preceding claims, **characterized in that** it is used, as acid for the adjustment of the pH, an acid solution corresponding to the salt used in the aqueous solvent (3, 30, 30', 31), said acid solution consisting either of hydrochloric acid HCl when the salt is chosen from NaCl, KCl and LiCl, or in sulfuric acid $H_2SO_4$ when said salt is selected from $Na_2SO_4$, the $K_2SO_4$ and $Li_2SO_4$, either in hydrobromic acid HBr when the salt is selected from KBr, NaBr and LiBr, or in hydroiodic acid HI when said salt is selected from KI, NaI and lithium LiI, or else nitric acid HNOs when said salt is selected from KNOs, NaNOs and LiNOs, said acid being added at a molar concentration equivalent to that of the salt in said aqueous solvent (3, 30, 30', 31).

13. The method for purifying, by gas-liquid absorption, an incoming gas flow (1) according to any one of the preceding claims, **characterized in that,** as a base for adjusting the pH, corresponding to the salt used in the aqueous solvent (3, 30, 30', 31), said basic solution is used consisting of potassium hydroxide KOH when the salt is selected from KCl, $K_2SO_4$, KBr, KI and KNO3, or of sodium hydroxide NaOH when the salt is chosen from NaCl, $Na_2SO_4$, NaBr, NaI and NaNOs, or of lithium hydroxide LiOH when the salt is chosen from LiCl, $Li_2SO_4$, LiBr, LiI and LiNOs wherein said base is added at a molar concentration equivalent to that of the salt in said aqueous solvent (3, 30, 30', 31).

14. The method for purifying, by gas-liquid absorption, an incoming gas flow (1) according to any one of the preceding claims, **characterized in that,** for implementing the bipolar membrane electrodialysis step:

- a degassed aqueous solvent flow (31) is conveyed from the closed loop circuit (5) to a bipolar membrane electrodialysis module (13) with three compartments (137, 138, 139), said module comprising a stack which consists of an alternating assembly of bipolar (MB) and monopolar (MA, MC) membranes (131), comprised between a cathode (140) and an anode (141);
- said flow of aqueous solvent arriving at the electrodialysis module (13) is divided, a first part of the flow being conveyed to an acid solution tank (132) that said module (13) comprises, a second part of the flow being conveyed to a basic solution tank (133) that said module (13) also comprises, a third part of the flow supplying a first compartment, called the "salts" compartment (137), of said stack that said module (13) comprises ;
- an electric field is applied between the cathode (140) and the anode (141) of said electrodialysis stack and, by ion generation and migration, the acid solution is formed in the second compartment, referred to as the "acid" compartment (138), of said stack that said module (13) comprises, and the basic solution is formed in the third compartment, referred to as the "base" compartment (139) of said stack that said module (13) comprises ;
- the acid solution is recirculated between the "acid" compartment (138) of said stack and said acid solution tank (132) and the basic solution is recirculated between the "base" compartment (139) of said stack and said basic solution tank (133);

- the acid solution is conveyed from said acid solution tank (132) and the basic solution is conveyed from said basic solution tank (133) to the pH adjustment means (7,10) that the closed-loop circuit (5) comprises;

- the aqueous solvent is returned from the first "salts" compartment (137) of said stack to the closed-loop circuit (5).

15. The method for purifying, by gas-liquid absorption, an incoming gas flow (1) according to the preceding claim, **characterized in that** an acid solution concentration of greater than 0.2 mol.L$^{-1}$ is maintained in the second "acid" compartment (138) of said stack that said electrodialysis module (13) comprises and a concentration of basic solution greater than 0.2 mol.L$^{-1}$ is maintained in the third "base" compartment (139) of said stack that said electrodialysis module (13) comprises.

16. The method for purifying, by gas-liquid absorption, an incoming gas flow (1) according to any one of the preceding claims, **characterized in that,** according to the absorption step, and prior to the adjustment of the pH before the desorption step or before the first desorption step, a preliminary degassing step is carried out during which, through an intermediate degassing membrane unit (15) comprising at least one gas-permeable and hydrophobic membrane (150), is brought into contact, and, by applying a transmembrane negative pressure, a fraction of the dissolved gases is removed from said rich aqueous solvent (30) circulating on one side (151) of said membrane (150), by passing through said membrane (150) toward the other side (152) thereof, in order to obtain a partially degassed aqueous solvent (30), and said fraction of the dissolved gases is returned to the incoming gas flow (1).

17. The method for purifying, by gas-liquid absorption, an incoming gas flow (1) according to any one of the preceding claims, **characterized in that** the absorbent aqueous solvent (3, 30, 30', 31) is circulated in the closed-loop circuit (5) at a relative pressure between 1 and 7 bar, preferably between 5 and 6 bar.

18. The method for purifying, by gas-liquid absorption, an incoming gas flow (1) according to any one of the preceding claims, **characterized in that** the incoming gas flow (1) which arrives in the absorption membrane unit (2) is kept at a relative pressure of between 10 mbar and 6 bar, preferably at a relative pressure of between 4.5 and 5.75 bar, even more preferably 5 bar.

19. The method for purifying, by gas-liquid absorption, an incoming gas flow (1) according to claims 17 and 18, **characterized in that** between the pressure of the absorbent aqueous solvent (3, 30, 30', 31) circulating in the closed-loop circuit 5, and the pressure of the gas flow (1) at absorption, a positive pressure difference between 0.2 and 1 bar is maintained.

20. The method for purifying, by gas-liquid absorption, an incoming gas flow (1) according to any one of the preceding claims, **characterized in that** an absolute pressure of between 25 mbar and 1 bar is applied in the desorption membrane unit (8) on the side (82) of the membrane (80) where the dissolved gases (9) exit.

21. A facility (100) for purifying an incoming gas flow (1) comprising a gas $G_0$ to be purified and at least one acid gas $Ga_1$ and/or a basic gas $Gb_1$, to be eliminated, for the implementation of the method according to any one of the preceding claims, comprising, at least, an absorption membrane unit (2) comprising at least one gas-permeable and hydrophobic membrane (20), said unit (2) being connected, on one side (21) of said membrane (20), to an inlet (4) of the incoming gas flow (1), and to an outlet (17) of the exiting gas flow (6) enriched with gas $G_0$ to be purified, and, on the other side (22) of said membrane (20), to a closed circuit (5) in which an aqueous solvent (3) that absorbs said at least one gas to be removed circulates in a loop, said facility (100) further comprising, downstream of said absorption membrane unit (2), taking into account the direction of circulation of said aqueous solvent in the closed circuit, at least one desorption membrane unit (8), which comprises at least one membrane (80) permeable to gases and hydrophobic, capable of allowing at least part of the gas to be removed that is dissolved in said aqueous solvent (3) and of allowing its removal, said facility (100) further comprising at least first means (10) for regulating the pH of the aqueous solvent (3, 31), positioned on the closed circuit (5) between said at least one desorption membrane unit (8) and said absorption membrane unit (2), taking into account the direction of circulation of the aqueous solvent (3, 30, 31) within said closed circuit (5), and second means (7) for regulating the pH of the aqueous solvent (30) positioned on the closed circuit (5) between the absorption membrane unit (2) and said at least one desorption membrane unit (8), taking into account the direction of circulation of the aqueous solvent (3, 30, 31) within said closed circuit (5), said solvent comprising at least one aqueous solution comprising water and at least one salt chosen from among NaCl, KCl, $Na_2SO_4$, $K_2SO_4$, NaNOs, KNOs NaBr, KBr, NaI, KI, LiCl, LiBr, LiI, $Li_2SO_4$LiNO$_3$, the molar concentration of salt in the absorbent liquid being between 0.2 and 6 mol.L$^{-1}$, said facility (100) being **characterized in that it** comprises at least one bipolar membrane electrodialysis module (13) with three compartments (137, 138, 139) to regenerate the acid and base used from the degassed aqueous solvent (31) by means of a stack consisting of a series of bipolar and monopolar membranes (131), arranged alternately, and comprised between a cathode (140) and an anode (141), which said module (13) comprises, said module being connected to a degassed aqueous solvent (31) tank (14) positioned on said closed circuit (5), between said at least one desorption membrane unit (8) and the absorption membrane unit (2), taking into account the direction of circulation of the aqueous solvent (3, 30, 31) within said closed circuit (5), the means for recirculating (134) said aqueous solvent (31) between said tank (14) and said bipolar membrane electrodialysis module (13) being also provided, as well as means for conveying (13, 136) the regenerated acid and basic solutions from, respectively, an acid solution tank (132) and a basic solution tank (133) that said module comprises (13), to the means (7, 10) for regulating the pH, said aqueous solvent having a buffer agent concentration of less than 0.02 mol.L$^{-1}$ within said facility (100).

22. The facility (100) for purifying an incoming gas flow (1) according to claim 21, **characterized in that** said first means (10) for regulating the pH of the aqueous solvent (3, 31) are composed of at least one means for measuring the pH, a tank for storing an acid solution and/or a tank for storing a basic solution, and means for injecting said acid or basic solution within the closed circuit (5) between said at least one desorption membrane unit (8) and said absorption membrane unit (2) and **in that** said second means (7) for regulating the pH of the aqueous solvent (30) are composed of at least one means for measuring the pH, of a tank for storing an acid solution and/or of a tank for storing a basic solution and means for injecting said acid or basic solution within the closed circuit (5) between the absorption membrane unit (2) and said at least one desorption membrane unit (8).

23. The facility (100) for purifying an incoming gas flow according to any one of claims 21 or 22, **characterized in that it** comprises, downstream of said absorption membrane unit (2), taking into account the direction of circulation of said aqueous solvent (3, 30, 30', 31) in the closed circuit (5), a first desorption membrane unit (8) and, downstream thereof, a second desorption membrane unit (8'), as well as third means (7') for regulating the pH of the aqueous solvent (30') positioned on the closed circuit (5) between said first desorption membrane unit (8) and said second desorption membrane unit (8'), said third means (7') for regulating the pH of the aqueous solvent (30') being composed of at least one means for measuring the pH, of a tank for storing an acid solution and/or a tank for storing a basic solution, and of means for injecting said acid solution or said basic solution within said closed circuit (5) between said first desorption membrane unit (8) and said second desorption membrane unit (8').

24. The facility (100) for purifying an incoming gas flow according to any one of claims 21 to 23, **characterized in that it** comprises, on the closed circuit (5), between the absorption membrane unit (2) and said at least one desorption membrane unit (8), taking into account the direction of circulation of the aqueous solvent (3, 30, 31) within the closed circuit (5), an intermediate degassing membrane unit (15) comprising at least one membrane (150) permeable to gases and hydrophobic, able to allow the passage of a fraction of the dissolved gases, as well as a recirculation loop (16) connected to the incoming gas flow (1) inlet (4) that the facility (100) comprises to return said fraction of dissolved gas to this inlet (4) of the incoming gas flow (1).

[Fig. 1]

[Fig. 2]

[Fig. 3a]

[Fig. 3b]

[Fig. 4]

[Fig. 5]

[Fig. 6]

**Pureté limite en fonction des conditions de pH**

[Fig. 7]

**Teneur résiduelle en fonction des conditions de pH**

[Fig. 8]

Intensité appliquée en fonction des conditions de pH

△ 0 A ・ 0,1 A ・ 0,5 A ▲ 1 A ▲ 2 A ○ 4 A ・ 6 A ・ 8 A ● 10 A

pH avant absorption / pH avant désorption

[Fig. 9]

Tampon phosphate en fonction des conditions de pH

● 0 M ・ 0,001 M ・ 0,005 M ○ 0,02 M ▲ 0,1 M ▲ 0,4 M ▲ 2 M

pH avant absorption / pH avant désorption

[Fig. 10]

Pureté limite en fonction des conditions de pH

[Fig. 11]

Pureté limite en fonction des conditions de pH

**RÉFÉRENCES CITÉES DANS LA DESCRIPTION**

**Documents brevets cités dans la description**

- EP 3372297 A
- US 2011223650 A
- US 2011174156 A